# EUROPEAN PATENT SPECIFICATION

(11) **EP 3 384 053 B1**
(45) Date of publication and mention of the grant of the patent: **11.08.2021**
(21) Application number: 16871194.3
(22) Date of filing: 25.08.2016
(51) Int. Cl.: C12Q 1/6886

(54) **MULTIPLEX CELLULAR REFERENCE MATERIALS**
ZELLULÄRE MULTIPLEX-REFERENZMATERIALIEN
MATÉRIAUX DE RÉFÉRENCE CELLULAIRES MULTIPLEX

(30) Priority: 01.12.2015 US 201562261514 P; 16.04.2016 US 201662323659 P
(43) Date of publication of application: 10.10.2018
(60) Divisional application: 21179226.2
(73) Proprietor: Seracare Life Sciences, Inc., Milford, MA 01757 (US)
(72) Inventor: HUANG, Catherine, Elkridge, MD 21075 (US); ANEKELLA, Bharathi, Clarksburg, MD 20871 (US)
(74) Representative: Potter Clarkson
(86) International application number: PCT/US2016/048661
(87) International publication number: WO 2017/095486

(56) References cited:
- WO-A1-2014/151117
- WO-A1-2015/073080
- WO-A1-2015/112974
- WO-A2-2014/071419
- CN-B- 103 468 813
- US-A1- 2008 044 819
- US-A1- 2010 184 057
- WAIBHAV D TEMBE ET AL: "Open-access synthetic spike-in mRNA-seq data for cancer gene fusions", BMC GENOMICS, BIOMED CENTRAL, vol. 15, no. 1, 30 September 2014 (2014-09-30), page 824, XP021199040, ISSN: 1471-2164, DOI: 10.1186/1471-2164-15-824

### SEQUENCE LISTING

<110> SERACARE LIFE SCIENCES, INC.
<120> MULTIPLEX RNA REFERENCE MATERIALS
<130> SCX-007.26
<140>
   <141>
<150> 62/323,659
   <151> 2016-04-16
<150> 62/261,514
   <151> 2015-12-01
<160> 13
<170> PatentIn version 3.5
<210> 1
   <211> 3900
   <212> DNA
   <213> Homo sapiens
<400> 1
   ggcggcgcgg cgcggcgctc gcggctgctg cctgggaggg aggccgggca ggcggctgag      60
   cggcgcggct ctcaacgtga cggggaagtg gttcgggcgg ccgcggctta ctaccccagg     120
   gcgaacggac ggacgacgga ggcgggagcc ggtagccgag ccgggcgacc tagagaacga     180
   gcgggtcagg ctcagcgtcg gccactctgt cggtccgctg aatgaagtgc ccgcccctct     240
   gagcccggag cccggcgctt tccccgcaag atggacggtt tcgccggcag tctcgatgat     300
   agtatttctg ctgcaagtac ttctgatgtt caagatcgcc tgtcagctct tgagtcacga     360
   gttcagcaac aagaagatga aatcactgtg ctaaaggcgg ctttggctga tgttttgagg     420
   cgtcttgcaa tctctgaaga tcatgtggcc tcagtgaaaa aatcagtctc aagtaaaggc     480
   caaccaagcc ctcgagcagt tattcccatg tcctgtataa ccaatggaag tggtgcaaac     540
   agaaaaccaa gtcataccag tgctgtctca attgcaggaa aagaaactct ttcatctgct     600
   gctaaaagtg gtacagaaaa aaagaaagaa aaaccacaag gacagagaga aaaaaaagag     660
   gaatctcatt ctaatgatca aagtccacaa attcgagcat caccttctcc ccagccctct     720
   tcacaacctc tccaaataca cagacaaact ccagaaagca agaatgctac tcccaccaaa     780
   agcataaaac gaccatcacc agctgaaaag tcacataatt cttgggaaaa ttcagatgat     840
   agccgtaata aattgtcgaa aataccttca acacccaaat taataccaaa agttaccaaa     900
   actgcagaca agcataaaga tgtcatcatc aaccaagaag gagaatatat taaaatgttt     960
   atgcgcggtc ggccaattac catgttcatt ccttccgatg ttgacaacta tgatgacatc    1020
   agaacggaac tgcctcctga gaagctcaaa ctggagtggg catatggtta tcgaggaaag    1080
   gactgtagag ctaatgttta ccttcttccg accggggaaa tagtttattt cattgcatca 1140
   gtagtagtac tatttaatta tgaggagaga actcagcgac actacctggg ccatacagac 1200
   tgtgtgaaat gccttgctat acatcctgac aaaattagga ttgcaactgg acagatagct 1260
   ggcgtggata aagatggaag gcctctacaa ccccacgtca gagtgtggga ttctgttact 1320
   ctatccacac tgcagattat tggacttggc acttttgagc gtggagtagg atgcctggat 1380
   ttttcaaaag cagattcagg tgttcattta tgtgttattg atgactccaa tgagcatatg 1440
   cttactgtat gggactggca gaagaaagca aaaggagcag aaataaagac aacaaatgaa 1500
   gttgttttgg ctgtggagtt tcacccaaca gatgcaaata ccataattac atgcggtaaa 1560
   tctcatattt tcttctggac ctggagcggc aattcactaa caagaaaaca gggaattttt 1620
   gggaaatatg aaaagccaaa atttgtgcag tgtttagcat tcttggggaa tggagatgtt 1680
   cttactggag actcaggtgg agtcatgctt atatggagca aaactactgt agagcccaca 1740
   cctgggaaag gacctaaagt gtaccgccgg aagcaccagg agctgcaagc catgcagatg 1800
   gagctgcaga gccctgagta caagctgagc aagctccgca cctcgaccat catgaccgac 1860
   tacaacccca actactgctt tgctggcaag acctcctcca tcagtgacct gaaggaggtg 1920
   ccgcggaaaa acatcaccct cattcggggt ctgggccatg gagcctttgg ggaggtgtat 1980
   gaaggccagg tgtccggaat gcccaacgac ccaagccccc tgcaagtggc tgtgaagacg 2040
   ctgcctgaag tgtgctctga acaggacgaa ctggatttcc tcatggaagc cctgatcatc 2100
   agcaaattca accaccagaa cattgttcgc tgcattgggg tgagcctgca atccctgccc 2160
   cggttcatcc tgctggagct catggcgggg ggagacctca agtccttcct ccgagagacc 2220
   cgccctcgcc cgagccagcc ctcctccctg gccatgctgg accttctgca cgtggctcgg 2280
   gacattgcct gtggctgtca gtatttggag gaaaaccact tcatccaccg agacattgct 2340
   gccagaaact gcctcttgac ctgtccaggc cctggaagag tggccaagat tggagacttc 2400
   gggatggccc gagacatcta cagggcgagc tactatagaa agggaggctg tgccatgctg 2460
   ccagttaagt ggatgccccc agaggccttc atggaaggaa tattcacttc taaaacagac 2520
   acatggtcct ttggagtgct gctatgggaa atcttttctc ttggatatat gccatacccc 2580
   agcaaaagca accaggaagt tctggagttt gtcaccagtg gaggccggat ggacccaccc 2640
   aagaactgcc ctgggcctgt ataccggata atgactcagt gctggcaaca tcagcctgaa 2700
   gacaggccca actttgccat cattttggag aggattgaat actgcaccca ggacccggat 2760
   gtaatcaaca ccgctttgcc gatagaatat ggtccacttg tggaagagga agagaaagtg 2820
   cctgtgaggc ccaaggaccc tgagggggtt cctcctctcc tggtctctca acaggcaaaa 2880
   cgggaggagg agcgcagccc agctgcccca ccacctctgc ctaccacctc ctctggcaag 2940
   gctgcaaaga aacccacagc tgcagaggtc tctgttcgag tccctagagg gccggccgtg 3000
   gaagggggac acgtgaatat ggcattctct cagtccaacc ctccttcgga gttgcacagg 3060
   gtccacggat ccagaaacaa gcccaccagc ttgtggaacc caacgtacgg ctcctggttt 3120
   acagagaaac ccaccaaaaa gaataatcct atagcaaaga aggagccaca cgagaggggt 3180
   aacctggggc tggagggaag ctgtactgtc ccacctaacg ttgcaactgg gagacttccg 3240
   ggggcctcac tgctcctaga gccctcttcg ctgactgcca atatgaagga ggtacctctg 3300
   ttcaggctac gtcacttccc ttgtgggaat gtcaattacg gctaccagca acagggcttg 3360
   cccttagaag ccgctactgc ccctggagct ggtcattacg aggataccat tctgaaaagc 3420
   aagaatagca tgaaccagcc tgggccctga gctcggtcac acactcactt ctcttccttg 3480
   ggatccctaa gaccgtggag gagagagagg caatcaatgg ctccttcaca aaccagagac 3540
   caaatgtcac gttttgtttt gtgccaacct attttgaagt accaccaaaa aagctgtatt 3600
   ttgaaaatgc tttagaaagg ttttgagcat gggttcatcc tattctttcg aaagaagaaa 3660
   atatcataaa aatgagtgat aaatacaagg cccagatgtg gttgcataag gtttttatgc 3720
   atgtttgttg tatacttcct tatgcttctt ttaaattgtg tgtgctctgc ttcaatgtag 3780
   tcagaattag ctgcttctat gtttcatagt tggggtcata gatgtttcct tgccttgttg 3840
   atgtggacat gagccatttg aggggagagg gaacggaaat aaaggagtta tttgtaatga 3900
<210> 2
   <211> 2043
   <212> DNA
   <213> Homo sapiens
<400> 2
   atggaagatt cgatggacat ggacatgagc cccctgaggc cccagaacta tcttttcggt 60
   tgtgaactaa aggccgacaa agattatcac tttaaggtgg ataatgatga aaatgagcac 120
   cagttatctt taagaacggt cagtttaggg gctggtgcaa aggatgagtt gcacattgtt 180
   gaagcagagg caatgaatta cgaaggcagt ccaattaaag taacactggc aactttgaaa 240
   atgtctgtac agccaacggt ttcccttggg ggctttgaaa taacaccacc agtggtctta 300
   aggttgaagt gtggttcagg gccagtgcat attagtggac agcacttagt agtgtaccgc 360
   cggaagcacc aggagctgca agccatgcag atggagctgc agagccctga gtacaagctg 420
   agcaagctcc gcacctcgac catcatgacc gactacaacc ccaactactg ctttgctggc 480
   aagacctcct ccatcagtga cctgaaggag gtgccgcgga aaaacatcac cctcattcgg 540
   ggtctgggcc atggcgcctt tggggaggtg tatgaaggcc aggtgtccgg aatgcccaac 600
   gacccaagcc ccctgcaagt ggctgtgaag acgctgcctg aagtgtgctc tgaacaggac 660
   gaactggatt tcctcatgga agccctgatc atcagcaaat tcaaccacca gaacattgtt 720
   cgctgcattg gggtgagcct gcaatccctg ccccggttca tcctgctgga gctcatggcg 780
   gggggagacc tcaagtcctt cctccgagag acccgccctc gcccgagcca gccctcctcc 840
   ctggccatgc tggaccttct gcacgtggct cgggacattg cctgtggctg tcagtatttg 900
   gaggaaaacc acttcatcca ccgagacatt gctgccagaa actgcctctt gacctgtcca 960
   ggccctggaa gagtggccaa gattggagac ttcgggatgg cccgagacat ctacagggcg 1020
   agctactata gaaagggagg ctgtgccatg ctgccagtta agtggatgcc cccagaggcc 1080
   ttcatggaag gaatattcac ttctaaaaca gacacatggt cctttggagt gctgctatgg 1140
   gaaatctttt ctcttggata tatgccatac cccagcaaaa gcaaccagga agttctggag 1200
   tttgtcacca gtggaggccg gatggaccca cccaagaact gccctgggcc tgtataccgg 1260
   ataatgactc agtgctggca acatcagcct gaagacaggc ccaactttgc catcattttg 1320
   gagaggattg aatactgcac ccaggacccg gatgtaatca acaccgcttt gccgatagaa 1380
   tatggtccac ttgtggaaga ggaagagaaa gtgcctgtga ggcccaagga ccctgagggg 1440
   gttcctcctc tcctggtctc tcaacaggca aaacgggagg aggagcgcag cccagctgcc 1500
   ccaccacctc tgcctaccac ctcctctggc aaggctgcaa agaaacccac agctgcagag 1560
   gtctctgttc gagtccctag agggccggcc gtggaagggg gacacgtgaa tatggcattc 1620
   tctcagtcca accctccttc ggagttgcac aaggtccacg gatccagaaa caagcccacc 1680
   agcttgtgga acccaacgta cggctcctgg tttacagaga aacccaccaa aaagaataat 1740
   cctatagcaa agaaggagcc acacgacagg ggtaacctgg ggctggaggg aagctgtact 1800
   gtcccaccta acgttgcaac tgggagactt ccgggggcct cactgctcct agagccctct 1860
   tcgctgactg ccaatatgaa ggaggtacct ctgttcaggc tacgtcactt cccttgtggg 1920
   aatgtcaatt acggctacca gcaacagggc ttgcccttag aagccgctac tgcccctgga 1980
   gctggtcatt acgaggatac cattctgaaa agcaagaata gcatgaacca gcctgggccc 2040
   tga 2043
<210> 3
   <211> 4400
   <212> DNA
   <213> Homo sapiens
<400> 3
   cggaagtgag cattagggtt aacggctgcc ggcgccggct cttcaagtcc cggctccccg      60
   gccgcctcca cccggggaag cgcagcgcgg cgcagctgac tgctgcctct cacggccctc     120
   gcgaccacaa gccctcaggt ccggcgcgtt ccctgcaaga ctgagcggcg gggagtggct     180
   cccggccgcc ggccccggct gcgagaaaga tggcggacct ggccgagtgc aacatcaaag     240
   tgatgtgtcg cttcagacct ctcaacgagt ctgaagtgaa ccgcggcgac aagtacatcg     300
   ccaagtttca gggagaagac acggtcgtga tcgcgtccaa gccttatgca tttgatcggg     360
   tgttccagtc aagcacatct caagagcaag tgtataatga ctgtgcaaag aagattgtta 420
   aagatgtact tgaaggatat aatggaacaa tatttgcata tggacaaaca tcctctggga 480
   agacacacac aatggagggt aaacttcatg atccagaagg catgggaatt attccaagaa 540
   tagtgcaaga tatttttaat tatatttact ccatggatga aaatttggaa tttcatatta 600
   aggtttcata ttttgaaata tatttggata agataaggga cctgttagat gtttcaaaga 660
   ccaacctttc agttcatgaa gacaaaaacc gagttcccta tgtaaagggg tgcacagagc 720
   gttttgtatg tagtccagat gaagttatgg ataccataga tgaaggaaaa tccaacagac 780
   atgtagcagt tacaaatatg aatgaacata gctctaggag tcacagtata tttcttatta 840
   atgtcaaaca agagaacaca caaacggaac aaaagctgag tggaaaactt tatctggttg 900
   atttagctgg tagtgaaaag gttagtaaaa ctggagctga aggtgctgtg ctggatgaag 960
   ctaaaaacat caacaagtca ctttctgctc ttggaaatgt tatttctgct ttggctgagg 1020
   gtagtacata tgttccatat cgagatagta aaatgacaag aatccttcaa gattcattag 1080
   gtggcaactg tagaaccact attgtaattt gctgctctcc atcatcatac aatgagtctg 1140
   aaacaaaatc tacactctta tttggccaaa gggccaaaac aattaagaac acagtttgtg 1200
   tcaatgtgga gttaactgca gaacagtgga aaaagaagta tgaaaaagaa aaagaaaaaa 1260
   ataagatcct gcggaacact attcagtggc ttgaaaatga gctcaacaga tggcgtaatg 1320
   gggagacggt gcctattgat gaacagtttg acaaagagaa agccaacttg gaagctttca 1380
   cagtggataa agatattact cttaccaatg ataaaccagc aaccgcaatt ggagttatag 1440
   gaaattttac tgatgctgaa agaagaaagt gtgaagaaga aattgctaaa ttatacaaac 1500
   agcttgatga caaggatgaa gaaattaacc agcaaagtca actggtagag aaactgaaga 1560
   cgcaaatgtt ggatcaggag gagcttttgg catctaccag aagggatcaa gacaatatgc 1620
   aagctgagct gaatcgcctt caagcagaaa atgatgcctc taaagaagaa gtgaaagaag 1680
   ttttacaggc cctagaagaa cttgctgtca attatgatca gaagtctcag gaagttgaag 1740
   acaaaactaa ggaatatgaa ttgcttagtg atgaattgaa tcagaaatcg gcaactttag 1800
   cgagtataga tgctgagctt cagaaactta aggaaatgac caaccaccag aaaaaacgag 1860
   cagctgagat gatggcatct ttactaaaag accttgcaga aataggaatt gctgtgggaa 1920
   ataatgatgt aaagcagcct gagggaactg gcatgataga tgaagagttc actgttgcaa 1980
   gactctacat tagcaaaatg aagtcagaag taaaaaccat ggtgaaacgt tgcaagcagt 2040
   tagaaagcac acaaactgag agcaacaaaa aaatggaaga aaatgaaaag gagttagcag 2100
   catgtcagct tcgtatctct caacatgaag ccaaaatcaa gtcattgact gaataccttc 2160
   aaaatgtgga acaaaagaaa agacagttgg aggaatctgt cgatgccctc agtgaagaac 2220
   tagtccagct tcgagcacaa gagaaagtcc atgaaatgga aaaggagcac ttaaataagg 2280
   ttcagactgc aaatgaagtt aagcaagctg ttgaacagca gatccagagc catagagaaa 2340
   ctcatcaaaa acagatcagt agtttgagag atgaagtaga agcaaaagca aaacttatta 2400
   ctgatcttca agaccaaaac cagaaaatga tgttagagca ggaacgtcta agagtagaac 2460
   atgagaagtt gaaagccaca gatcaggaaa agagcagaaa actacatgaa cttacggtta 2520
   tgcaagatag acgagaacaa gcaagacaag acttgaaggg tttggaagag acagtggcaa 2580
   aagaacttca gactttacac aacctgcgca aactctttgt tcaggacctg gctacaagag 2640
   ttaaaaagag tgctgagatt gattctgatg acaccggagg cagcgctgct cagaagcaaa 2700
   aaatctcctt tcttgaaaat aatcttgaac agctcactaa agtgcacaaa cagttggtac 2760
   gtgataatgc agatctccgc tgtgaacttc ctaagttgga aaagcgactt cgagctacag 2820
   ctgagagagt gaaagctttg gaatcagcac tgaaagaagc taaagaaaat gcatctcgtg 2880
   atcgcaaacg ctatcagcaa gaagtagatc gcataaagga agcagtcagg tcaaagaata 2940
   tggccagaag agggcattct gcacagattg atccactgtg cgacgagctg tgccgcacgg 3000
   tgatcgcagc cgctgtcctc ttctccttca tcgtctcggt gctgctgtct gccttctgca 3060
   tccactgcta ccacaagttt gcccacaagc cacccatctc ctcagctgag atgaccttcc 3120
   ggaggcccgc ccaggccttc ccggtcagct actcctcttc cggtgcccgc cggccctcgc 3180
   tggactccat ggagaaccag gtctccgtgg atgccttcaa gatcctggag gatccaaagt 3240
   gggaattccc tcggaagaac ttggttcttg gaaaaactct aggagaaggc gaatttggaa 3300
   aagtggtcaa ggcaacggcc ttccatctga aaggcagagc agggtacacc acggtggccg 3360
   tgaagatgct gaaagagaac gcctccccga gtgagcttcg agacctgctg tcagagttca 3420
   acgtcctgaa gcaggtcaac cacccacatg tcatcaaatt gtatggggcc tgcagccagg 3480
   atggcccgct cctcctcatc gtggagtacg ccaaatacgg ctccctgcgg ggcttcctcc 3540
   gcgagagccg caaagtgggg cctggctacc tgggcagtgg aggcagccgc aactccagct 3600
   ccctggacca cccggatgag cgggccctca ccatgggcga cctcatctca tttgcctggc 3660
   agatctcaca ggggatgcag tatctggccg agatgaagct cgttcatcgg gacttggcag 3720
   ccagaaacat cctggtagct gaggggcgga agatgaagat ttcggatttc ggcttgtccc 3780
   gagatgttta tgaagaggat tcctacgtga agaggagcca gggtcggatt ccagttaaat 3840
   ggatggcaat tgaatccctt tttgatcata tctacaccac gcaaagtgat gtatggtctt 3900
   ttggtgtcct gctgtgggag atcgtgaccc taggggggaa cccctatcct gggattcctc 3960
   ctgagcggct cttcaacctt ctgaagaccg gccaccggat ggagaggcca gacaactgca 4020
   gcgaggagat gtaccgcctg atgctgcaat gctggaagca ggagccggac aaaaggccgg 4080
   tgtttgcgga catcagcaaa gacctggaga agatgatggt taagaggaga gactacttgg 4140
   accttgcggc gtccactcca tctgactccc tgatttatga cgacggcctc tcagaggagg 4200
   agacaccgct ggtggactgt aataatgccc ccctccctcg agccctccct tccacatgga 4260
   ttgaaaacaa actctatggt agaatttccc atgcatttac tagattctag caccgctgtc 4320
   ccctctgcac tatccttcct ctctgtgatg ctttttaaaa atgtttctgg tctgaacaaa 4380
   accaaagtct gctctgaacc 4400
<210> 4
   <211> 900
   <212> DNA
   <213> Homo sapiens
<400> 4
   tctcagtcca cccaaggtct cctcggatcg cctggagagg cactcggacc tggagcagtg 60
   aggagaatga ataccttcca agaccagagt ggcagctcca gtaatagaga accccttttg 120
   aggtgtagtg atgcacggag ggacttggag cttgctattg gtggagttct ccgggctgaa 180
   cagcaaatta aagataactt gcgagaggtc aaagctcaga ttcacagttg cataagccgt 240
   cacctggaat gtcttagaag ccgtgaggta tggctgtatg aacaggtgga ccttatttat 300
   cagcttaaag aggagacact tcaacagcag gctcagcagc tctactcgtt attgggccag 360
   ttcaattgtc ttactcatca actggagtgt acccaaaaca aagatctagc caatcaagtc 420
   tctgtgtgcc tggagagact gggcagtttg acccttaagc ctgaagattc aactgtcctg 480
   ctctttgaag ctgacacaat tactctgcgc cagaccatca ccacatttgg gtctctcaaa 540
   accattcaaa ttcctgagca cttgatggct catgctagtt cagcaaatat tgggcccttc 600
   ctggagaaga gaggctgtat ctccatgcca gagcagaagt cagcatccgg tattgtagct 660
   gtccctttca gcgaatggct ccttggaagc aaacctgcca gtggttatca agctccttac 720
   atacccagca ccgaccccca ggactggctt atccaaaagc agaccttgga gaacagtcag 780
   gaggatccaa agtgggaatt ccctcggaag aacttggttc ttggaaaaac tctaggagaa 840
   ggcgaatttg gaaaagtggt caaggcaacg gccttccatc tgaaaggcag agcagggtac 900
<210> 5
   <211> 2112
   <212> DNA
   <213> Homo sapiens
<400> 5
   atgcacagga ggagaagcag gagctgtcgg gaagatcaga agccagtcat ggatgaccag      60
   cgcgacctta tctccaacaa tgagcaactg cccatgctgg gccggcgccc tggggccccg     120
   gagagcaagt gcagccgcgg agccctgtac acaggctttt ccatcctggt gactctgctc     180
   ctcgctggcc aggccaccac cgcctacttc ctgtaccagc agcagggccg gctggacaaa     240
   ctgacagtca cctcccagaa cctgcagctg gagaacctgc gcatgaagct tcccaagcct 300
   cccaagcctg tgagcaagat gcgcatggcc accccgctgc tgatgcaggc gctgcccatg 360
   ggagccctgc cccaggggcc catgcagaat gccaccaagt atggcaacat gacagaggac 420
   catgtgatgc acctgctcca gaatgctgac cccctgaagg tgtacccgcc actgaagggg 480
   agcttcccgg agaacctgag acaccttaag aacaccatgg agaccataga ctggaaggtc 540
   tttgagagct ggatgcacca ttggctcctg tttgaaatga gcaggcactc cttggagcaa 600
   aagcccactg acgctccacc gaaagatgat ttttggatac cagaaacaag tttcatactt 660
   actattatag ttggaatatt tctggttgtt acaatcccac tgacctttgt ctggcataga 720
   agattaaaga atcaaaaaag tgccaaggaa ggggtgacag tgcttataaa cgaagacaaa 780
   gagttggctg agctgcgagg tctggcagcc ggagtaggcc tggctaatgc ctgctatgca 840
   atacatactc ttccaaccca agaggagatt gaaaatcttc ctgccttccc tcgggaaaaa 900
   ctgactctgc gtctcttgct gggaagtgga gcctttggag aagtgtatga aggaacagca 960
   gtggacatct taggagttgg aagtggagaa atcaaagtag cagtgaagac tttgaagaag 1020
   ggttccacag accaggagaa gattgaattc ctgaaggagg cacatctgat gagcaaattt 1080
   aatcatccca acattctgaa gcagcttgga gtttgtctgc tgaatgaacc ccaatacatt 1140
   atcctggaac tgatggaggg aggagacctt cttacttatt tgcgtaaagc ccggatggca 1200
   acgttttatg gtcctttact caccttggtt gaccttgtag acctgtgtgt agatatttca 1260
   aaaggctgtg tctacttgga acggatgcat ttcattcaca gggatctggc agctagaaat 1320
   tgccttgttt ccgtgaaaga ctataccagt ccacggatag tgaagattgg agactttgga 1380
   ctcgccagag acatctataa aaatgattac tatagaaaga gaggggaagg cctgctccca 1440
   gttcggtgga tggctccaga aagtttgatg gatggaatct tcactactca atctgatgta 1500
   tggtcttttg gaattctgat ttgggagatt ttaactcttg gtcatcagcc ttatccagct 1560
   cattccaacc ttgatgtgtt aaactatgtg caaacaggag ggagactgga gccaccaaga 1620
   aattgtcctg atgatctgtg gaatttaatg acccagtgct gggctcaaga acccgaccaa 1680
   agacctactt ttcatagaat tcaggaccaa cttcagttat tcagaaattt tttcttaaat 1740
   agcatttata agtccagaga tgaagcaaac aacagtggag tcataaatga aagctttgaa 1800
   ggtgaagatg gcgatgtgat ttgtttgaat tcagatgaca ttatgccagt tgctttaatg 1860
   gaaacgaaga accgagaagg gttaaactat atggtacttg ctacagaatg tggccaaggt 1920
   gaagaaaagt ctgagggtcc tctaggctcc caggaatctg aatcttgtgg tctgaggaaa 1980
   gaagagaagg aaccacatgc agacaaagat ttctgccaag aaaaacaagt ggcttactgc 2040
   ccttctggca agcctgaagg cctgaactat gcctgtctca ctcacagtgg atatggagat 2100
   gggtctgatt aa 2112
<210> 6
   <211> 1866
   <212> DNA
   <213> Homo sapiens
<400> 6
   atggctccct ggcctgaatt gggagatgcc cagcccaacc ccgataagta cctcgaaggg 60
   gccgcaggtc agcagcccac tgcccctgat aaaagcaaag agaccaacaa aacagataac 120
   actgaggcac ctgtaaccaa gattgaactt ctgccgtcct actccacggc tacactgata 180
   gatgagccca ctgaggtgga tgacccctgg aacctaccca ctcttcagga ctcggggatc 240
   aagtggtcag agagagacac caaagggaag attctctgtt tcttccaagg gattgggaga 300
   ttgattttac ttctcggatt tctctacttt ttcgtgtgct ccctggatat tcttagtagc 360
   gccttccagc tggttggaga tgatttttgg ataccagaaa caagtttcat acttactatt 420
   atagttggaa tatttctggt tgttacaatc ccactgacct ttgtctggca tagaagatta 480
   aagaatcaaa aaagtgccaa ggaaggggtg acagtgctta taaacgaaga caaagagttg 540
   gctgagctgc gaggtctggc agccggagta ggcctggcta atgcctgcta tgcaatacat 600
   actcttccaa cccaagagga gattgaaaat cttcctgcct tccctcggga aaaactgact 660
   ctgcgtctct tgctgggaag tggagccttt ggagaagtgt atgaaggaac agcagtggac 720
   atcttaggag ttggaagtgg agaaatcaaa gtagcagtga agactttgaa gaagggttcc 780
   acagaccagg agaagattga attcctgaag gaggcacatc tgatgagcaa atttaatcat 840
   cccaacattc tgaagcagct tggagtttgt ctgctgaatg aaccccaata cattatcctg 900
   gaactgatgg agggaggaga ccttcttact tatttgcgta aagcccggat ggcaacgttt 960
   tatggtcctt tactcacctt ggttgacctt gtagacctgt gtgtagatat ttcaaaaggc 1020
   tgtgtctact tggaacggat gcatttcatt cacagggatc tggcagctag aaattgcctt 1080
   gtttccgtga aagactatac cagtccacgg atagtgaaga ttggagactt tggactcgcc 1140
   agagacatct ataaaaatga ttactataga aagagagggg aaggcctgct cccagttcgg 1200
   tggatggctc cagaaagttt gatggatgga atcttcacta ctcaatctga tgtatggtct 1260
   tttggaattc tgatttggga gattttaact cttggtcatc agccttatcc agctcattcc 1320
   aaccttgatg tgttaaacta tgtgcaaaca ggagggagac tggagccacc aagaaattgt 1380
   cctgatgatc tgtggaattt aatgacccag tgctgggctc aagaacccga ccaaagacct 1440
   acttttcata gaattcagga ccaacttcag ttattcagaa atttttcttt aaatagcatt 1500
   tataagtcca gagatgaagc aaacaacagt ggagtcataa atgaaagctt tgaaggtgaa 1560
   gatggcgatg tgatttgttt gaattcagat gacattatgc cagttgcttt aatggaaacg 1620
   aagaaccgag aagggttaaa ctatatggta cttgctacag aatgtggcca aggtgaagaa 1680
   aagtctgagg gtcctctagg ctcccaggaa tctgaatctt gtggtctgag gaaagaagag 1740
   aaggaaccac atgcagacaa agatttctgc caagaaaaac aagtggctta ctgcccttct 1800
   ggcaagcctg aaggcctgaa ctatgcctgt ctcactcaca gtggatatgg agatgggtct 1860
   gattaa 1866
<210> 7
   <211> 2301
   <212> DNA
   <213> Homo sapiens
<400> 7
   gtcgaccgga gggcaggagg agcaggagga gcaggagcag gaggagcagg aggagcagga      60
   ggagcaggag gagcaggagg agcaggaaca ggaggaggag gaggaggaga aggaggagca     120
   ggaagagcag gaggaggagg agcaggagca ggaggagcag gagggagagg aggctgcaac     180
   gccgagcgga ggaggcagga accggagcgc gagcagtagc tgggtgggca ccatggctgg     240
   gatcaccacc atcgaggcgg tgaagcgcaa gatccaggtt ctgcagcagc aggcagatga     300
   tgcagaggag cgagctgagc gcctccagcg agaagttgag ggagaaaggc gggcccggga     360
   acaggctgag gctgaggtgg cctccttgaa ccgtaggatc cagctggttg aagaagagct     420
   ggaccgtgct caggagcgcc tggccactgc cctgcaaaag ctggaagaag ctgaaaaagc     480
   tgctgatgag agtgagagag gtatgaaggt tattgaaaac cgggccttaa aagatgaaga     540
   aaagatggaa ctccaggaaa tccaactcga agaagctaag cacattgcag aagaggcaga     600
   taggaagtat gaagaggtgg ctcgtaagtt ggtgatcatt gaaggagact tggaacgcac     660
   agaggaacga gctgagctgg cagagtcgcg ttgccgagag atggatgagc agattagact     720
   gatggaccag aacctgaagt gtctgagtgc tgccgaagaa aagtactctc aaaaagaaga     780
   taaatatgag gaagaaatca agattcttac tgataaactc aaggaggcag agacccgtgc     840
   tgagtttgct gagagatcgg tagccaagct ggaaaagaca attgatgacc tggaagacac     900
   taacagcaca tctggagacc cggtggagaa gaaggacgaa acaccttttg gggtctcggt      960
   ggctgtgggc ctggccgtct ttgcctgcct cttcctttct acgctgctcc ttgtgctcaa    1020
   caaatgtgga cggagaaaca agtttgggat caaccgcccg gctgtgctgg ctccagagga    1080
   tgggctggcc atgtccctgc atttcatgac attgggtggc agctccctgt cccccaccga    1140
   gggcaaaggc tctgggctcc aaggccacat catcgagaac ccacaatact tcagtgatgc    1200
   ctgtgttcac cacatcaagc gccgggacat cgtgctcaag tgggagctgg gggagggcgc    1260
   ctttgggaag gtcttccttg ctgagtgcca caacctcctg cctgagcagg acaagatgct    1320
   ggtggctgtc aaggcactga aggaggcgtc cgagagtgct cggcaggact tccaacgtga    1380
   ggctgagctg ctcaccatgc tgcagcacca gcacatcgtg cgcttcttcg gcgtctgcac    1440
   cgagggccgc cccctgctca tggtcttcga gtatatgcgg cacggggacc tcaaccgctt 1500
   cctccgatcc catggacccg atgccaagct gctggctggt ggggaggatg tggctccagg 1560
   ccccctgggt ctggggcagc tgctggccgt ggctagccag gtcgctgcgg ggatggtgta 1620
   cctggcgggt ctgcattttg tgcaccggga cctggccaca cgcaactgtc tagtgggcca 1680
   gggactggtg gtcaagattg gtgattttgg catgagcagg gatatctaca gcaccgacta 1740
   ttaccgtgtg ggaggccgca ccatgctgcc cattcgctgg atgccgcccg agagcatcct 1800
   gtaccgtaag ttcaccaccg agagcgacgt gtggagcttc ggcgtggtgc tctgggagat 1860
   cttcacctac ggcaagcagc cctggtacca gctctccaac acggaggcaa tcgactgcat 1920
   cacgcaggga cgtgagttgg agcggccacg tgcctgccca ccagaggtct acgccatcat 1980
   gcggggctgc tggcagcggg agcccagcaa cgccacagca tcaaggatgt gcacgcccgg 2040
   ctgcaagccc tggcctaggc acctcctgtc tacctggatg tcctgggcta gggggccggc 2100
   ccaggggctg ggagtggtta gccggaatac tggggcctgc cctcagcatc ccccatagct 2160
   cccagcagcc ccagggtgat ctcgaagtat ctaattcgcc ctcagcatgt gggaagggac 2220
   aggtgggggc tgggagtaga ggatgttcct gcttctctag gcaaggtccc gtcgtagcaa 2280
   ttatatttat tatgggaatt c 2301
<210> 8
   <211> 2003
   <212> DNA
   <213> Homo sapiens
<400> 8
   aacatcctgg agtccaccat gaacggacag ttggatctaa gtgggaagct aatcgtcaaa      60
   gctcaacttg gggaggatat tcggcgaatt cctattcata atgaagatat tacttatgat     120
   gaattagtgc taatgatgca acgagttttc agaggaaaac ttctgagtaa tgatgaagta     180
   acaataaagt ataaagatga agatggagat cttataacaa tttttgatag ttctgacctt     240
   tcctttgcaa ttcagtgcag taggatactg aaactgacat tatttgttaa tggccagcca     300
   agaccccttg aatcaagtca ggtgaaatat ctccgtcgag aactgataga acttcgaaat     360
   aaagtgaatc gtttattgga tagcttggaa ccacctggag aaccaggacc ttccaccaat     420
   attcctgaaa atgatactgt ggatggtagg gaagaaaagt ctgcttctga ttcttctgga     480
   aaacagtcta ctcaggttat ggcagcaagt atgtctgctt ttgatccttt aaaaaaccaa     540
   gatgaaatca ataaaaatgt tatgtcagcg tttggcttaa cagatgatca ggtttcagac     600
   actaacagca catctggaga cccggtggag aagaaggacg aaacaccttt tggggtctcg     660
   gtggctgtgg gcctggccgt ctttgcctgc ctcttccttt ctacgctgct ccttgtgctc     720
   aacaaatgtg gacggagaaa caagtttggg atcaaccgcc cggctgtcct ggctccagag     780
   gatgggctgg ccatgtccct gcatttcatg acattgggtg gcagctccct gtcccccacc 840
   gagggcaaag gctctgggct ccaaggccac atcatcgaga acccacaata cttcagtgat 900
   gcctgtgttc accacatcaa gcgccgggac atcgtgctca agtgggagct gggggagggc 960
   gcctttggga aggtcttcct tgctgagtcc cacaacctcc tgcctgagca ggacaagatg 1020
   ctggtggctg tcaaggcact gaaggaggcg tccgagagtg ctcggcagga cttccaacgt 1080
   gaggctgagc tgctcaccat gctgcagcac cagcacatcg tgcgcttctt cggcgtctgc 1140
   accgagggcc gccccctgct catggtcttt gagtatatgc ggcacgggga cctcaaccgc 1200
   ttcctccgat cccatggacc tgatgccaag ctgctggctg gtggggagga tgtggctcca 1260
   ggccccctgg gtctggggca gctgctggct gtggctagcc aggtcgctgc ggggatggtg 1320
   tacctggcgg gtctgcattt tgtgcaccgg gacctggcca cacgcaactg tctagtgggc 1380
   cagggactgg tggtcaagat tggtgatttt ggcatgagca gggatatcta cagcaccgac 1440
   tattaccgtg tgggaggccg caccatgctg cccattcgct ggatgccgcc cgagagcatc 1500
   ctgtaccgta agttcaccac cgagagcgac gtgtggagct tcggcgtggt gctctgggag 1560
   atcttcacct acggcaagca gccctggtac cagctctcca acacggaggc aatcgactgc 1620
   atcacgcagg gacgtgagtt ggagcggcca cgtgcctgcc caccagaggt ctacgccatc 1680
   atgcggggct gctggcagcg ggagccccag caacgccaca gcatcaagga tgtgcacgcc 1740
   cggctgcaag ccctggccca ggcacctcct gtctacctgg atgtcctggg ctagggggcc 1800
   ggcccagggg ctgggagtgg ttagccggaa tactggggcc tgccctcagc atcccccata 1860
   gctcccagca gccccagggt gatctcgaag tatctaattc gccctcagca tgtgggaagg 1920
   gacaggtggg ggctgggagt agaggatgtt cctgcttctc taggcaaggt cccgtcatag 1980
   caattatatt tattatccct tga 2003
<210> 9
   <211> 2523
   <212> DNA
   <213> Unknown
<220>
   <223> Description of Unknown: Genomic polynucleotide
<400> 9
   atgcctcaca actccatcag atctggccat ggagggctga accagctggg aggggccttt      60
   gtgaatggca gacctctgcc ggaagtggtc cgccagcgca tcgtagacct ggcccaccag     120
   ggtgtaaggc cctgcgacat ctctcgccag ctccgcgtca gccatggctg cgtcagcaag     180
   atccttggca ggtactacga gactggcagc atccggcctg gagtgatagg gggctccaag     240
   cccaaggtgg ccacccccaa ggtggtggag aagattgggg actacaaacg ccagaaccct     300
   accatgtttg cctgggagat ccgagaccgg ctcctggctg agggcgtctg tgacaatgac 360
   actgtgccca gtgtcagctc cattaataga atcatccgga ccaaagtgca gcaaccattc 420
   aacctcccta tggacagctg cgtggccacc aagtccctga gtcccggaca cacgctgatc 480
   cccagctcag ctgtaactcc cccggagtca ccccagtcgg attccctggg ctccacctac 540
   tccatcaatg ggctcctggg catcgctcag cctggcagcg acaagaggaa aatggatgac 600
   agtgatcagg atagctgccg actaagcatt gactcacaga gcagcagcag cggaccccga 660
   aagcaccttc gcacggatgc cttcagccag caccacctcg agccgctcga gtgcccattt 720
   gagcggcagc actacccaga ggcctatgcc tcccccagcc acaccaaagg cgagcagggc 780
   ctctacccgc tgcccttgct caacagcacc ctggacgacg ggaaggccac cctgacccct 840
   tccaacacgc cactggggcg caacctctcg actcaccaga cctaccccgt ggtggcagat 900
   cctcactcac ccttggccat aaagcaggaa acccccgagg tgtccagttc tagctccacc 960
   ccttgctctt tatctagctc cgcccttttg gatctgcagc aagtcggctc cggggtcccg 1020
   cccttcaatg cctttcccca tgctgcctcc gtgtacgggc agttcacggg ccaggccctc 1080
   ctctcagaaa tgaccatggt tgacacagag atgccattct ggcccaccaa ctttgggatc 1140
   agctccgtgg atctctccgt aatggaagac cactcccact cctttgatat caagcccttc 1200
   actactgttg acttctccag catttctact ccacattacg aagacattcc attcacaaga 1260
   acagatccag tggttgcaga ttacaagtat gacctgaaac ttcaagagta ccaaagtgca 1320
   atcaaagtgg agcctgcatc tccaccttat tattctgaga agactcagct ctacaataag 1380
   cctcatgaag agccttccaa ctccctcatg gcaattgaat gtcgtgtctg tggagataaa 1440
   gcttctggat ttcactatgg agttcatgct tgtgaaggat gcaagggttt cttccggaga 1500
   acaatcagat tgaagcttat ctatgacaga tgtgatctta actgtcggat ccacaaaaaa 1560
   agtagaaata aatgtcagta ctgtcggttt cagaaatgcc ttgcagtggg gatgtctcat 1620
   aatgccatca ggtttgggcg gatgccacag gccgagaagg agaagctgtt ggcggagatc 1680
   tccagtgata tcgaccagct gaatccagag tccgctgacc tccgggccct ggcaaaacat 1740
   ttgtatgact catacataaa gtccttcccg ctgaccaaag caaaggcgag ggcgatcttg 1800
   acaggaaaga caacagacaa atcaccattc gttatctatg acatgaattc cttaatgatg 1860
   ggagaagata aaatcaagtt caaacacatc acccccctgc aggagcagag caaagaggtg 1920
   gccatccgca tctttcaggg ctgccagttt cgctccgtgg aggctgtgca ggagatcaca 1980
   gagtatgcca aaagcattcc tggttttgta aatcttgact tgaacgacca agtaactctc 2040
   ctcaaatatg gagtccacga gatcatttac acaatgctgg cctccttgat gaataaagat 2100
   ggggttctca tatccgaggg ccaaggcttc atgacaaggg agtttctaaa gagcctgcga 2160
   aagccttttg gtgactttat ggagcccaag tttgagtttg ctgtgaagtt caatgcactg 2220
   gaattagatg acagcgactt ggcaatattt attgctgtca ttattctcag tggagaccgc      2280
   ccaggtttgc tgaatgtgaa gcccattgaa gacattcaag acaacctgct acaagccctg      2340
   gagctccagc tgaagctgaa ccaccctgag tcctcacagc tgtttgccaa gctgctccag      2400
   aaaatgacag acctcagaca gattgtcacg gaacacgtgc agctactgca ggtgatcaag      2460
   aagacggaga cagacatgag tcttcacccg ctcctgcagg agatctacaa ggacttgtac      2520
   tag                                                                    2523
<210> 10
   <211> 1372
   <212> DNA
   <213> Homo sapiens
<400> 10
   gagaacaacc accaggagtc ctaccctctg tcagtgtctc ccatggagaa taatcactgc 60
   ccagcgtcct ccgagtccca cccgaagcca tccagccccc ggcaggagag cacacgcgtg 120
   atccagctga tgcccagccc catcatgcac cctctgatcc tgaacccccg gcactccgtg 180
   gatttcaaac agtccaggct ctccgaggac gggctgcata gggaagggaa gcccatcaac 240
   ctctctcatc gggaagacct ggcttacatg aaccacatca tggtctctgt ctccccgcct 300
   gaagagcacg ccatgcccat tgggagaata gcagatgtgc agcacattaa gaggagagac 360
   atcgtgctga agcgagaact gggtgaggga gcctttggaa aggtcttcct ggccgagtgc 420
   tacaacctca gcccgaccaa ggacaagatg cttgtggctg tgaaggccct gaaggatccc 480
   accctggctg cccggaagga tttccagagg gaggccgagc tgctcaccaa cctgcagcat 540
   gagcacattg tcaagttcta tggagtgtgc ggcgatgggg accccctcat catggtcttt 600
   gaatacatga agcatggaga cctgaataag ttcctcaggg cccatgggcc agatgcaatg 660
   atccttgtgg atggacagcc acgccaggcc aagggtgagc tggggctctc ccaaatgctc 720
   cacattgcca gtcagatcgc ctcgggtatg gtgtacctgg cctcccagca ctttgtgcac 780
   cgagacctgg ccaccaggaa ctgcctggtt ggagcgaatc tgctagtgaa gattggggac 840
   ttcggcatgt ccagagatgt ctacagcacg gattattaca gggtgggagg acacaccatg 900
   ctccccattc gctggatgcc tcctgaaagc atcatgtacc ggaagttcac tacagagagt 960
   gatgtatgga gcttcggggt gatcctctgg gagatcttca cctatggaaa gcagccatgg 1020
   ttccaactct caaacacgga ggtcattgag tgcattaccc aaggtcgtgt tttggagcgg 1080
   ccccgagtct gccccaaaga ggtgtacgat gtcatgctgg ggtgctggca gagggaacca 1140
   cagcagcggt tgaacatcaa ggagatctac aaaatcctcc atgctttggg gaaggccacc 1200
   ccaatctacc tggacattct tggctagtgg tggctggtgg tcatgaattc atactctgtt 1260
   gcctcctctc tccctgcctc acatctccct tccacctcac aactccttcc atccttgact 1320
   gaagcgaaca tcttcatata aactcaagtg cctgctacac atacaacact ga 1372
<210> 11
   <211> 3237
   <212> DNA
   <213> Artificial Sequence
<220>
   <223> Description of Artificial Sequence: Synthetic polynucleotide
<400> 11
   atttaggtga cactatagaa gtggctgtgg agtttcaccc aacagatgca aataccataa 60
   ttacatgcgg taaatctcat attttcttct ggacctggag cggcaattca ctaacaagaa 120
   aacagggaat ttttgggaaa tatgaaaagc caaaatttgt gcagtgttta gcattcttgg 180
   ggaatggaga tgttcttact ggagactcag gtggagtcat gcttatatgg agcaaaacta 240
   ctgtagagcc cacacctggg aaaggaccta aagtgtaccg ccggaagcac caggagctgc 300
   aagccatgca gatggagctg cagagccctg agtacaagct gagcaagctc cgcacctcga 360
   ccatcatgac cgactacaac cccaactact gctttgctgg caagacctcc tccatcagtg 420
   acctgaagga ggtgccgcgg aaaaacatca ccctcattcg gggtctgggc catggagcct 480
   ttggggaggt gtatgaaggc caggtgtccg gaatgcccaa cgaaatcttg aacagctcac 540
   taaagtgcac aaacagttgg tacgtgataa tgcagatctc cgctgtgaac ttcctaagtt 600
   ggaaaagcga cttcgagcta cagctgagag agtgaaagct ttggaatcag cactgaaaga 660
   agctaaagaa aatgcatctc gtgatcgcaa acgctatcag caagaagtag atcgcataaa 720
   ggaagcagtc aggtcaaaga atatggccag aagagggcat tctgcacaga ttgatccact 780
   gtgcgacgag ctgtgccgca cggtgatcgc agccgctgtc ctcttctcct tcatcgtctc 840
   ggtgctgctg tctgccttct gcatccactg ctaccacaag tttgcccaca agccacccat 900
   ctcctcagct gagatgacct tccggaggcc cgcccaggcc ttcccggtca gctactcctc 960
   ttccggtgcc cgccggccct cgctggactc catggagaac caggtctccg tggatgcctt 1020
   caaaggggcc catgcagaat gccaccaagt atggcaacat gacagaggac catgtgatgc 1080
   acctgctcca gaatgctgac cccctgaagg tgtacccgcc actgaagggg agcttcccgg 1140
   agaacctgag acaccttaag aacaccatgg agaccataga ctggaaggtc tttgagagct 1200
   ggatgcacca ttggctcctg tttgaaatga gcaggcactc cttggagcaa aagcccactg 1260
   acgctccacc gaaagatgat ttttggatac cagaaacaag tttcatactt actattatag 1320
   ttggaatatt tctggttgtt acaatcccac tgacctttgt ctggcataga agattaaaga 1380
   atcaaaaaag tgccaaggaa ggggtgacag tgcttataaa cgaagacaaa gagttggctg 1440
   agctgcgagg tctggcagcc ggagtaggcc tggctaatgc ctgctatgca atacatactc 1500
   ttccaaccca agaggagatt gaaacttgga acgcacagag gaacgagctg agctggcaga 1560
   gtcgcgttgc cgagagatgg atgagcagat tagactgatg gaccagaacc tgaagtgtct 1620
   gagtgctgcc gaagaaaagt actctcaaaa agaagataaa tatgaggaag aaatcaagat 1680
   tcttactgat aaactcaagg aggcagagac ccgtgctgag tttgctgaga gatcggtagc 1740
   caagctggaa aagacaattg atgacctgga agacactaac agcacatctg gagacccggt 1800
   ggagaagaag gacgaaacac cttttggggt ctcggtggct gtgggcctgg ccgtctttgc 1860
   ctgcctcttc ctttctacgc tgctccttgt gctcaacaaa tgtggacgga gaaacaagtt 1920
   tgggatcaac cgcccggctg tgctggctcc agaggatggg ctggccatgt ccctgcattt 1980
   catgacattg ggtggcagct ccctgtcccc caccgagggc aaatgacgtc tggtcctttg 2040
   gggtcctgct ctgggagatc ttcacgctgg ggggctcccc gtaccccggc atccctgtgg 2100
   aggagctctt caagctgctg aaggagggcc accgcatgga caagcccgcc aactgcacac 2160
   acgacctgta catgatcatg cgggagtgct ggcatgccgc gccctcccag aggcccacct 2220
   tcaagcagct ggtggaggac ctggaccgtg tccttaccgt gacgtccacc gacaatgtta 2280
   tggaacagtt caatcctggg ctgcgaaatt taataaacct ggggaaaaat tatgagaaag 2340
   ctgtaaacgc tatgatcctg gcaggaaaag cctactacga tggagtggcc aagatcggtg 2400
   agattgccac tgggtccccc gtgtcaactg aactgggaca tgtcctcata gagatttcaa 2460
   gtacccacaa gaaactcaac gagagtcttg atgaaaattt taaaaaattc cacaaagaga 2520
   ttaacccctt ccaacacgcc actggggcgc aacctctcga ctcaccagac ctaccccgtg 2580
   gtggcagatc ctcactcacc cttggccata aagcaggaaa cccccgaggt gtccagttct 2640
   agctccaccc cttgctcttt atctagctcc gcccttttgg atctgcagca agtcggctcc 2700
   ggggtcccgc ccttcaatgc ctttccccat gctgcctccg tgtacgggca gttcacgggc 2760
   caggccctcc tctcagaaat gaccatggtt gacacagaga tgccattctg gcccaccaac 2820
   tttgggatca gctccgtgga tctctccgta atggaagacc actcccactc ctttgatatc 2880
   aagcccttca ctactgttga cttctccagc atttctactc cacattacga agacattcca 2940
   ttcacaagaa cagatccagt ggttgcagat tacaagtatg acctgaaact tcaagagtac 3000
   caaagtgcaa tcaaagtgga gccgaaacaa aggcgtggtt tagagtaggt ataacaggca 3060
   ctttagcagt ggccgtgacg acccggtatg aggtagacaa tattacacct gtcctactgg 3120
   cattgagaac ttttgcccag agcaaaagta acttttatta tttcttttat taatcaacaa 3180
   aattttgttt ttaacatttc aaaaaaaaaa aaaaaaaaaa aaaaaaaaaa aaaaaaa 3237
<210> 12
   <211> 3237
   <212> DNA
   <213> Artificial Sequence
<220>
   <223> Description of Artificial Sequence: Synthetic polynucleotide
<400> 12
   atttaggtga cactatagaa gtggatgaaa atgagcacca gttatcttta agaacggtca      60
   gtttaggggc tggtgcaaag gatgagttgc acattgttga agcagaggca atgaattacg     120
   aaggcagtcc aattaaagta acactggcaa ctttgaaaat gtctgtacag ccaacggttt     180
   cccttggggg ctttgaaata acaccaccag tggtcttaag gttgaagtgt ggttcagggc     240
   cagtgcatat tagtggacag cacttagtag tgtaccgccg gaagcaccag gagctgcaag     300
   ccatgcagat ggagctgcag agccctgagt acaagctgag caagctccgc acctcgacca     360
   tcatgaccga ctacaacccc aactactgct ttgctggcaa gacctcctcc atcagtgacc     420
   tgaaggaggt gccgcggaaa aacatcaccc tcattcgggg tctgggccat ggcgcctttg     480
   gggaggtgta tgaaggccag gtgtccggaa tgcccaacga cccgtctctc aaaaccattc     540
   aaattcctga gcacttgatg gctcatgcta gttcagcaaa tattgggccc ttcctggaga     600
   agagaggctg tatctccatg ccagagcaga agtcagcatc cggtattgta gctgtccctt     660
   tcagcgaatg gctccttgga agcaaacctg ccagtggtta tcaagctcct tacataccca     720
   gcaccgaccc ccaggactgg cttacccaaa agcagacctt ggagaacagt caggaggatc     780
   caaagtggga attccctcgg aagaacttgg ttcttggaaa aactctagga gaaggcgaat     840
   ttggaaaagt ggtcaaggca acggccttcc atctgaaagg cagagcaggg tacaccacgg     900
   tggccgtgaa gatgctgaaa gagaacgcct ccccgagtga gcttcgagac ctgctgtcag     960
   agttcaacgt cctgaagcag gtcaaccacc cacatgtcat caaattgtat ggggcctgca    1020
   gcctgaggca cctgtaacca agattgaact tctgccgtcc tactccacgg ctacactgat    1080
   agatgagccc actgaggtgg atgacccctg gaacctaccc actcttcagg actcggggat    1140
   caagtggtca gagagagaca ccaaagggaa gattctctgt ttcttccaag ggattgggag    1200
   attgatttta cttctcggat ttctctactt tttcgtgtgc tccctggata ttcttagtag    1260
   cgccttccag ctggttggag atgatttttg gataccagaa acaagtttca tacttactat     1320
   tatagttgga atatttctgg ttgttacaat cccactgacc tttgtctggc atagaagatt    1380
   aaagaatcaa aaaagtgcca aggaaggggt gacagtgctt ataaacgaag acaaagagtt    1440
   ggctgagctg cgaggtctgg cagccggagt aggcctggct aatgcctgct atgcaataca    1500
   tactcttcca acccaagagg agaagaactt cgaaataaag tgaatcgttt attggatagc    1560
   ttggaaccac ctggagaacc aggaccttcc accaatattc ctgaaaatga tactgtggat    1620
   ggtagggaag aaaagtctgc ttctgattct tctggaaaac agtctactca ggttatggca    1680
   gcaagtatgt ctgcttttga tcctttaaaa aaccaagatg aaatcaataa aaatgttatg 1740
   tcagcgtttg gcttaacaga tgatcaggtt tcagacacta acagcacatc tggagacccg 1800
   gtggagaaga aggacgaaac accttttggg gtctcggtgg ctgtgggcct ggccgtcttt 1860
   gcctgcctct tcctttctac gctgctcctt gtgctcaaca aatgtggacg gagaaacaag 1920
   tttgggatca accgcccggc tgtcctggct ccagaggatg ggctggccat gtccctgcat 1980
   ttcatgacat tgggtggcag ctccctgtcc cccaccgagg gcatgacgtc tggtcctttg 2040
   gggtcctgct ctgggagatc ttcacgctgg ggggctcccc gtaccccggc atccctgtgg 2100
   aggagctctt caagctgctg aaggagggcc accgcatgga caagcccgcc aactgcacac 2160
   acgacctgta catgatcatg cgggagtgct ggcatgccgc gccctcccag aggcccacct 2220
   tcaagcagct ggtggaggac ctggaccgtg tccttaccgt gacgtccacc gacgtaaagg 2280
   cgacacagga ggagaaccgg gagctgagga gcaggtgtga ggagctccac gggaagaacc 2340
   tggaactggg gaagatcatg gacaggttcg aagaggttgt gtaccaggcc atggaggaag 2400
   ttcagaagca gaaggaactt tccaaagctg aaatccagaa agttctaaaa gaaaaagacc 2460
   aacttaccac agatctgaac tccatggaga agtccttctc cgacctcttc aagcgttttg 2520
   agaccatcca gcccccggca ggagagcaca cgcgtgatcc agctgatgcc cagccccatc 2580
   atgcaccctc tgatcctgaa cccccggcac tccgtggatt tcaaacagtc caggctctcc 2640
   gaggacgggc tgcataggga agggaagccc atcaacctct ctcatcggga agacctggct 2700
   tacatgaacc acatcatggt ctctgtctcc ccgcctgaag agcacgccat gcccattggg 2760
   agaatagcag atgtgcagca cattaagagg agagacatcg tgctgaagcg agaactgggt 2820
   gagggagcct ttggaaaggt cttcctggcc gagtgctaca acctcagccc gaccaaggac 2880
   aagatgcttg tggctgtgaa ggccctgaag gatcccaccc tggctgcccg gaaggatttc 2940
   cagagggagg ccgagctgct caccaacctg cagcatgagc acattgtcaa gttctatgga 3000
   gtgtgcggcg atggggaccc cctgaaacaa aggcgtggtt tagagtaggt ataacaggca 3060
   ctttagcagt ggccgtgacg acccggtatg aggtagacaa tattacacct gtcctactgg 3120
   cattgagaac ttttgcccag agcaaaagta acttttatta tttcttttat taatcaacaa 3180
   aattttgttt ttaacatttc aaaaaaaaaa aaaaaaaaaa aaaaaaaaaa aaaaaaa 3237
<210> 13
   <211> 14
   <212> DNA
   <213> Artificial Sequence
<220>
   <223> Description of Artificial Sequence: Synthetic oligonucleotide
<400> 13
   aaaaaaaaaa aaaa 14

## Description

### RELATED APPLICATIONS

This application claims the benefit of priority to U.S. Provisional Patent Application No. 62/261,514, filed December 1, 2015, and U.S. Provisional Patent Application No. 62/323,659, filed April 16, 2016.

### SEQUENCE LISTING

The instant application contains a Sequence Listing which has been submitted electronically in ASCII format. Said ASCII copy, created on August 8, 2016, is named SCX-007_26_SL.txt and is 40,863 bytes in size.

### BACKGROUND

Cell-based reference materials are useful as process controls in analyzing samples or validating methods. Reference materials are limited, however, in that a library of controls may be necessary to analyze a sample with unknown features. For example, certain cancer assays screen for a number of different biomarkers, and each biomarker may require a different reference material, which complicates the analysis. Streamlined approaches for analyzing samples with unknown features are therefore desirable.

Waibhav et al., 2014, BMC Genomics 15(1):824 discloses open-access synthetic spike-in mRNA-seq data for cancer gene fusions.

CN 103 468 813 B discloses an EML4-ALK (Echinoderm microtubule associated protein like 4-anaplastic lymphoma kinase) fusion gene fluorescent quantitative PCR (polymerase chain reaction) assay kit.

WO 2015/073080 A1 discloses a plurality of nucleic acid sequences comprising multiple variants of a reference sequence; and plasmids, cells, methods, and kits comprising the same.

### SUMMARY

Aspects of the invention relate to nucleic acids comprising a plurality of nucleotide sequences, wherein each nucleotide sequence corresponds to a genotype. Embodiments of the invention are set out in the claims. The nucleic acids are useful for developing biological reference materials comprising a number of different genotypes. These reference materials have many advantages. For example, each genotype of a nucleic acid will appear in a reference material at the same frequency, which simplifies the preparation of the reference material. Additionally, different nucleic acids may be combined to allow for much larger combinations of different genotypes relative to libraries of nucleic acids that each comprise a single genotype.

### BRIEF DESCRIPTION OF THE FIGURES

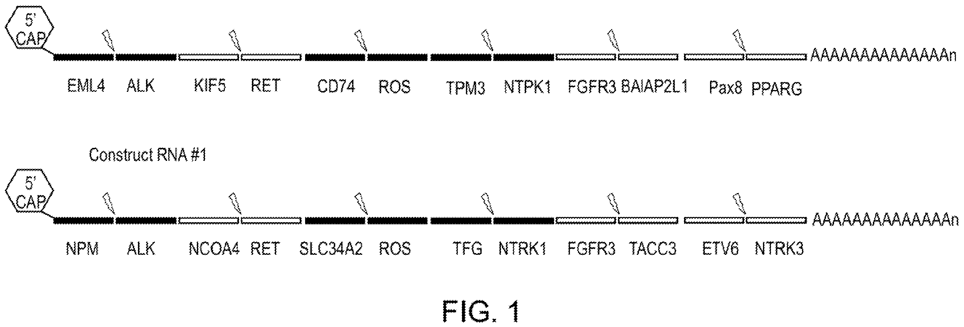

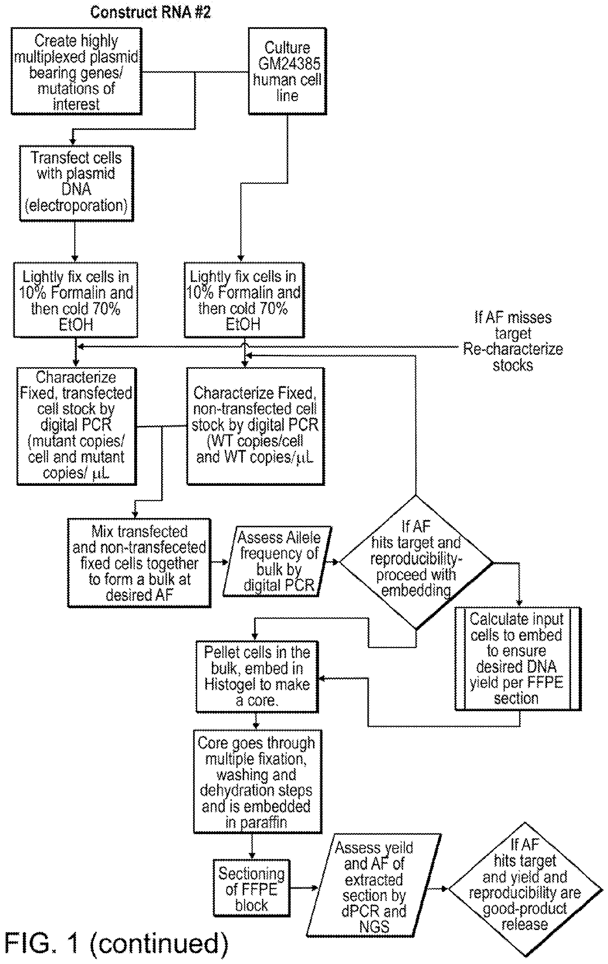

**Figure** 1 shows two embodiments of the invention, labeled "Construct RNA #1" and "Construct RNA #2." Each construct comprises a 5' [m7G(5')ppp(5')G] cap, labeled "5' cap," and a poly-A tail. Each construct comprises six nucleotide sequences that consist of two subsequences each, wherein each nucleotide sequence is associated with cancer. For example, the first nucleotide sequence of Construct RNA #1 consists of a subsequence of EML4 exon 13 and a subsequence of ALK exon 20, to serve as a control for a EML4 exon 13-ALK exon 20 fusion, which is associated with non-small-cell lung cancer. The constructs are examples of multiplex oncology reference materials. Figure 1 also shows a flow chart for constructing reference materials from the constructs or from other multiplexed nucleic acids. Figure 1 also discloses two instances of SEQ ID NO: 13.
**Figure 2** shows next generation sequencing results for an RNA library prepared from nucleic acids extracted from formalin-fixed cells comprising Construct #1 diluted with untransfected cells. The sequencing results correctly identified each gene fusion in the construct.
**Figure 3** shows next generation sequencing results for an RNA library prepared from nucleic acids extracted from formalin-fixed cells comprising Construct #2 diluted with untransfected cells. The sequencing results correctly identified each gene fusion in the construct.
**Figure 4** is a graph that shows the number of reads through each junction spanning a gene fusion of Construct #1, which was transfected into human cells that were fixed with formalin and diluted with untransfected cells.
**Figure 5** is a graph that shows the number of reads through each junction spanning a gene fusion of Construct #2, which was transfected into human cells that were fixed with formalin and diluted with untransfected cells.
**Figure 6** is a graph that shows the number of reads through each junction spanning a gene fusion of Construct #1, which was transfected into human cells that were fixed with formalin and diluted with untransfected cells.
**Figure 7** is a graph that shows the number of reads through each junction spanning a gene fusion of Construct #2, which was transfected into human cells that were fixed with formalin and diluted with untransfected cells.
**Figure 8** is a graph that shows the number of reads through each junction spanning a gene fusion of (1) a formalin-fixed paraffin-embedded sample diluted at a fusion construct to cell ratio of 1:1000 described in Example 4 ("FFPE Med") and (2) a similarly-prepared sample with approximately five-times as many cells described in Example 5 ("102380").
**Figure 9** is a graph that shows the size distribution of RNA extracted from reference material 102380, which is described in Examples 5 and 6.

### DETAILED DESCRIPTION

Aspects of the invention relate to nucleic acids comprising a number of different genotypes for use in producing biological reference materials. A biological reference material may comprise, for example, a cell comprising such a nucleic acid. A nucleic acid comprising several different genotypes of interest may be used to transfect a group of cells to generate a reference material comprising each genotype of the nucleic acid. The single nucleic acid format is desirable for many reasons. For example, having a number of genotypes on a single nucleic acid simplifies quantification of the nucleic acid because one nucleic acid needs to be accurately quantified only once. This format also enables "mega" mixes (mixtures of multiple nucleic acids, each bearing multiple different genotypes) allowing hundreds of genotypes to be incorporated into the same control, e.g., thereby allowing a biosynthetic control that mimics multiple heterozygous variants. Additionally, nucleic acids comprising a number of different genotypes allows one to quantitatively transfect each genotype into a cell at the same concentration. Advantages for end users include confirmation that genotypes were assessed in a Whole Exome Sequencing test (WES-test) and confirmation that difficult to sequence genotypes were detected in a sequencing run by using the reference material as a positive control. Finally, multiplexed controls are cheaper than libraries of numerous single-mutant controls.

### I. NUCLEIC ACIDS

In some aspects, the invention relates to a nucleic acid, comprising a plurality of nucleotide sequences, wherein each nucleotide sequence of the plurality is associated with a disease or condition. The nucleic acid may be DNA or RNA. When the term refers to RNA, each thymine T of a nucleotide sequence may be substituted with uracil U. A nucleic acid as described herein may be referred to as a "full-length nucleic acid" for clarity, *e.g.,* to differentiate a nucleic acid and fragment thereof.

Each nucleotide sequence of the plurality of nucleotide sequences may comprise a first subsequence and a second subsequence, wherein the first subsequence comprises a 3' sequence of a first exon, the second subsequence comprises a 5' sequence of a second exon, and the first subsequence and second subsequence are adjoining sequences in the nucleic acid (and in the nucleotide sequence). The first subsequence may be 5' relative to the second subsequence, *i.e.,* the first subsequence may occur first in the nucleotide sequence and be immediately followed by the second subsequence. The first exon may be from a first gene, and the second exon may be from a second gene, e.g., wherein the first gene and second gene are different genes. Thus, each nucleotide sequence of a plurality of nucleotide sequence may replicate a gene fusion, for example, of a misprocessed mRNA, wherein the misprocessed mRNA contains exons from two different genes. Each nucleotide sequence of the plurality of nucleotide sequences may comprise a 3' sequence of a different first exon and/or a 5' sequence of a different second exon.

mRNA that comprises a gene fusion often occurs in diseased cells including cancer cells, and a nucleotide sequence of a plurality of nucleotide sequences may therefore be a naturally occurring nucleotide sequence. The combination of multiple gene fusions in a single nucleic acid according to various embodiments of the invention, however, is not known to occur in nature. The first subsequence and second subsequence may be adjoining "in frame" such that the translation of the nucleotide sequence comprising the first subsequence and second subsequence would result in a polypeptide.

A nucleotide sequence may be associated with a disease or condition if a subject having the sequence has an increased risk of developing the disease or condition. A nucleotide sequence may be associated with a disease or condition if its presence or absence correlates with the progression or severity of a disease or condition. For example, certain nucleotide sequences correlate with the aggressiveness of various neoplasms such as adenocarcinomas, transitional cell carcinomas, neuroblastomas, AML, CML, CMML, JMML, ALL, Burkitt's lymphoma, Hodgkin's lymphoma, plasma cell myeloma, hepatocellular carcinoma, large cell lung carcinoma, non-small cell lung carcinoma, squamous cell carcinoma, lung neoplasia, ductal adenocarcinomas, endocrine tumors, basal cell carcinoma, malignant melanomas, angiosarcoma, leiomyosarcoma, liposarcoma, rhabdomyosarcoma, myxoma, malignant fibrous histiocytoma-pleomorphic sarcoma, germinoma, seminoma, anaplastic carcinoma, follicular carcinoma, papillary carcinoma, and Hurthle cell carcinoma. For example, gene fusions are known to occur in various cancers, including lung cancer, non-small cell lung cancer, soft tissue cancer, lymphoid cancer, acute lymphoid leukemia, acute myeloid leukemia, chronic myelogenous leukemia, non-Hodgkin's lymphoma, Burkitt lymphoma, melanoma, intraocular melanoma, central nervous system cancer, neuroblastoma, thyroid cancer, parathyroid cancer, hepatocellular cancer, stomach cancer, large intestine cancer, colon cancer, urinary tract cancer, bladder cancer, kidney cancer, prostate cancer, cervical cancer, ovarian cancer, or breast cancer.

A plurality of nucleotide sequences may comprise at least 2 nucleotide sequences, e.g., at least 2 nucleotide sequences that do not overlap on the nucleic acid. A plurality of nucleotide sequences may comprise at least 3, at least 4, at least 5, at least 6, at least 7, at least 8, at least 9, or at least 10 nucleotide sequences. A plurality of nucleotide sequences may comprise 2 to 1000 nucleotide sequences (e.g., 2 to 1000 nucleotide sequences that do not overlap). A plurality of nucleotide sequences may comprise 2 to 100 nucleotide sequences, such as 2 to 50, 2 to 20, 2 to 12, 3 to 1000, 3 to 100, 3 to 50, 3 to 20, 3 to 12, 4 to 1000, 4 to 100, 4 to 50, 4 to 20, 4 to 12, 5 to 1000, 5 to 100, 5 to 50, 5 to 20, 5 to 12, 6 to 1000, 6 to 100, 6 to 50, 6 to 20, 6 to 12, 7 to 1000, 7 to 100, 7 to 50, 7 to 20, 7 to 12, 8 to 1000, 8 to 100, 8 to 50, 8 to 20, 8 to 12, 9 to 1000, 9 to 100, 9 to 50, 9 to 20, 9 to 12, 10 to 1000, 10 to 100, 10 to 50, 10 to 20, 10 to 16, or 10 to 12 nucleotide sequences. A plurality of nucleotide sequences may consist of 2, 3, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, or 200 different nucleotide sequences.

In certain embodiments, each nucleotide sequence of a plurality is the nucleotide sequence of a naturally-occurring gene or mRNA (*e.g.,* a gene or mRNA that is associated with a disease or a condition of interest) or a subsequence thereof. A naturally-occurring gene or mRNA includes healthy genotypes and genotypes that are associated with a disease or condition. The term "genotype" refers to a genetic trait, such as a splice variant or gene fusion. For example, a nucleotide sequence may comprise a subsequence of a gene fusion, and in such embodiments, the subsequence may comprise a portion of each gene of the gene fusion. In certain embodiments, each nucleotide sequence of a plurality comprises a genotype, *e.g.,* a junction of a gene fusion. A nucleotide sequence of a plurality may comprise a healthy genotype in a nucleotide sequence in which deleterious splice variants or gene fusions are known to occur. A nucleotide sequence of a plurality may comprise an exon of a gene or a subsequence of an exon. A nucleotide sequence may consist of an exon of a first gene, or a subsequence thereof, and an exon of a second gene, or a subsequence thereof.

A nucleotide sequence may comprise more than one exon of a first gene (*e.g.,* either two full, consecutive exons or one full exon and a subsequence of a second, consecutive exon), and an exon of a second gene, or a subsequence thereof. A nucleotide sequence may comprise an exon of a first gene or a subsequence thereof, and more than one exon of a second gene (*e.g.,* either two full, consecutive exons or one full exon and a subsequence of a second, consecutive exon). A nucleotide sequence may comprise more than one exon of the same gene, for example, when a single exon is not long enough to be reliably identified by next generation sequencing.

In certain embodiments, each nucleotide sequence of a plurality is sufficiently long to be identified by nucleic acid sequencing, e.g., next generation sequencing (NGS). In certain embodiments, a nucleotide sequence of a plurality comprises a genotype of interest at a position that can be identified by nucleic acid sequencing, e.g., the genotype of interest, such as a gene fusion (*e.g.,* gene fusion breakpoint), may be positioned in or near the middle of the nucleotide sequence.

A nucleic acid may be about 1000 nucleotides to about 100,000 nucleotides long, such as about 3000 to about 60,000 nucleotides long, about 5000 to about 50,000 nucleotides long, or about 8000 to about 20,000 nucleotides long.

A nucleotide sequence of a plurality may be at least 20 nucleotides (or base pairs) long, such as at least 20, 30, 40, 50, 60, 70, 80, 90, 100, 120, 150, 200, or at least 250 nucleotides (or base pairs) long. A nucleotide sequence of a plurality may be 20 to 10,000 nucleotides (or base pairs) long, such as 20 to 5000, 20 to 2000, 20 to 1000, 20 to 500, 30 to 5000, 30 to 2000, 30 to 1000, 30 to 500, 40 to 5000, 40 to 2000, 40 to 1000, 40 to 500, 50 to 5000, 50 to 2000, 50 to 1000, 50 to 500, 60 to 5000, 60 to 2000, 60 to 1000, 60 to 500, 70 to 5000, 70 to 2000, 70 to 1000, 70 to 500, 80 to 5000, 80 to 2000, 80 to 1000, 80 to 500, 90 to 5000, 90 to 2000, 90 to 1000, 90 to 500, 100 to 5000, 100 to 2000, 100 to 1000, 100 to 500, 120 to 5000, 120 to 2000, 120 to 1000, 120 to 500, 150 to 5000, 150 to 2000, 150 to 1000, 150 to 500, 200 to 5000, 200 to 2000, 200 to 1000, or 200 to 500 nucleotides (or base pairs) long.

A subsequence of a nucleotide sequence (e.g., first subsequence or second subsequence) may be at least 20 nucleotides (or base pairs) long, such as at least 25, 30, 40, 50, 60, 70, 80, 90, 100, 120, 150, 200, or at least 250 nucleotides (or base pairs) long. A subsequence of a nucleotide sequence (e.g., first subsequence or second subsequence) may be 20 to 10,000 nucleotides (or base pairs) long, such as 20 to 5000, 20 to 2000, 20 to 1000, 20 to 500, 25 to 5000, 25 to 2000, 25 to 1000, 25 to 500, 25 to 250, 30 to 5000, 30 to 2000, 30 to 1000, 30 to 500, 30 to 250, 30 to 5000, 40 to 2000, 40 to 1000, 40 to 500, 40 to 250, 50 to 5000, 50 to 2000, 50 to 1000, 50 to 500, 50 to 250, 60 to 5000, 60 to 2000, 60 to 1000, 60 to 500, 60 to 250, 70 to 5000, 70 to 2000, 70 to 1000, 70 to 500, 70 to 250, 80 to 5000, 80 to 2000, 80 to 1000, 80 to 500, 80 to 250, 90 to 5000, 90 to 2000, 90 to 1000, 90 to 500, 90 to 250, 100 to 5000, 100 to 2000, 100 to 1000, 100 to 500, 100 to 250, 120 to 5000, 120 to 2000, 120 to 1000, 120 to 500, 120 to 250, 150 to 5000, 150 to 2000, 150 to 1000, 150 to 500, 150 to 250, 200 to 5000, 200 to 2000, 200 to 1000, 200 to 500, or 200 to 250 nucleotides (or base pairs) long.

A nucleotide sequence of a plurality may comprise a genotype of interest (e.g., gene fusion breakpoint) at a position that is at least 20 nucleotides (or base pairs) from the 5' end and/or 3' end of the nucleotide sequence, such as at least 25, 30, 40, 50, 60, 70, 80, 90, 100, 120, 150, 200, or 250 nucleotides (or base pairs) from the 5' and/or 3' end of the nucleotide sequence. A nucleotide sequence of a plurality may comprise a genotype of interest (*e.g*., gene fusion breakpoint) at a position that is 20 to 5000 nucleotides (or base pairs) from the 5' end and/or 3' end of the nucleotide sequence, such as 25 to 5000, 30 to 5000, 40 to 5000, 50 to 5000, 60 to 5000, 70 to 5000, 80 to 5000, 90 to 5000, 100 to 5000, 120 to 5000, 150 to 5000, 200 to 5000, 250 to 5000, 25 to 2000, 30 to 2000, 40 to 2000, 50 to 2000, 60 to 2000, 70 to 2000, 80 to 2000, 90 to 2000, 100 to 2000, 120 to 2000, 150 to 2000, 200 to 2000, 250 to 2000, 25 to 1000, 30 to 1000, 40 to 1000, 50 to 1000, 60 to 1000, 70 to 1000, 80 to 1000, 90 to 1000, 100 to 1000, 120 to 1000, 150 to 1000, 200 to 1000, 250 to 1000, 25 to 750, 30 to 750, 40 to 750, 50 to 750, 60 to 750, 70 to 750, 80 to 750, 90 to 750, 100 to 750, 120 to 750, 150 to 750, 200 to 750, 250 to 750, 25 to 500, 30 to 500, 40 to 500, 50 to 500, 60 to 500, 70 to 500, 80 to 500, 90 to 500, 100 to 500, 120 to 500, 150 to 500, 200 to 500, or 250 to 500 nucleotides (or base pairs) from the 5' and/or 3' end of the nucleotide sequence.

In some embodiments, a nucleotide sequence of a plurality comprises a gene fusion. For example, a nucleotide sequence of a plurality may comprise a first subsequence and a second subsequence, wherein the first subsequence comprises a 3' sequence of a first exon and the second subsequence comprises the 5' sequence of a second exon. The first subsequence and second subsequence may be adjoining sequences in the nucleotide sequence, and the first subsequence may be 5' relative to the second subsequence. Thus, the 3' end of the first subsequence, consisting of the 3' end of the first exon, may be joined to the 5' end of the second subsequence, consisting of the 5' end of the second exon, thereby replicating the junction of a gene fusion. In some embodiments, each nucleotide sequence of a plurality comprises a gene fusion. For example, each nucleotide sequence of the plurality may comprise a first subsequence of a first exon and a second subsequence of a second exon. In certain embodiments, each nucleotide sequence of the plurality comprises a 3' sequence of a different first exon or a 5' sequence of a different second exon.

A nucleotide sequence may comprise an exon upstream (5') of the first exon, wherein the upstream exon and the first exon are consecutive exons in the same gene and the upstream exon and first exon are joined as in a naturally-occurring, mature mRNA of the gene. A nucleotide sequence may comprise an exon downstream (3') of the second exon, wherein the downstream exon and the second exon are consecutive exons in the same gene and the downstream exon and second exon are joined as in a naturally-occurring, mature mRNA of the gene. An upstream exon or downstream exon may be useful, for example, when the first exon or second exon, respectively, is shorter than 200 nucleotides long (such as shorter than 180, 160, 150, 140, 130, 120, 120, or 100 nucleotides long) because short exons may be difficult to identify in the absence of additional sequence of the gene from which the exon originated. For example, a first subsequence may comprise two or more exons, wherein the first exon of the first subsequence is less than 250 nucleotides long (such as less than 200, 190, 180, 170, 160, 150, 140, 130, 120, 110, 100, 90, 80, 70, 60, or 50 nucleotides long), *e.g.,* and the sum of the lengths of the two or more exons is at least 50 nucleotides long (such as at least 60, 70, 80, 90, 100, 110, 120, 130, 140, 150, 160, 170, 180, 190, 200, or 250 nucleotides long). Similarly, a second subsequence may comprise two or more exons, wherein the second exon of the second subsequence is less than 250 nucleotides long (such as less than 200, 190, 180, 170, 160, 150, 140, 130, 120, 110, 100, 90, 80, 70, 60, or 50 nucleotides long), *e.g.,* and the sum of the lengths of the two or more exons is at least 50 nucleotides long (such as at least 60, 70, 80, 90, 100, 110, 120, 130, 140, 150, 160, 170, 180, 190, 200, or 250 nucleotides long).

In some embodiments, a nucleotide sequence of a plurality comprises a gene fusion, complex junction, illegitimate splicing, exon skipping, or complex gene joint. A nucleotide sequence of a plurality may comprise a first subsequence and a second subsequence, wherein the first subsequence comprises a 3' sequence of a first gene and the second subsequence comprises the 5' sequence of a second gene. The first subsequence and second subsequence may be adjoining sequences in the nucleotide sequence, and the first subsequence may be 5' relative to the second subsequence. Thus, the 3' end of the first subsequence, consisting of the 3' end of the first gene, may be joined to the 5' end of the second subsequence, consisting of the 5' end of the second gene, e.g., thereby replicating the junction of a gene fusion. The first gene and second gene may be the same gene or a different gene. In embodiments of the invention wherein the first gene and second gene are the same gene, the first subsequence may occur upstream (5') or downstream (3') of the second subsequence in a genome. The first subsequence and/or second subsequence may be a subsequence of an intron and/or exon.

A nucleic acid may comprise a nucleotide sequence that comprises an intron, *e.g.,* wherein the nucleotide sequence is designed to replicate a gene fusion or illegitimate splicing. A nucleotide sequence that comprises an intron may either be part of the plurality of nucleotide sequences or exist independently of the plurality of nucleotide sequences. A nucleotide sequence that comprises an intron may comprise a first subsequence and a second subsequence, wherein the first subsequence comprises a 3' sequence of a first intron or exon, the second subsequence comprises a 5' sequence of a second intron or exon, and the first subsequence adjoins the second subsequence in the nucleic acid and nucleotide sequence. Either the first subsequence, the second subsequence, or both the first subsequence and the second subsequence may comprise an intron. Either the first subsequence, the second subsequence, or both the first subsequence and the second subsequence may comprise an exon. The first subsequence may occur upstream (5') relative to the second subsequence in the nucleotide sequence and nucleic acid. Because the nucleotide sequence comprises an intron, the full nucleotide sequence may not be capable of being translated into a polypeptide, e.g., because the intron may comprise stop codons or low-efficiency codons in frame with the exons of the nucleotide sequence. The first gene and second gene may be the same gene or different genes.

In some embodiments, a nucleic acid may comprise poly-adenosine, e.g., a 3' poly-adenosine tail (poly-A tail). Either DNA or RNA may comprise poly-adenosine. If DNA comprises poly-adenosine, the DNA may be double-stranded, such that a complementary poly-thymidine sequence is transcribed into mRNA comprising a poly-adenosine tail.

A nucleic acid may be methylated or substantially free of methylated nucleosides. In certain embodiments, a nucleic acid is RNA, and the nucleic acid comprises a 5'-cap. For example, a RNA may comprise 7-methyl guanosine, e.g., in a 5' [m7G(5')ppp(5')G] cap.

In some embodiments, the nucleic acid comprises a promoter, e.g., when the nucleic acid is DNA. A promoter binds to an RNA polymerase, such as SP6 RNA polymerase. A promoter may be a SP6 promoter. The nucleotide sequence of a promoter may be of a different species (*e.g.,* virus, bacteria, yeast) than a nucleotide sequence of a plurality, *e.g.,* for *in vitro* transcription of the plurality of nucleotide sequences, which may be human nucleotide sequences). The nucleotide sequence of a promoter may be of a different species (*e.g*., virus, bacteria, yeast) than each nucleotide sequence of a plurality.

In some embodiments, the nucleic acid is a plasmid, such as a supercoiled plasmid, relaxed circular plasmid, or linear plasmid. In some embodiments, the nucleic acid comprises an origin of replication. The origin of replication may allow for cloning and/or batch-production of the nucleic acid. The origin of replication may be an origin of replication from yeast (*e.g., Saccharomyces cerevisiae)* or bacteria (*e.g., Escherichia coli*), *e.g.,* such that the nucleic acid may be cloned and/or produced in yeast (*e.g., Saccharomyces cerevisiae*) or bacteria *(e.g., Escherichia coli).*

Also disclosed herein is a plurality of nucleic acid fragments, wherein each nucleic acid of the plurality of nucleic acid fragments is a fragment of a full-length nucleic acid as described herein, *supra,* and each nucleotide sequence of the plurality of nucleotide sequences of the full-length nucleic acid is encoded by at least one nucleic acid fragment of the plurality of nucleic acid fragments. A plurality of nucleic acid fragments may be obtained, for example, by processing multiple copies of a single, full-length RNA nucleic acid comprising a plurality of nucleotide sequences, e.g., by transfecting cells with the single, full-length RNA nucleic acid (*e.g.,* by electroporation), fixing the cells (*e.g.,* with formalin), embedding the cells (*e.g.,* in paraffin), and/or extracting nucleic acids (*e.g.,* RNA) from the cells. The processing of a multiple copies of a single, full-length RNA nucleic acid corresponding to one of the nucleic acids described herein, *supra,* may degrade the single, full-length RNA nucleic acid into smaller RNA fragments, e.g., a plurality of nucleic acid fragments. This plurality of nucleic acid fragments may comprise the same plurality of nucleotide sequences as the single RNA nucleic acid, but any given nucleotide sequence of the plurality of nucleotide sequences may occur on different nucleic acid fragments of the plurality of nucleic acid fragments rather than on the same nucleic acid fragment. Next generation sequencing may be used to identify nucleotide sequences that occur across two or more nucleic acid fragments of a plurality of nucleic acid fragments. Thus, the sequencing of a plurality of nucleic acid fragments should identify the same plurality of nucleotide sequences as the sequencing of the single, full-length RNA nucleic acid from which the plurality of nucleic acid fragments originated. A plurality of nucleic acid fragments may be admixed with cellular nucleic acids (*e.g.,* RNA and/or DNA) from cells transfected with the single, full-length RNA nucleic acid and/or untransfected cells (*e.g*., untransfected cells added to a reference material, *see infra*). Thus, a plurality of nucleic acid fragments may be admixed with cellular RNA, such as a transcriptome and/or ribosomal RNA.

In some aspects, the invention relates to a method for making a nucleic acid as described herein. The method may comprise incubating a reaction mixture comprising a DNA template, RNA polymerase, and ribonucleotide triphosphates (*e.g.*, at a temperature at which the RNA polymerase displays polymerase activity), thereby making an RNA nucleic acid. The DNA template may also be a nucleic acid as described herein. The RNA polymerase may be of a different species than the nucleotide sequences of the plurality of nucleotide sequences. For example, the RNA polymerase may be from a virus (*e.g.,* T7 RNA polymerase; SP6 RNA polymerase), bacteria, or yeast and the nucleotide sequences of the plurality of nucleotide sequences may be human. The RNA polymerase may be RNA polymerase II.

Also disclosed herein is a reaction mixture comprising a nucleic acid as described herein, a polymerase, and either ribonucleotide triphosphates or deoxyribonucleotide triphosphates. The polymerase may be a DNA polymerase (*e.g*., for use with deoxyribonucleotide triphosphates) or an RNA polymerase (*e.g.*, for use with ribonucleotide triphosphates). The polymerase may be from a different species than a nucleotide sequence of a plurality. The reaction mixture may comprise an RNAse inhibitor, e.g., from a different species than a nucleotide sequence of a plurality.

A nucleic acid may comprise nucleotide sequences of any origin, such as viral, bacterial, protist, fungal, plant, or animal origin. In certain embodiments, the nucleotide sequences of a plurality are human nucleotide sequences.

In some instances, the disclosure relates to a composition comprising a nucleic acid as described herein and genomic DNA. In certain instances, the ratio of (a) the copy number of a nucleotide sequence corresponding to a gene in the nucleic acid relative to (b) the copy number of the gene in the genomic DNA is about 1:15,000 to about 500:1 in the composition, such as about 1:10,000 to about 1:500, about 1:5,000 to about 500:1, about 1:2,000 to about 500:1, about 1:1,000 to about 500:1, about 1:500 to about 500:1, 1:5,000 to about 100:1, about 1:2,000 to about 100:1, about 1:1,000 to about 100:1, about 1:500 to about 100:1, about 1:250 to about 100:1, about 1:200 to about 100:1, about 1:100 to about 100:1, about 1:50 to about 50:1, about 1:25 to about 25:1, about 1:20 to about 20:1, or about 1:10 to about 10:1 in the composition. In certain instances, the ratio of (a) the copy number of a nucleotide sequence corresponding to a gene in the nucleic acid relative to (b) the copy number of the gene in the genomic DNA is about 6:1, 4:1, about 3:1, about 2:1, about 1:1, about 1:2, about 1:3, about 1:4, or about 1:6 in the composition; in certain embodiments the ratio is about 1:1.

A composition may comprise at least two nucleic acids as described herein, e.g., wherein at least two of the nucleic acids comprise different pluralities of nucleotide sequences. For example, a composition may comprise a plurality of nucleic acids as described herein, wherein 2 to 50, 2 to 40, 2 to 30, 2 to 20, 2 to 10, 2 to 9, 2 to 8, 2 to 7, 2 to 6, 2 to 5, or 2 to 4 nucleic acids of the plurality each comprise different pluralities of nucleotide sequences.

A nucleic acid may comprise nucleotide sequences from genes that occur on different chromosomes. A plurality of nucleotides sequences may comprise nucleotide sequences from genes that occur on 2, 3, 4, 5, 6, 7, 8, 9, or 10 different human chromosomes.

A nucleic acid may comprise the nucleotide sequence set forth in SEQ ID NO:11 or SEQ ID NO: 12. A nucleic acid may comprise a nucleotide sequence having at least about 80%, about 85%, about 90%, about 95%, about 96%, about 97%, about 98%, or 99% sequence identity with the sequence set forth in SEQ ID NO:11 or SEQ ID NO: 12.

### II. GENE FUSIONS FOR ONCOLOGY REFERENCE MATERIALS

The disease or condition may be, for example, a neoplasm, such as cancer. Neoplasms include lung cancer, lymphoid cancer, acute lymphoid leukemia, acute myeloid leukemia, chronic myelogenous leukemia, Burkitt's lymphoma, Hodgkin's lymphoma, plasma cell myeloma, biliary tract cancer, bladder cancer, liver cancer, pancreatic cancer, prostate cancer, skin cancer, thyroid cancer, stomach cancer, large intestine cancer, colon cancer, urinary tract cancer, central nervous system cancer, neuroblastoma, kidney cancer, breast cancer, cervical cancer, testicular cancer, and soft tissue cancer. The disease or condition may be adenocarcinoma, transitional cell carcinoma, breast carcinoma, cervical adenocarcinoma, colon adenocarcinoma, colon adenoma, neuroblastoma, AML, CML, CMML, JMML, ALL, Burkitt's lymphoma, Hodgkin's lymphoma, plasma cell myeloma, hepatocellular carcinoma, large cell lung carcinoma, non-small cell lung carcinoma, squamous cell lung carcinoma, lung neoplasia, ductal adenocarcinoma, endocrine tumor, prostate adenocarcinoma, basal cell skin carcinoma, squamous cell skin carcinoma, melanoma, malignant melanoma, angiosarcoma, leiomyosarcoma, liposarcoma, rhabdomyosarcoma, myxoma, malignant fibrous histiocytoma-pleomorphic sarcoma, stomach adenocarcinoma, germinoma, seminoma, anaplastic carcinoma, follicular carcinoma, papillary carcinoma, or Hurthle cell carcinoma. A nucleotide sequence of a plurality of nucleotide sequences may be associated with a solid tumor. Each nucleotide sequence of a plurality of nucleotide sequences may be associated with a solid tumor.

In some embodiments, a nucleotide sequence of a plurality comprises a subsequence of a gene selected from the group consisting of anaplastic lymphoma receptor tyrosine kinase (ALK), brain-specific angiogenesis inhibitor 1-associated protein 2-like protein 1 (BAIAP2L1), CD74, echinoderm microtubule-associated protein-like 4 (EML4), ETS variant 6 (ETV6), fibroblast growth factor receptor 3 (FGFR3), kinesin-1 heavy chain (KIF5B), nuclear receptor coactivator 4 (NCOA4), nucleophosmin (NPM1), neurotrophic tyrosine receptor kinase 1 (NTRK1), neurotrophic tyrosine receptor kinase 3 (NTRK3), paired box gene 8 (Pax8), peroxisome proliferator-activated receptor gamma (PPARG), RET proto-oncogene (RET), ROS proto-oncogene 1 (ROS1), sodium-dependent phosphate transport protein SLC34A, transforming acidic coiled-coil-containing protein 3 (TACC3), TRK-fused gene (TFG), and tropomyosin 3 (TPM3). In certain embodiments, a nucleotide sequence of the plurality comprises a subsequence of two genes selected from the group consisting of a ALK, BAIAP2L1, CD74, EML4, ETV6, FGFR3, KIF5B, NCOA4, NPM1, NTRK1, NTRK3, Pax8, PPARG, RET, ROS1, SLC34A, TACC3, TFG, and TPM3. For example, a nucleotide sequence of the plurality may consist of a subsequence of EML4 and a subsequence of ALK. Each subsequence may consist of a subsequence from a single exon of any one of the foregoing genes. For example, each nucleotide sequence of the plurality may consist of a subsequence of an exon of EML4 (*e.g.,* a 3' subsequence) and a subsequence of an exon of ALK (*e.g.,* a 5' subsequence).

In some embodiments, each nucleotide sequence of the plurality comprises a subsequence of a gene selected from the group consisting of ALK, BAIAP2L1, CD74, EML4, ETV6, FGFR3, KIF5B, NCOA4, NPM1, NTRK1, NTRK3, Pax8, PPARG, RET, ROS1, SLC34A, TACC3, TFG, and TPM3. In certain embodiments, each nucleotide sequence of the plurality comprises a subsequence of two genes selected from the group consisting of a ALK, BAIAP2L1, CD74, EML4, ETV6, FGFR3, KIF5B, NCOA4, NPM1, NTRK1, NTRK3, Pax8, PPARG, RET, ROS1, SLC34A, TACC3, TFG, and TPM3.

In some embodiments, a nucleotide sequence of the plurality comprises a subsequence of an exon selected from the group consisting of ALK exon 20, BAIAP2L1 exon 2, CD74 exon 6, EML4 exon 13, ETV6 exon 5, FGFR3 exon 18, KIF5B exon 24, NCOA4 exon 8, NPM1 exon 5, NTRK1 exon 10, NTRK3 exon 13, Pax8 exon 8, PPARG exon 1, RET exon 11, RET exon 12, ROS1 exon 34, SLC34A exon 4, TACC3 exon 11, TFG exon 5, and TPM3 exon 8. In certain embodiments, a nucleotide sequence of the plurality comprises a subsequence of two exons selected from the group consisting of a ALK exon 20, BAIAP2L1 exon 2, CD74 exon 6, EML4 exon 13, ETV6 exon 5, FGFR3 exon 18, KIF5B exon 24, NCOA4 exon 8, NPM1 exon 5, NTRK1 exon 10, NTRK3 exon 13, Pax8 exon 8, PPARG exon 1, RET exon 11, RET exon 12, ROS1 exon 34, SLC34A exon 4, TACC3 exon 11, TFG exon 5, and TPM3 exon 8. For example, a nucleotide sequence of the plurality may consist of a subsequence of EML4 exon 13 and a subsequence of ALK exon 20.

In some embodiments, each nucleotide sequence of the plurality comprises a subsequence of an exon selected from the group consisting of ALK exon 20, BAIAP2L1 exon 2, CD74 exon 6, EML4 exon 13, ETV6 exon 5, FGFR3 exon 18, KIF5B exon 24, NCOA4 exon 8, NPM1 exon 5, NTRK1 exon 10, NTRK3 exon 13, Pax8 exon 8, PPARG exon 1, RET exon 11, RET exon 12, ROS1 exon 34, SLC34A exon 4, TACC3 exon 11, TFG exon 5, and TPM3 exon 8. In certain embodiments, each nucleotide sequence of the plurality comprises a subsequence of two exons selected from the group consisting of a ALK exon 20, BAIAP2L1 exon 2, CD74 exon 6, EML4 exon 13, ETV6 exon 5, FGFR3 exon 18, KIF5B exon 24, NCOA4 exon 8, NPM1 exon 5, NTRK1 exon 10, NTRK3 exon 13, Pax8 exon 8, PPARG exon 1, RET exon 11, RET exon 12, ROS1 exon 34, SLC34A exon 4, TACC3 exon 11, TFG exon 5, and TPM3 exon 8.

In some embodiments, a nucleotide sequence of the plurality comprises a subsequence of two exons *(e.g.,* a subsequence of a first exon and a subsequence of a second exon), wherein the first exon and second exon, respectively, are selected from the group consisting of EML4 exon 13 and ALK exon 20; NPM1 exon 5 and ALK exon 20; KIF5B exon 24 and RET exon 11; NCOA4 exon 8 and RET exon 12; CD74 exon 6 and ROS1 exon 34; SLC34A exon 4 and ROS1 exon 34; TPM3 exon 8 and NTRK1 exon 10; TFG exon 5 and NTRK1 exon 10; FGFR3 exon 18 and BAIAP2L1 exon 2; FGFR3 exon 18 and TACC3 exon 11; PAX8 exon 8 and PPARG exon 1; and ETV6 exon 5 and NTRK3 exon 13. In certain embodiments, a subsequence includes the 3' end of the first exon. In certain embodiments, a subsequence includes the 5' end of the second exon.

In some embodiments, each nucleotide sequence of the plurality comprises a subsequence of two exons *(e.g.,* a subsequence of a first exon and a subsequence of a second exon), wherein the first exon and second exon, respectively, are selected from the group consisting of EML4 exon 13 and ALK exon 20; NPM1 exon 5 and ALK exon 20; KIF5B exon 24 and Ret exon 11; NCOA4 exon 8 and RET exon 12; CD74 exon 6 and Ros 1 exon 34; SLC34A exon 4 and Ros 1 exon 34; TPM3 exon 8 and NTRK1 exon 10; TFG exon 5 and NTRK1 exon 10; FGFR3 exon 18 and BAIAP2L1 exon 2; FGFR3 exon 18 and TACC3 exon 11; Pax8 exon 8 and PPARG exon 1; and ETV6 exon 5 and NTRK3 exon 13. In certain embodiments, a subsequence includes the 3' end of the first exon. In certain embodiments, a subsequence includes the 5' end of the second exon.

In some embodiments, a nucleotide sequence of the plurality comprises a spanning subsequence of a nucleotide sequence selected from the group consisting of SEQ ID NO: 1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, and SEQ ID NO: 10; wherein the spanning subsequence comprises a first subsequence (*e.g.,* of a first exon) and a second subsequence (*e.g.,* of a second exon) as described herein. In some embodiments, each nucleotide sequence of the plurality comprises a spanning subsequence of a nucleotide sequence selected from the group consisting of SEQ ID NO: 1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, and SEQ ID NO:10; wherein the spanning subsequence comprises a first subsequence (*e.g.,* of a first exon) and a second subsequence (*e.g.,* of a second exon) as described herein.

A nucleotide sequence of the plurality may comprise the nucleotide sequence set forth in SEQ ID NO: 1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, or SEQ ID NO: 10. Each nucleotide sequence of the plurality may comprise a nucleotide sequence set forth in one of SEQ ID NO: 1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, and SEQ ID NO: 10. A nucleotide sequence of the plurality may comprise a nucleotide sequence with at least about 80%, about 85%, about 90%, about 95%, about 96%, about 97%, about 98%, or 99% sequence identity with the sequence set forth in SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, or SEQ ID NO: 10. Each nucleotide sequence of the plurality may comprise a nucleotide sequence with at least about 80%, about 85%, about 90%, about 95%, about 96%, about 97%, about 98%, or 99% sequence identity with a sequence set forth in SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, or SEQ ID NO: 10.

In some embodiments, each nucleotide sequence of the plurality comprises a subsequence of a first exon and a second exon, wherein the first exon and second exon, respectively, are selected from the group consisting of an exon of ACBD6 and RRP15; ACSL3 and ETV1; ACTB and GLI1; AGPAT5 and MCPH1; AGTRAP and BRAF; AKAP9 and BRAF; ARFIP1 and FHDC1; ARID1A and MAST2; ASPSCR1 and TFE3; ATG4C and FBXO38; ATIC and ALK; BBS9 and PKD1L1; BCR and ABL1; BCR and JAK2; BRD3 and NUTM1; BRD4 and NUTM1; C2orf44 and ALK; CANT1 and ETV4; CARS and ALK; CCDC6 and RET; CD74 and NRG1; CD74 and ROS1; CDH11 and USP6; CDKN2D and WDFY2; CEP89 and BRAF; CHCHD7 and PLAG1; CIC and DUX4L1; CIC and FOXO4; CLCN6 and BRAF; CLIP1 and ROS1; CLTC and ALK; CLTC and TFE3; CNBP and USP6; COL1A1 and PDGFB; COL1A1 and USP6; COL1A2 and PLAG1; CRTC1 and MAML2; CRTC3 and MAML2; CTAGE5 and SIP1; CTNNB1 and PLAG1; DCTN1 and ALK; DDX5 and ETV4; DNAJB1 and PRKACA; EIF3E and RSPO2; EIF3K and CYP39A1; EML4 and ALK; EPC1 and PHF1; ERC1 and RET; ERC1 and ROS1; ERO1L and FERMT2; ESRP1 and RAF1; ETV6 and ITPR2; ETV6 and JAK2; ETV6 and NTRK3; EWSR1 and ATF1; EWSR1 and CREB1; EWSR1 and DDIT3; EWSR1 and ERG; EWSR1 and ETV1; EWSR1 and ETV4; EWSR1 and FEV; EWSR1 and FLI1; EWSR1 and NFATC1; EWSR1 and NFATC2; EWSR1 and NR4A3; EWSR1 and PATZ1; EWSR1 and PBX1; EWSR1 and POU5F1; EWSR1 and SMARCA5; EWSR1 and SP3; EWSR1 and WT1; EWSR1 and YY1; EWSR1 and ZNF384; EWSR1 and ZNF444; EZR and ROS1; FAM131B and BRAF; FBXL18 and RNF216; FCHSD1 and BRAF; FGFR1 and ZNF703; FGFR1 and PLAG1; FGFR1 and TACC1; FGFR3 and BAIAP2L1; FGFR3 and TACC3; FN1 and ALK; FUS and ATF1; FUS and CREB3L1; FUS and CREB3L2; FUS and DDIT3; FUS and ERG; FUS and FEV; GATM and BRAF; GMDS and PDE8B; GNAI1 and BRAF; GOLGA5 and RET; GOPC and ROS1; GPBP1L1 and MAST2; HACL1 and RAF1; HAS2 and PLAG1; HERPUD1 and BRAF; HEY1 and NCOA2; HIP1 and ALK; HLA-A and ROS1; HMGA2 and ALDH2; HMGA2 and CCNB1IP1; HMGA2 and COX6C; HMGA2 and EBF1; HMGA2 and FHIT; HMGA2 and LHFP; HMGA2 and LPP; HMGA2 and NFIB; HMGA2 and RAD51B; HMGA2 and WIF1; HN1 and USH1G; HNRNPA2B1 and ETV1; HOOK3 and RET; IL6R and ATP8B2; INTS4 and GAB2; IRF2BP2 and CDX1; JAZF1 and PHF1; JAZF1 and SUZ12; KIAA1549 and BRAF; KIAA1598 and ROS1; KIF5B and ALK; KIF5B and RET; KLC1 and ALK; KLK2 and ETV1; KLK2 and ETV4; KMT2A and ABI1; KMT2A and ABI2; KMT2A and ACTN4; KMT2A and AFF1; KMT2A and AFF3; KMT2A and AFF4; KMT2A and ARHGAP26; KMT2A and ARHGEF12; KMT2A and BTBD18; KMT2A and CASC5; KMT2A and CASP8AP2; KMT2A and CBL; KMT2A and CREBBP; KMT2A and CT45A2; KMT2A and DAB2IP; KMT2A and EEFSEC; KMT2A and ELL; KMT2A and EP300; KMT2A and EPS15; KMT2A and FOXO3; KMT2A and FOXO4; KMT2A and FRYL; KMT2A and GAS7; KMT2A and GMPS; KMT2A and GPHN; KMT2A and KIAA0284; KMT2A and KIAA1524; KMT2A and LASP1; KMT2A and LPP; KMT2A and MAPRE1; KMT2A and MLLT1; KMT2A and MLLT10; KMT2A and MLLT11; KMT2A and MLLT3; KMT2A and MLLT4; KMT2A and MLLT6; KMT2A and MYO1F; KMT2A and NCKIPSD; KMT2A and NRIP3; KMT2A and PDS5A; KMT2A and PICALM; KMT2A and PRRC1; KMT2A and SARNP; KMT2A and SEPT2; KMT2A and SEPT5; KMT2A and SEPT6; KMT2A and SEPT9; KMT2A and SH3GL1; KMT2A and SORBS2; KMT2A and TET1; KMT2A and TOP3A; KMT2A and ZFYVE19; KTN1 and RET; LIFR and PLAG1; LMNA and NTRK1; LRIG3 and ROS1; LSM14A and BRAF; MARK4 and ERCC2; MBOAT2 and PRKCE; MBTD1 and CXorf67; MEAF6 and PHF1; MKRN1 and BRAF; MSN and ALK; MYB and NFIB; MYO5A and ROS1; NAB2 and STAT6; NACC2 and NTRK2; NCOA4 and RET; NDRG1 and ERG; NF1 and ACCN1; NFIA and EHF; NFIX and MAST1; NONO and TFE3; NOTCH1 and GABBR2; NPM1 and ALK; NTN1 and ACLY; NUP107 and LGR5; OMD and USP6; PAX3 and FOXO1; PAX3 and NCOA1; PAX3 and NCOA2; PAX5 and JAK2; PAX7 and FOXO1; PAX8 and PPARG; PCM1 and JAK2; PCM1 and RET; PLA2R1 and RBMS1; PLXND1 and TMCC1; PPFIBP1 and ALK; PPFIBP1 and ROS1; PRCC and TFE3; PRKAR1A and RET; PTPRK and RSPO3; PWWP2A and ROS1; QKI and NTRK2; RAF1 and DAZL; RANBP2 and ALK; RBM14 and PACS1; RGS22 and SYCP1; RNF130 and BRAF; SDC4 and ROS1; SEC16A_NM_014866.1 and NOTCH1; SEC31A and ALK; SEC31A and JAK2; SEPT8 and AFF4; SFPQ and TFE3; SLC22A1 and CUTA; SLC26A6 and PRKAR2A; SLC34A2 and ROS1; SLC45A3 and BRAF; SLC45A3 and ELK4; SLC45A3 and ERG; SLC45A3 and ETV1; SLC45A3 and ETV5; SND1 and BRAF; SQSTM1 and ALK; SRGAP3 and RAF1; SS18 and SSX1; SS18 and SSX2; SS18 and SSX4; SS18L1 and SSX1; SSBP2 and JAK2; SSH2 and SUZ12; STIL and TALI; STRN and ALK; SUSD1 and ROD1; TADA2A and MAST1; TAF15 and NR4A3; TCEA1 and PLAG1; TCF12 and NR4A3; TCF3 and PBX1; TECTA and TBCEL; TFG and ALK; TFG and NR4A3; TFG and NTRK1; THRAP3 and USP6; TMPRSS2 and ERG; TMPRSS2 and ETV1; TMPRSS2 and ETV4; TMPRSS2 and ETV5; TP53 and NTRK1; TPM3 and ALK; TPM3 and NTRK1; TPM3 and ROS1; TPM3 and ROS1; TPM4 and ALK; TRIM24 and RET; TRIM27 and RET; TRIM33 and RET; UBE2L3 and KRAS; VCL and ALK; VTI1A and TCF7L2; YWHAE and FAM22A; YWHAE and NUTM2B; ZC3H7B and BCOR; ZCCHC8 and ROS1; ZNF700 and MAST1; and ZSCAN30 and BRAF. Gene fusions of the foregoing gene pairs that correlate with cancer may be identified, for example, in the Catalogue of Somatic Mutations in Cancer (COSMIC) database (http://cancer.sanger.ac.uk/cosmic/fusion). Each of the gene pairs described in this paragraph correspond to a gene fusion listed in the COSMIC database, which has been identified as being associated with cancer. The COSMIC database may be used to identify synonyms for the gene names as well as the nucleotide sequences of the genes and gene fusions Other databases exist that curate gene fusions associated with cancer, e.g. FusionCancer (http://donglab.ecnu.edu.cn/databases/FusionCancer/index.html) and the databases from which ArcherDx draws (http://archerdx.com/software/quiver), and the nucleotide sequences of a plurality may be selected from any of the gene fusions listed in these databases.

### III. COMPOSITIONS COMPRISING A PLURALITY OF NUCLEIC ACID FRAGMENTS

A single, multiplexed nucleic acid, however, may fragment and/or degrade during manufacturing, storage, and/or processing. A multiplexed nucleic acid comprising multiple different nucleotide sequences presents many advantages for preparing reference materials. Fragmentation and/or degradation does not necessarily affect the performance of a reference material, however, because next generation sequencing strategies assemble relatively long nucleotide sequences from relatively short nucleic acids. Further, the fragmentation and/or degradation of a single, multiplexed nucleic acid may be desirable, for example, because shorter nucleic acids more closely replicate the mRNAs of a transcriptome after it has been extracted from a cell.

In some instances, the disclosure relates to a composition comprising a plurality of nucleic acid fragments. Sequence assembly of the nucleotide sequences of the plurality of nucleic acid fragments may result in the complete nucleotide sequence of a full-length nucleic acid as described in sections I and II, *supra.* The term "sequence assembly" refers to the alignment and merging of the nucleotide sequences of a plurality of nucleic acid fragments into longer nucleotide sequences in order to reconstruct the original nucleotide sequence (*see, e.g.,* El-Metwally, S. et al., PLoS Computational Biology 9(12): e1003345 (2013); Nagarajan, N. and M. Pop, Nature Reviews Genetics 14(3): 157 (2013); Paszkiewicz, K. and D.J. Studholme, Briefings Bioinformatics 11(5):457 (2010)). Sequence assembly of the nucleotide sequences of a plurality of nucleic acid fragments may result in less than the complete nucleotide sequence of a full-length nucleic acid so long as each nucleotide sequence of the plurality of nucleotide sequences of the full-length nucleic acid *(e.g.,* as described in sections I and II) is encoded by at least one nucleic acid fragment of the plurality of nucleic acids. For example, sequence assembly of the nucleotide sequences of the nucleic acid fragments of the plurality may result in assembled sequences that align with at least 50%, 60%, 70%, 80%, 90%, 95%, 96%, 97%, 98%, or 99% of the nucleotide sequence of the full-length nucleic acid. Omitted nucleotide sequences may include, for example, unstable nucleotide sequences and/or specific nucleotide sequences that are intentionally depleted or otherwise selected against (*e.g*., during a hybridization or amplification step).

A plurality of nucleic acid fragments may be produced from a full-length nucleic acid as described in sections I and II, *supra* (*e.g.,* the plurality of nucleic acid fragments may be produced from a number of copies of the same full-length nucleic acid). The plurality of nucleic acid fragments may consist of fragments or degradation products of a full-length nucleic acid as described in sections I and II, *supra* (*e.g.,* the plurality of nucleic acid fragments may consist of fragments or degradation products from a number of copies of the same full-length nucleic acid).

Each nucleotide sequence of a plurality of nucleotide sequences of a full-length nucleic acid as described in sections I and II, *supra,* may be encoded by at least one nucleic acid fragment of a plurality of nucleic acid fragments.

Different copies of the same nucleic acid may be fragmented/degraded in many different ways, and thus, a plurality of nucleic acid fragments may or may not comprise identical nucleic acid fragments. Further, portions of individual nucleic acids may be lost, for example, during a purification step, or degraded to a length that lacks sequenceable content. Nevertheless, next generation sequencing can reassemble the nucleotide sequence of the original, unfragmented, full-length nucleic acid from the plurality of nucleic acid fragments so long as the plurality of nucleic acid fragments contains sufficient redundancy. For example, the plurality of nucleic acid fragments may comprise about 2x to about 1,000,000x coverage of the nucleotide sequence of an original, unfragmented, full-length nucleic acid, such as about 10x to about 100,000x, about 20x to about 50,000x, about 100x to about 10,000x, or about 100x to about 1000x coverage. Thus, the nucleotide sequence of the original, unfragmented, full-length nucleic acid may be identified by sequencing the plurality of nucleic acid fragments by next generation sequencing.

The plurality of nucleic acid fragments may comprise about 2x to about 1,000,000x coverage of each nucleotide sequence of the plurality of nucleotide sequences of an original, unfragmented, full-length nucleic acid, such as about 10x to about 100,000x, about 20x to about 50,000x, about 100x to about 10,000x, or about 100x to about 1000x coverage. Thus, each nucleotide sequence of the plurality of nucleotide sequences of the original, unfragmented, full-length nucleic acid may be identified by sequencing the plurality of nucleic acid fragments by next generation sequencing.

A composition comprising a plurality of nucleic acid fragments may further comprise substantially all of the transcriptome of a cell. The ratio of the nucleotide sequence of the original, unfragmented, full-length nucleic acid (*e.g.,* the nucleic acid from which the plurality of nucleic acid fragments originated) to a single copy of the transcriptome of the cell may be about 1:10 to about 1000:1, such as about 1:5 to about 500:1, about 1:3 to about 300:1, about 1:2 to about 200:1, or about 1:1 to about 100:1 in the composition. The ratio of each copy of a nucleotide sequence of a plurality of nucleotide sequences of the original, unfragmented, full-length nucleic acid (*e.g.,* the nucleic acid from which the plurality of nucleic acid fragments originated) to a single copy of the transcriptome of the cell may be about 1:10 to about 1000:1, such as about 1:5 to about 500:1, about 1:3 to about 300:1, about 1:2 to about 200:1, or about 1:1 to about 100:1 in the composition. "A single copy of a transcriptome of a cell" refers to all of the mRNA of a single cell, which may contain multiple copies of the same mRNA.

A composition comprising a plurality of nucleic acid fragments may further comprise a cell. The cell may be the cell of the transcriptome, *supra, i.e.,* the composition may comprise substantially all of a transcriptome of a cell because the composition comprises a cell. The cell may be a human cell. The cell may be a fibroblast or a lymphocyte, such as an immortalized B lymphocyte. The cell may be GM24385. The cell may be any of the cells described herein, *infra.*

In some embodiments, the composition may comprise a plurality of cells. The plurality of cells may comprise the cell, *supra, e.g.,* wherein the transcriptome of the composition is the transcriptome of the cell. Each cell of a plurality of cells may comprise substantially the same genome. "Substantially the same genome" refers to genomes from the same individual (*e.g*., person), from the same parent cell, or from the same cell line, which may contain slight differences, such as small epigenetic differences, spontaneous mutations, and mutations arising from processing, such as transfection and cell-fixation (*e.g*., which may affect the integrity of cellular DNA).

The plurality of nucleic acid fragments of a composition may be intracellular nucleic acid fragments, e.g., the plurality of nucleic acid fragments may exist intracellularly, for example, in the cytoplasm and/or nucleus of a cell. The plurality of cells may comprise the plurality of nucleic acid fragments of the composition. The plurality of nucleic acid fragments may have been introduced into cells of the composition (*e.g.*, a plurality of cells) by transfection. "Transfection" refers to the introduction of exogenous material into a cell, and the term includes the introduction of exogenous nucleic acids by transformation, transfection, infection (*e.g*., with a recombinant virus), and electroporation, as well as other known methods. A full-length nucleic acid as described in sections I and II, *supra,* may be introduced into cells of the composition by transfection, and the full-length nucleic acid may be fragmented and/or degraded into the plurality of nucleic acid fragments during transfection or after transfection, thereby generating the plurality of nucleic acid fragments.

In some embodiments, each cell of the plurality of cells is fixed. Methods for fixing cells are described herein, *infra,* and include formalin-fixation. In some embodiments, the cells of the composition are embedded in paraffin.

In some embodiments, the composition does not comprise cells. For example, the composition may simply comprise a plurality of nucleic acid fragments generated from a full-length nucleic acid described in sections I and II, *supra.* The composition may comprise nucleic acids extracted from cells described in the preceding paragraphs, e.g., the plurality of nucleic acid fragments may be extracted from a plurality of cells as described in the preceding paragraphs, e.g., along with the transcriptome and/or genomes of the plurality of cells. Thus, the plurality of nucleic acid fragments may have been extracted from a cell or from a plurality of cells.

The composition may further comprise urea (*e.g.,* 100 mM to 8 M urea), guanidine (*e.g.,* 100 mM to 6 M guanidine), an RNAse inhibitor, a metal chelator *(e.g.,* ethylenediaminetetraacetate), a protease (*e.g.,* proteinase K), a DNAse (*e.g.,* DNAse I), ethanol (*e.g.,* 10-99% ethanol), isopropanol (*e.g.,* 10-99% isopropanol), and/or a reverse transcriptase. Methods of extracting and purifying RNA from cells using the foregoing reagents are well known. The plurality of nucleic acid fragments may be associated with a solid support, such as beads (e.g. magnetic beads), to assist in purification.

### IV. CELLS

In some aspects, the invention relates to a cell comprising a nucleic acid as described herein. In some embodiments, the invention relates to a plurality of cells comprising a nucleic acid as described herein. A nucleic acid of the invention may be integrated into the genome of a cell, or it may be present on a plasmid or as a linear nucleic acid, such as mRNA or a linear plasmid. For example, a cell may comprise a nucleic acid as described herein, *supra,* wherein the nucleic acid is a single-stranded RNA.

A cell may comprise at least two nucleic acids as described herein, e.g., wherein at least two of the nucleic acids comprise different pluralities of nucleotide sequences. For example, a cell may comprise a plurality of nucleic acid fragments as described herein, wherein 2 to 50, 2 to 40, 2 to 30, 2 to 20, 2 to 10, 2 to 9, 2 to 8, 2 to 7, 2 to 6, 2 to 5, or 2 to 4 nucleic acid fragments of the plurality each comprise different pluralities of nucleotide sequences.

A cell may comprise more than one copy of the same nucleic acid. For example, a cell may comprise 1, 2, 3, 4, 5, 6, 7, 8, 9, 10, 15, 20, 25, 30, 35, 40, 45, 50, 60, 70, 80, 90, 100, 120, 150, or 200 copies of the same nucleic acid. A cell may comprise at least 1, 2, 3, 4, 5, 6, 7, 8, 9, 10, 15, 20, 25, 30, 35, 40, 45, 50, 60, 70, 80, 90, 100, 120, 150, or 200 copies of the same nucleic acid. A cell may comprise 1 to 1000, 2 to 1000, 5 to 1000, 10 to 1000, 20 to 1000, 50 to 1000, 100 to 1000, 150 to 1000, 200 to 1000, 250 to 1000, 1 to 500, 2 to 500, 5 to 500, 10 to 500, 20 to 1000, 50 to 500, 100 to 500, 150 to 500, or 200 to 500, 250 to 500, 1 to 400, 2 to 400, 5 to 400, 10 to 400, 20 to 400, 50 to 400, 100 to 400, 150 to 400, 200 to 400, or 250 to 400 copies of the same nucleic acid.

A nucleic acid may become fragmented or otherwise degrade before, during, or after transfection of the nucleic acid into a cell. Accordingly, in some embodiments, a cell may comprise a plurality of nucleic acid fragments (*e.g.*, that are either fragments of a single, full-length nucleic acid as described herein, *supra,* or fragments of multiple copies of a single, full-length nucleic acid as described herein, *supra*)*.* The plurality of nucleic acid fragments may be admixed with the nucleic acids of the cell, *e.g.,* cytosolic and/or nuclear nucleic acids. The cell may comprise multiple copies of each nucleotide sequence of the plurality of nucleotide sequences, such as 1 to 1000, 2 to 1000, 5 to 1000, 10 to 1000, 20 to 1000, 50 to 1000, 100 to 1000, 150 to 1000, 200 to 1000, 250 to 1000, 1 to 500, 2 to 500, 5 to 500, 10 to 500, 20 to 1000, 50 to 500, 100 to 500, 150 to 500, or 200 to 500, 250 to 500, 1 to 400, 2 to 400, 5 to 400, 10 to 400, 20 to 400, 50 to 400, 100 to 400, 150 to 400, 200 to 400, or 250 to 400 copies of each nucleotide sequence. A cell may comprise 1, 2, 3, 4, 5, 6, 7, 8, 9, 10, 15, 20, 25, 30, 35, 40, 45, 50, 60, 70, 80, 90, 100, 120, 150, or 200 copies of each nucleotide sequence of a plurality of nucleotide sequences as described herein, *supra.* A cell may comprise at least 1, 2, 3, 4, 5, 6, 7, 8, 9, 10, 15, 20, 25, 30, 35, 40, 45, 50, 60, 70, 80, 90, 100, 120, 150, or 200 copies of each nucleotide sequence of a plurality of nucleotide sequences as described herein, *supra.* Each nucleotide sequence of a plurality of nucleotide sequences that originates from the same full-length nucleic acid may be present in a plurality of nucleic acid fragments at approximately the same copy number. Some nucleotide sequences are more or less stable than other nucleotide sequences, however, and thus, a cell may contain different nucleotide sequences of a plurality of nucleotide sequences at different copy numbers. A copy of a nucleotide sequence may occur, for example, on a single nucleic acid fragment of the plurality of nucleic acid fragments.

A cell may be a human cell. A cell may be a fibroblast or lymphocyte. A cell may be the cell of a cell line. A cell may be an adherent cell or a suspension cell.

A cell may be selected from the group consisting of 721, 293T, 721, A172, A253, A2780, A2780ADR, A2780cis, A431, A-549, BCP-1 cells, BEAS-2B, BR 293, BxPC3, Cal-27, CML T1, COR-L23, COR-L23/5010, COR-L23/CPR, COR-L23/R23, COV-434, DU145, DuCaP, EM2, EM3, FM3, H1299, H69, HCA2, HEK-293, HeLa, HL-60, HMEpC, HT-29, HUVEC, Jurkat, JY cells, K562 cells, KBM-7 cells, KCL22, KG1, Ku812, KYO1, LNCap, Ma-Mel, MCF-10A, MCF-7, MDA-MB-157, MDA-MB-231, MDA-MB-361, MG63, MONO-MAC 6, MOR/0.2R, MRC5, NCI-H69/CPR, NCI-H69/LX10, NCI-H69/LX20, NCI-H69/LX4, Peer, Raji, Saos-2 cells, SiHa, SKBR3, SKOV-3, T2, T-47D, T84, U373, U87, U937, VCaP, WM39, WT-49, and YAR cells.

A cell may be any cell available from the ATCC (*e.g.*, http://www.atcc.org). In certain embodiments, the cell is a mammalian cell, such as a human cell. The cell may be a cell from any of the National Institute of General Medical Sciences (NIGMS) Human Genetic Cell Repository cell lines available from the Coriell Institute for Medical Research (https://catalog.coriell.org/1/NIGMS), such as a cell line from the "Apparently Healthy" collection. The cell may be may be a fibroblast, lymphoblast, or lymphocyte. The cell may be transformed, e.g., with Epstein-Barr virus. The cell may be an immortalized cell. For example, the cell may be an immortalized lymphocyte, such as an immortalized B lymphocyte. The cell may be an Epstein-Barr virus-transformed lymphocyte, such as an Epstein-Barr virus-transformed B lymphocyte. The cell may be GM12878 (*see* Zook, J.M. et al., Nature Biotechnology 32:246 (2014)). The cell may be GM12878, GM24149, GM24143, GM24385, GM24631, GM24694, or GM24695 (*see* Zook, J.M. et al., Scientific Data 3:160025 (2016)). In certain embodiments, the cell is GM24385.

A cell may be a bacterial, yeast, insect, mouse, rat, hamster, dog, or monkey cell, *e.g.,* for cloning or validating a construct. For example, the cell may be *E. coli* or *Saccharomyces cerevisiae, e.g.,* for cloning a nucleic acid of the invention.

In some aspects, the invention relates to composition comprising a first plurality of cells and a second plurality of cells (referred to as a "composition comprising cells"). The first plurality of cells may comprise either a full-length nucleic acid as described herein, *supra,* or a plurality of nucleic acid fragments, e.g., wherein sequence assembly of the nucleotide sequences of the plurality of nucleic acid fragments results in nucleotide sequences(s) that taken together comprise a plurality of nucleotide sequences as described herein, *supra.* The second plurality of cells may consist of cells that do not comprise either a full-length nucleic acid or plurality of nucleic acid fragments as described herein. The first plurality of cells and second plurality of cells may be the same type of cells, *e.g.,* the cells of the first and second pluralities may be human cells, such as immortalized lymphocytes, such as GM24385 cells. The cells of the first plurality and the second plurality may be admixed in the composition. The ratio of the number of cells of the first plurality to the number of cells of the second plurality may be about 1:1 to about 1:10,000, such as about 1:2 to about 1:2000, or about 1:10 to about 1:1000 in the composition. The ratio may depend in part on either the average copy number of the nucleic acid in the first plurality of cells or the average copy number of the nucleotide sequences of the plurality of nucleotide sequences in the first plurality of cells. The ratio of the number of cells of the first plurality of cells to the number of cells of the second plurality of cells may be adjusted, for example, such that the composition comprises about 0.01 copies of the nucleic acid (or about 0.01 copies of each nucleotide sequence of the plurality of nucleotide sequences) to about 100 copies of the nucleic acid (or about 100 copies of each nucleotide sequence of the plurality of nucleotide sequences) per cell of the composition. The ratio may be adjusted such that the composition comprises about 0.1 to about 50 copies, about 0.5 to about 20 copies, or about 1 to about 10 copies of the nucleic acid per cell of the composition (or about 0.1 to about 50 copies, about 0.5 to about 20 copies, or about 1 to about 10 copies of each nucleotide sequence of the plurality of nucleotide sequences per cell of the composition).

A cell, plurality of cells, or composition comprising cells may be fixed. In certain embodiments, a cell, plurality of cells, or composition comprising cells is fixed with formalin. A cell, plurality of cells, or composition comprising cells may be fixed with glutaraldehyde, ethanol, methanol, acetone, methyl benzoate, xylene, acetic acid, picrate, HOPE fixative, osmium tetroxide, and/or uranyl acetate.

A cell, plurality of cells, or composition comprising cells may be dehydrated, e.g., using ethanol or an organic solvent.

A cell, plurality of cells, or composition comprising cells may be embedded in paraffin. For example, a cell, plurality of cells, or composition comprising cells may be fixed in formalin and embedded in paraffin. A cell, plurality of cells, or composition comprising cells may be mounted on a slide.

In some aspects, the invention relates to a paraffin section comprising a plurality of cells or composition comprising cells. The paraffin section may comprise 1 to about 1,000,000 cells, such as about 10 to about 100,000 cells, about 50 to about 50,000 cells, about 100 to about 10,000 cells, about 500 to about 5,000 cells, about 200 to about 2000 cells, about 100 to about 1000 cells, or about 50 to about 1000 cells. The paraffin section may be about 1 µm to about 50 µm thick, such as about 2 µm to about 25 µm thick, or about 5 µm to about 20 µm thick. The paraffin section may be about 2, 3, 4, 5, 6, 7, 8, 9, 10, 11, 12, 13, 14, 15, 16, 17, 18, 19, or 20 µm thick. The paraffin section may be about 1 mm to about 100 mm in length, width, or diameter, such as about 5 mm to about 50 mm, or about 10 mm to about 40 mm. For example, a paraffin section may be about 5 mm to about 50 mm in length, about 5 mm to about 50 mm in width, and about 5 µm to about 20 µm thick. A paraffin section may be about 5 mm to about 50 mm in diameter and about 5 µm to about 20 µm thick.

A cell, plurality of cells, or composition comprising cells may be present in a cell pellet. A cell, plurality of cells, or composition comprising cells may be suspended in blood plasma, such as a mammalian blood plasma. In certain instances, a cell, plurality of cells, or composition comprising cells may be suspended in human blood plasma or a solution designed to replicate human blood plasma.

In some aspects, the invention relates to a method for making a biological reference material, comprising transfecting a plurality of cells with a nucleic acid described herein, a plurality thereof, or a plurality of nucleic acid fragments as described herein.

A method may comprise fixing a plurality of cells or composition comprising cells. For example, the method may comprise fixing a plurality of cells or composition comprising cells with formalin. A method may comprise fixing a plurality of cells or composition comprising cells with glutaraldehyde, ethanol, methanol, acetone, methyl benzoate, xylene, acetic acid, picrate, HOPE fixative, osmium tetroxide, and/or uranyl acetate.

A method may comprise embedding a plurality of cells or composition comprising cells in paraffin. A method may comprise sectioning paraffin-embedded cells. A method may comprise mounting a plurality of cells on a slide, e.g., paraffin-embedded cells or cells that are not embedded in paraffin.

A method may comprise mounting a plurality of cells or composition comprising cells on a slide.

In some aspects, the invention relates to a biological reference material comprising a cell, plurality of cells, or composition comprising cells as described herein.

A biological reference material may further comprise paraffin, e.g., wherein the cell, plurality of cells, or composition comprising cells are fixed, and the cell, plurality of cells, or composition comprising cells are embedded in the paraffin

A biological reference material may further comprise untransfected cells, e.g., wherein the untransfected cells do not comprise the nucleic acid. In certain embodiments, the untransfected cells are the same species as the cells of the plurality, *e.g.,* the untransfected cells may be from the same source (*e.g.,* cell line) as the cells of the plurality. The ratio of cells of the plurality of cells to untransfected cells may be about 4:1 to about 1:10,000, such as about 1:1 to about 1:5,000, about 1:1 to about 1:1000, about 1:10 to about 1:1000, or about 1:50 to about 1:500. The ratio of cells of the plurality of cells to untransfected cells may be about 45:55, about 50:50, about 55:45, about 1:1, about 1:2, about 1:3, about 1:4, about 1:5, about 1:6, about 1:7, about 1:8, about 1:9, about 1:10, about 1:20, about 1:25, about 1:50, about 1:100, about 1:200, about 1:250, about 1:500, or about 1:1000.

In some embodiments, the ratio of the copy number of the nucleic acid to the copy number of cell genomes in the biological reference material is about 10:1 to about 1:10,000, such as about 5:1 to about 1:1000, about 2:1 to about 1:100, about 1:1 to about 1:50, or about 1:2 to about 1:20. In general, each genome contains two copies of a gene (*e.g.,* for genes occurring on diploid chromosomes, such as autosomes). The copy number of a nucleic acid to the copy number of a gene in the cell genome in the biological reference material may be about 10:1 to about 1:10,000, such as about 5:1 to about 1:1000, about 1:1 to about 1:100, about 1:2 to about 1:50, or about 1:4 to about 1:40. Thus, the ratio of a genotype of a nucleic acid to the copy number of a gene in the cell genome that is associated with the genotype (*e.g.,* the wild type allele) in the biological reference material may be about 10:1 to about 1:10,000, such as about 5:1 to about 1:1000, about 1:1 to about 1:100, about 1:2 to about 1:50, or about 1:4 to about 1:40.

A biological reference material may further comprise a liquid, such as saline, phosphate-buffered saline, or blood plasma, such as a mammalian blood plasma. A cell, plurality of cells, or composition comprising cells of a biological reference material may be suspended in plasma, such as human blood plasma or a solution designed to replicate human blood plasma.

A biological reference material may be a cell pellet, *e.g.,* made by centrifuging a plurality of cells or composition comprising cells as described herein.

In some aspects, the invention relates to a composition comprising a purified nucleic acid, wherein the purified nucleic acid is isolated from a biological reference material as described herein. The composition may comprise a buffer, such as tris buffer (*i.e.,* tris(hydroxymethyl)aminomethane or a salt thereof). The composition may comprise a chelating agent, such as ethylenediaminetetraacetic acid, or a salt thereof. The composition may comprise trace amounts of formaldehyde and/or paraffin, although the composition may be free of formaldehyde and paraffin.

### EXEMPLIFICATION

### Example 1. Nucleic acid design for oncology targets

A list of gene fusion targets was developed that represents clinically relevant fusions for which diagnostic testing using next generation sequencing (NGS) technology is currently available (Table 1). The targets were selected based on the availability of assays to detect the fusions as well as a review of literature indicating clinical relevance. The list favored mutations in lung and thyroid cancers. Details about the exact sequences included are given in Table 2.

**Table 1. Oncology Gene Fusion targets for Reference Materials**

| | **RNA Fusion** | **Primary Cancer Tissue** | **5' Partner-Exon #** | **3' Partner-Exon #** |
|---|---|---|---|---|
| 1 | **EML4-ALK** | **Lung** | **EML4 Exon 13** | **ALK Exon 20** |
| 2 | **NPM1-ALK** | **Lymphoid** | **NPM1 exon 5** | **ALK Exon 20** |
| 3 | **KIF5B-RET** | **Lung** | **KIF5B Exon 24** | **Ret Exon 11** |
| 4 | **NCOA4-RET** | **Thyroid** | **NCOA4 Exon 8** | **RET exon 12** |
| 5 | **CD74-ROS1** | **Lung** | **CD74 Exon 6** | **Ros 1 Exon 34** |
| 6 | **SLC34A-ROS1** | **Lung, Stomach** | **SLC34A Exon 4** | **Ros 1 Exon 34** |
| 7 | **TPM3-NTRK1** | **Lung, Large Intestine** | **TPM3 Exon 8** | **NTRK1 Exon 10** |
| 8 | **TFG-NTRK1** | **Thyroid (rare)** | **TFG Exon 5** | **NTRK1 Exon 10** |
| 9 | **FGFR3-BAIAP2L1** | **Urinary tract (rare)** | **FGFR3 Exon 18** | **BAIAP2L1 Exon 2** |
| 10 | **FGFR3-TACC3** | **Urinary tract, CNS** | **FGFR3 exon 18** | **TACC3 Exon 11** |
| 11 | **PAX-PPARG** | **Thyroid** | **Pax8 Exon 8** | **PPARG Exon 1** |
| 12 | **ETV6-NTRK3** | **Kidney, Breast, Soft Tissue** | **ETV6 Exon 5** | **NTRK3 Exon 13** |

**Table 2. GenBank sequences used to design the multiplex fusion constructs.**

| | **Fusion** | **GenBank Accession for Fusion Sequences** | **SEQ ID NO** |
|---|---|---|---|
| *1* | *EML4-ALK* | AB274722.1 | 1 |
| *2* | *NPM1-ALK* | U04946.1 | 2 |
| *3* | *KIF5B-RET* | AB795257.1 | 3 |
| *4* | *NCOA4-RET* | S71225.1 | 4 |
| *5* | *CD74-ROS1* | EU236945.1 | 5 |
| *6* | *SLC34A2-ROS1* | EU236947.1 | 6 |
| *7* | *TPM3-NTRK1* | X03541.1 | 7 |
| *8* | *TFG-NTRK1* | X85960.1 | 8 |
| *9* | *FGFR3-BAIAP2L1* | - | |
| *10* | *FGFR3-TACC3* | - | |
| *11* | *PAX8-PPARG* | AR526805.1 | 9 |
| *12* | *ETV6-NTRK3* | AF041811.2 | 10 |

Two different plasmid DNA constructs were designed such that each plasmid contained 6 of the 12 fusion targets. All the even numbered lines in Table 1 were incorporated in construct #1 (SEQ ID NO:11) and all the odd numbered lines in Table 1 were incorporated into construct #2 (SEQ ID NO:12).

Table 1 includes two fusions for ALK, two fusions for RET, two fusions for ROS1, two fusions for NTRK1, and two fusions for FGFR3. The two fusions for each gene were separated onto different plasmids in part to prevent plasmids from containing significant stretches of identical sequence, which could be unstable and subject to recombination.

Each fusion in the construct was designed to include 250 nucleotides upstream and downstream of the break point that connects two different genes in a fusion pair. For example, the EML4-ALK fusion contains approximately 250 nucleotides of EML4 joined to approximately 250 nucleotides of ALK.

An SP6 promoter was placed before the fusion targets so that RNA could be transcribed from the plasmid.

A short, approximately 125 base pair sequence was added downstream of the fusion targets. This sequence was used for validation of the construct by a TaqMan based real time PCR assay, which targets the sequence. The sequence allowed for the quantification of transcribed RNA, to increase the precision and accuracy of RNA measurements for subsequent transfection steps.

A poly-A tail was added downstream of both the fusion targets and the sequence used for quantitation to increase RNA stability in transfected cells.

### Example 2. Transfecting cells with RNA

RNA was transcribed using the mMessage mMachine SP6 Transcription kit from Ambion-now Thermo Fisher. This kit was used because it incorporates a cap analog [m7G(5')ppp(5')G], which is incorporated only as the first or 5' terminal G of the transcript, because its structure precludes its incorporation at any other position in the RNA molecule. RNAs lacking a 5' cap structure may be targeted to intracellular degradation pathways, and thus, the capped transcription kit was used to increase the stability of RNA within a cell.

The RNAs were electroporated into the GM24385 human cell line. This cell line is a National Institutes of Standards Genome in a Bottle reference genome, which has been well characterized by NGS and can be used in commercial products.

The RNA was introduced into cells using electroporation. 10 µg of RNA was used to transfect 40 million cells. The electroporation conditions were as follows: 300 Volts/500µF/1 Pulse/4mm Cuvette.

After electroporation, the cells were allowed to recover for 6 hours. At 6 hours post electroporation, the cells were pelleted, the supernatant was removed, and new media was added. Removing the transfection media helps to remove unincorporated RNA from the sample.

At 24 hours post electroporation, the cells were gently pelleted, and washed using phosphate buffered saline. The cells were resuspended in phosphate buffered saline at approximately 4.4E+06 cells/mL. 2 mL of the washed cells were transferred to fixative and fixed for 20 minutes in formalin to kill the cells and preserve the cell structure. The cells were then dehydrated through a series of washes in ethanol and stored at the same concentration (∼4.4E+06 cells/mL) at -20°C in 70% ethanol.

An aliquot of the cells was flash frozen rather than fixed to verify that the biosynthetic RNA was in fact incorporated into the cells (via TaqMan based Real Time PCR).

Nucleic acids were extracted from the fixed cells using an Agencourt FormaPure - Nucleic Acid Extraction from FFPE Tissue Kit. TaqMan real time PCR was performed on the extracted nucleic acids. RNA was recovered from the fixed cells at about the same level as from unfixed cells. The copy number of the multiplex RNA was calculated to be greater than 250 copies per cell.

**Table 3. Quantification of biosynthetic RNA within transfected cells.**

| **Sample** | **Copies/mL (Formapure Extraction of Fixed Cells)** | **Copies/mL (QiaAmp viral mini kit with flash frozen cells)** | **Approximate RNA Copies per cell** |
|---|---|---|---|
| Transfection: Construct RNA#1 | 9. 62E+08 | 1.23E+09 | 274 |
| Transfection: Construct RNA#2 | 1.72E+09 | 1.79E+09 | 417 |
| Non-Transfected GM24385 cells | Not Detected | Not Detected | 0 |

Because there appeared to be hundreds of copies of the biosynthetic RNA per cell, the transfected cells were diluted with non-transfected cells to bring the amount of fusion RNAs down to physiological levels. Transfected cells were diluted into the non-transfected cells in 10-fold serial dilutions to make a 1:10, 1:100, and 1:1000 dilution of each construct.

In parallel experiments, nucleic acid was extracted from transfected cells and non-transfected cells. The nucleic acid was normalized to the same concentration and then the nucleic acid from the transfected cells was serially diluted into the nucleic acid from the non-transfected cells to achieve 1:10, 1:100 and 1:1000 dilutions.

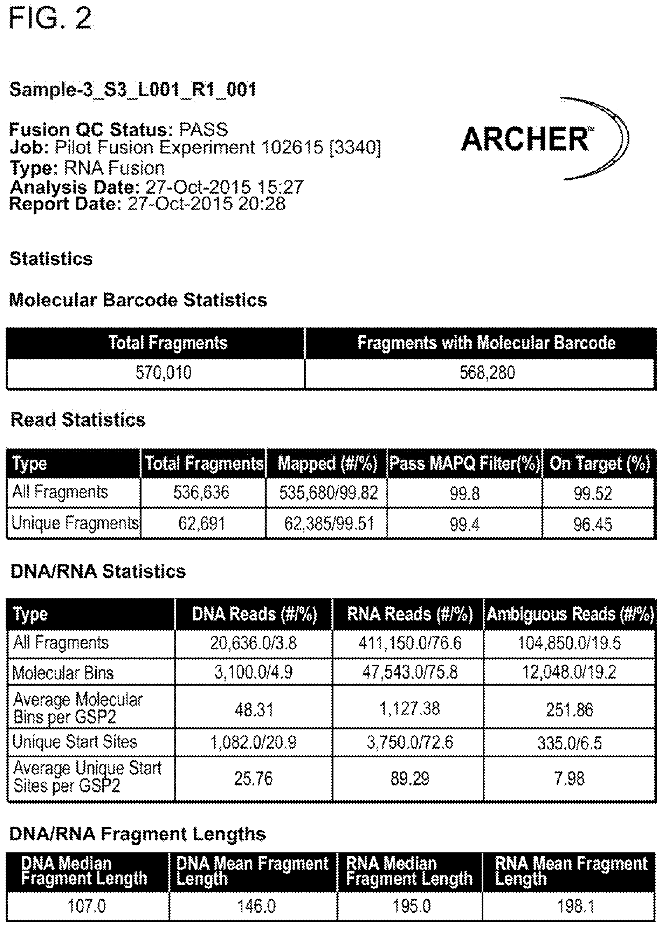

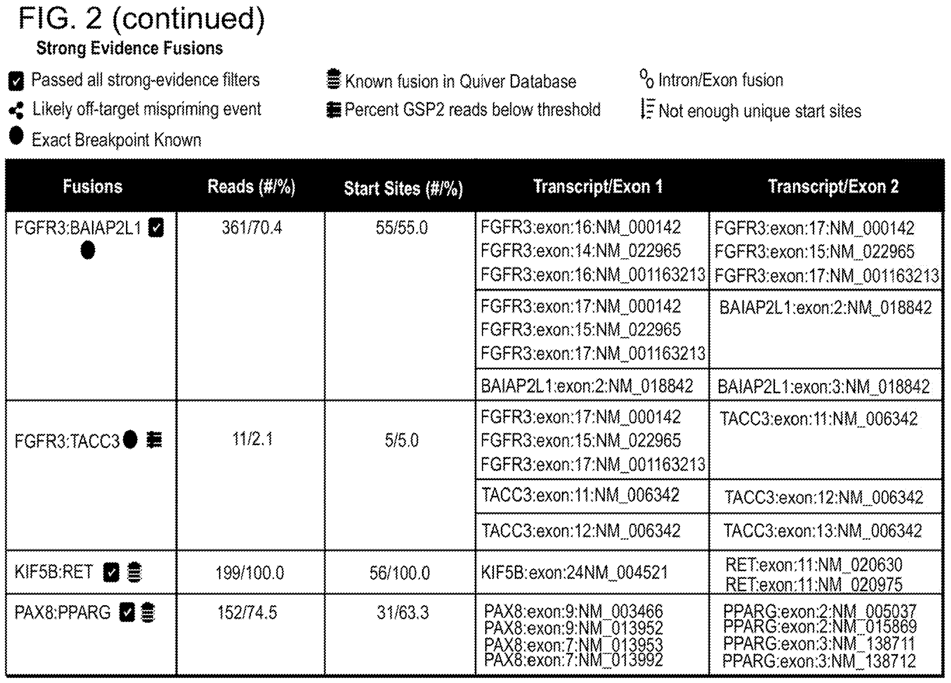

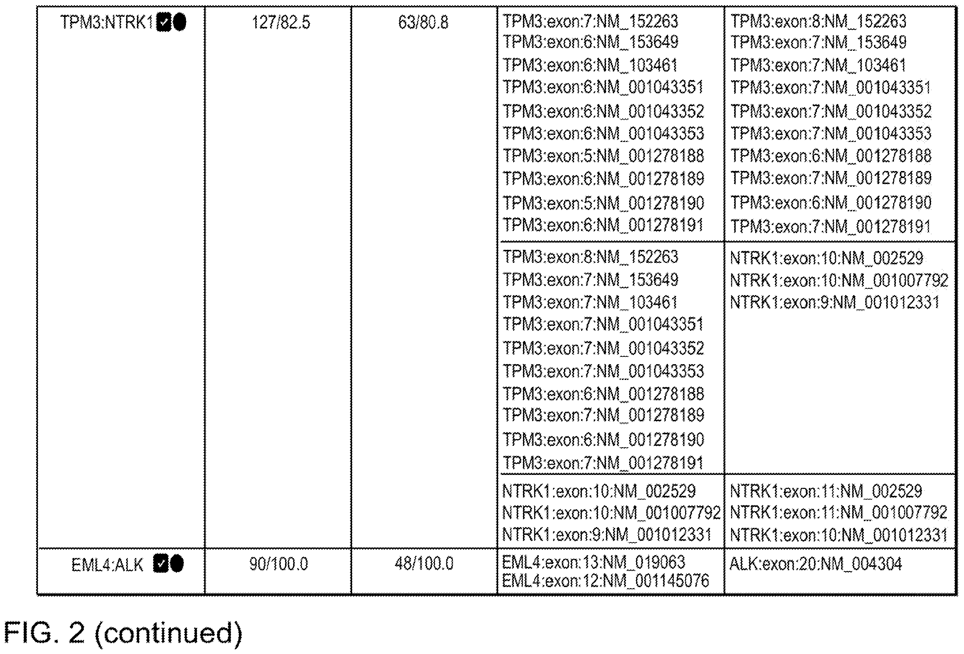

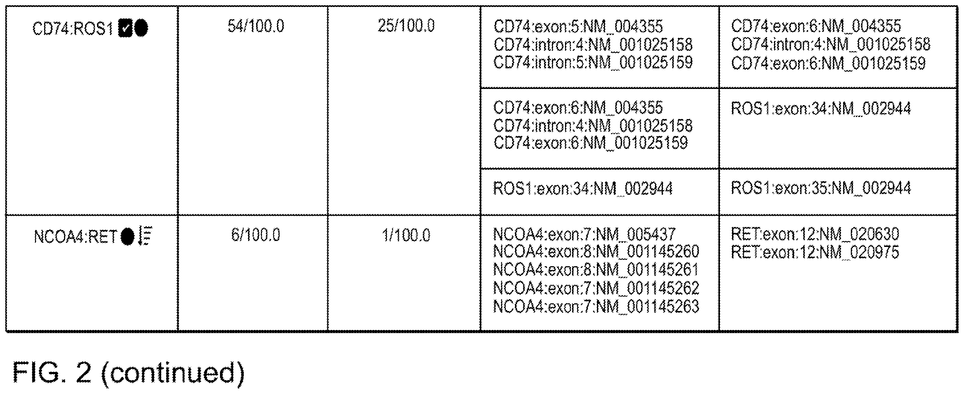

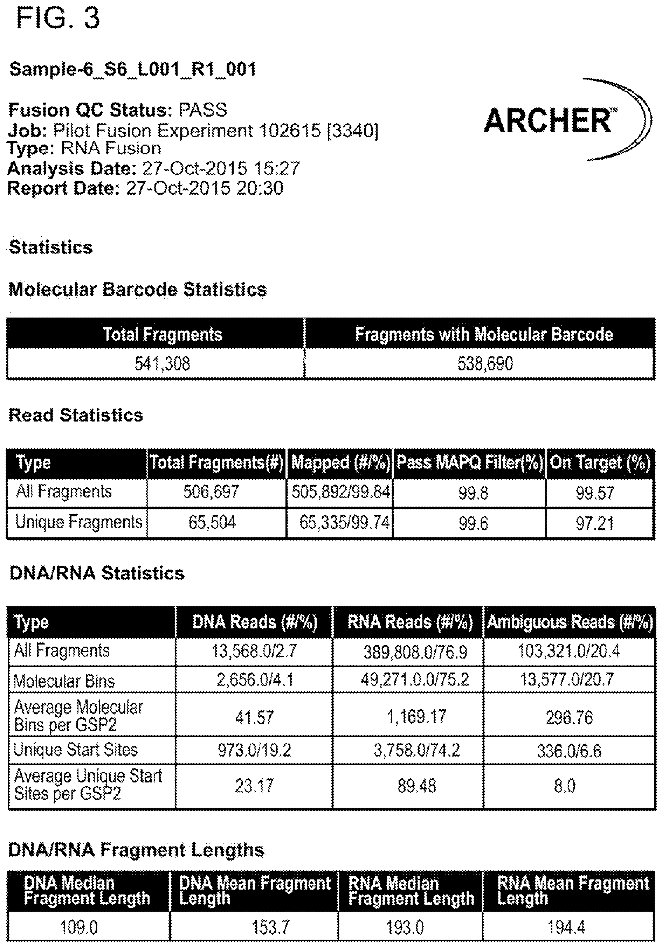

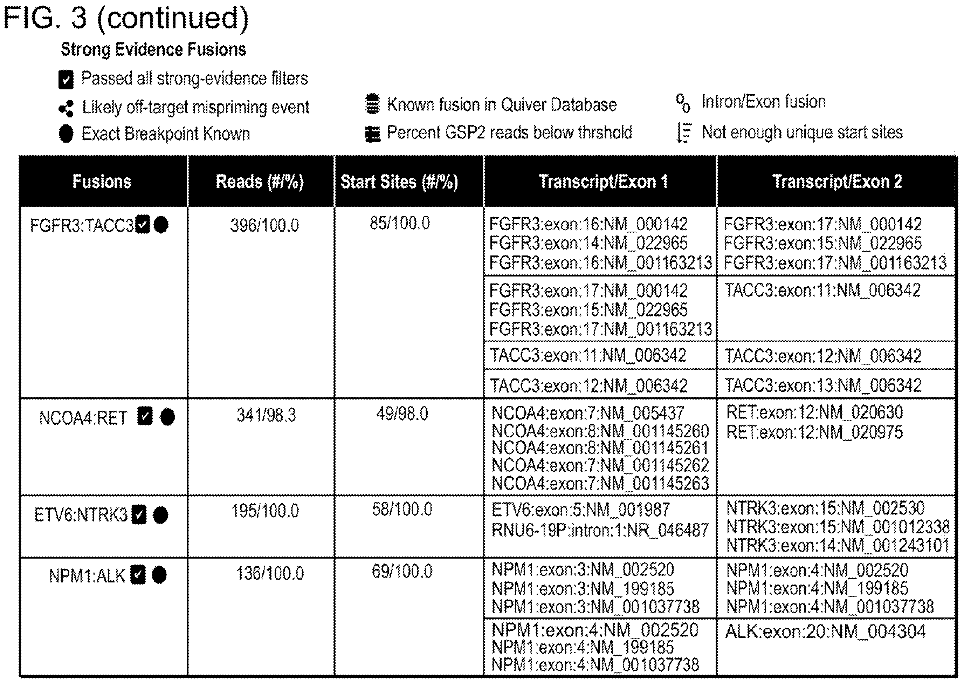

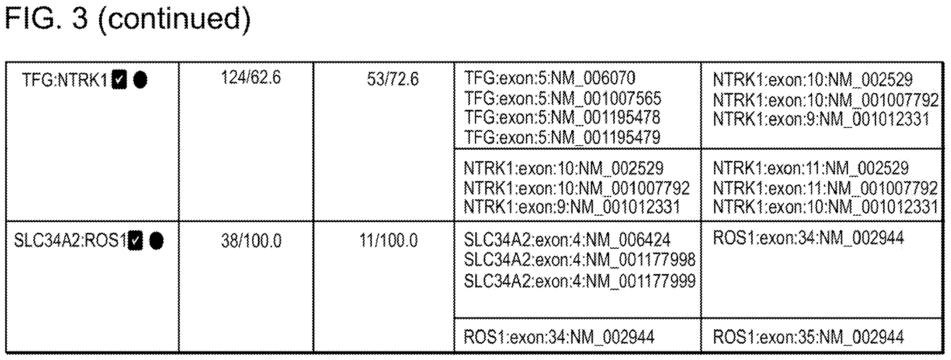

Total nucleic acid was extracted from the cells using a FormaPure extraction kit according to the modifications to the FormaPure extraction protocol recommended by ArcherDx. Total nucleic acid was used for library preparation using the Archer™ Universal RNA Reagent Kit v2 for Illumina and the Archer™ FusionPlex™ Lung Thyroid Panel. Library preparation followed the instructions from ArcherDx, and the library was analyzed using an Illumina MiSeq instrument. All the expected oncology gene fusions were appropriately identified by the software (Figures 2 and 3).

**Table 4. Numbers of reads across the junction of each gene fusion for Construct #1**

| | **EML4-ALK** | **KIF5B-RET** | **CD74-ROS** | **TPM3-NTRK1** | **FGFR3-BAIAP2L1** | **Pax8-PPARG** |
|---|---|---|---|---|---|---|
| Sample 1 (1:10 dilution) | 4,995 | 9,622 | 1,870 | 5,468 | 10,986 | 5,865 |
| Sample 2 (1:100 dilution) | 820 | 1,912 | 405 | 1,014 | 2,116 | 1,154 |
| Sample 3 (1:1000 dilution) | 90 | 199 | 54 | 127 | 361 | 152 |

**Table 5. Numbers of reads across the junction of each gene fusion for Construct #2**

| | **NPM-ALK** | **NCOA4-RET** | **SLC34A2-ROS1** | **TFG-NTRK1** | **FGFR3-TACC3** | **ETV6-NTRK3** |
|---|---|---|---|---|---|---|
| Sample 4 (1:10 dilution) | 8,737 | 9,699 | 1,083 | 5,277 | 12,478 | 6,828 |
| Sample 5 (1:100 dilution) | 1,467 | 2,216 | 221 | 1,070 | 3,205 | 1,692 |
| Sample 6 (1:1000 dilution) | 136 | 341 | 38 | 124 | 396 | 195 |

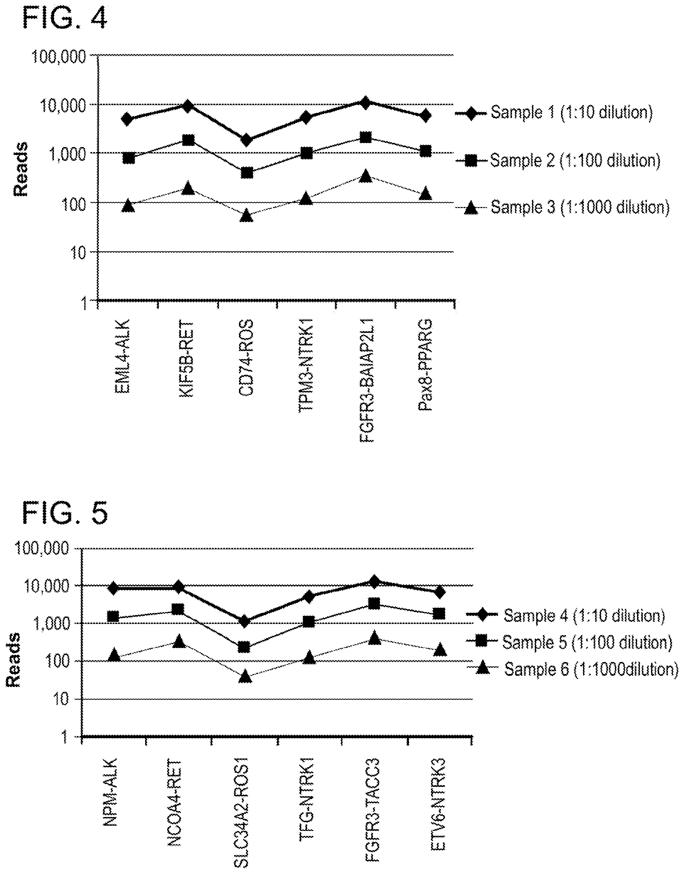

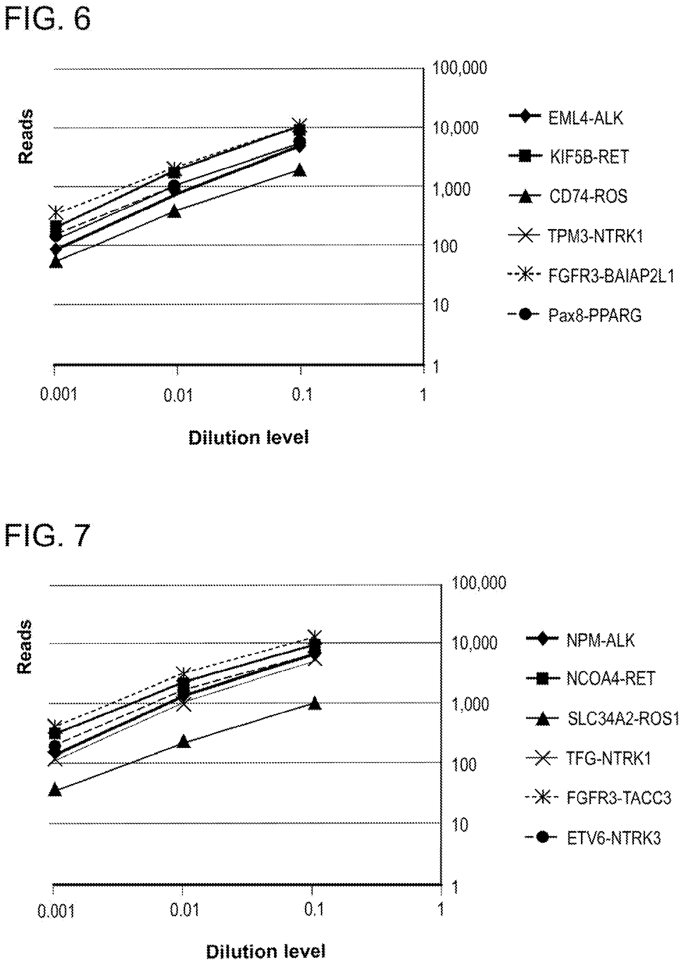

The number of reads across each fusion junction were graphed for the 1:10, 1:100 and 1:1000 dilutions of both construct #1 and construct #2 (Figures 4 and 5). When the dilution level is plotted against the number of reads, there is a linear relationship (Figures 6 and 7). This demonstrates that a reference material may be adjusted to achieve the desired number of reads by simply diluting transfected cells prior to subsequent processing steps. Since there is a linear response, the dilution amount can be easily calculated.

The cell mixtures (1:10, 1:100, and 1:1000 dilutions) were extracted again using the Agencourt FormaPure extraction kit, following a protocol to produce pure RNA (*i.e.,* with a DNAse treatment step). The RNA product was analyzed using an Ion AmpliSeq™ RNA Fusion Lung Cancer Research panel. This panel is limited to only fusions of ALK, RET, ROS1, and NTRK1, and it focuses only on those fusions found in lung cancer. Therefore, not all the fusions contained in the multiplex material were assayed in the panel. However, the assayed fusions were each detected at all three dilution levels. Total reads are shown in Tables 6 and 7.

**Table 6. Number of Ion AmpliSeq reads across the junction of each fusion for Construct #1.**

| | **EML4-ALK** | **KIF5B-RET** | **CD74-ROS** | **TPM3-NTRK1** | **FGFR3-BAIAP2L1** | **Pax8-PPARG** |
|---|---|---|---|---|---|---|
| Sample 1 (1:10 dilution) | 129253 | 159064 | 96158 | 166297 | Not assayed by panel | Not assayed by panel |
| Sample 2 (1:100 dilution) | 42901 | 59097 | 27093 | 58240 | Not assayed by panel | Not assayed by panel |
| Sample 3 (1:1000 dilution) | 11328 | 15506 | 8126 | 13728 | Not assayed by panel | Not assayed by panel |

**Table 7. Number of Ion AmpliSeq reads across the junction of each fusion for Construct #2.**

| | **NPM-ALK** | **NCOA4-RET** | **SLC34A2 -RS1** | **TFG-NTRK1** | **FGFR3-TACC3** | **ETV6-NTRK3** |
|---|---|---|---|---|---|---|
| Sample 4 (1:10 dilution) | Lymphoid -Not assayed | Thyroid-Not assayed | 93057 | 163517 | Not assayed by panel | Not assayed by panel |
| Sample 5 (1:100 dilution) | Lymphoid - Not assayed | Thyroid-Not assayed | 27656 | 58255 | Not assayed by panel | Not assayed by panel |
| Sample 6 (1:1000 dilution) | Lymphoid - Not assayed | Thyroid-Not assayed | 3600 | 7618 | Not assayed by panel | Not assayed by panel |

### Example 3. Pooled constructs

Fixed, transfected cells bearing construct #1 and fixed, transfected cells bearing construct #2 were mixed and diluted into non-transfected cells at a 1:1000 dilution level. Total nucleic acids were extracted using the FormaPure extraction kit. Lot number 102342 was assigned to the total nucleic acid.

Next generation sequencing was performed according to the instructions for the Archer Dx FusionPlex Lung Thyroid Panel. All 12 fusions were detected, and each fusion passed all strong-evidence filters. Interestingly, although the KIF5B-RET fusion was identified, the Archer software did not indicate that the fusion was known. The sample thus identified a discrepancy in the Archer software. The PAX8-PPARG was similarly identified, but the Archer software did not indicate that the fusion was known, which was expected because this fusion is not annotated in the Archer software. All other gene fusions were flagged as known.

**Table 8. Number of ArcherDx reads across the junction of each fusion for lot 102342.**

| **Fusion** | **Spanning Reads** |
|---|---|
| EML4-ALK | 118 |
| NPM-ALK | 108 |
| KIF5B-RET | 191 |
| NCOA4-RET | 226 |
| CD74-ROS1 | 65 |
| SLC34A2-ROS1 | 34 |
| TPM3-NTRK1 | 143 |
| TFG-NTRK1 | 115 |
| FGFR3-BAIAP2L1 | 412 |
| FGFR3-TACC3 | 328 |
| Pax8-PPARG | 179 |
| ETV6-NTRK3 | 172 |

### Example 4. Embedding Cells

Fixed, transfected cells bearing construct #1 and fixed, transfected cells bearing construct #2 were mixed and diluted into non-transfected cells at 1:10, 1:100, and 1:1000 dilutions levels (called "high," "medium," and "low" copy number samples). 1 mL of each cell mixture was pelleted and resuspended in HistoGel. The HistoGel/cell mixture was transferred to the barrel of a 3 mL syringe and allowed to solidify. After solidification, each of the three "cores" (high, medium, and low) was trimmed and cut into two pieces. The cores were placed in 10% formalin at 2-8°C for 18-24 hours. After the overnight fixation, the cores were dehydrated by incubation with increasing concentrations of ethanol (50%, 70%, 80%, and 100%). After dehydration in ethanol, the cores were incubated in naphtha (a xylene substitute) overnight. On the third day, the naptha was exchanged several times, and the cores were embedded in paraffin.

The paraffin blocks were sectioned into 10 µm sections. Based on the number of cells embedded, a 10 micron section should contain the DNA/RNA equivalent of about 10,000 cells. Each 10 µm section would contain roughly 1,400 transfected cells in the "High" block, 140 transfected cells in the "Med" block, and 14 transfected cells in the "Low" block.

Five sections from each block were extracted using the Agencourt FormaPure extraction protocol to obtain total nucleic acid (Table 9).

**Table 9. Nucleic Acid yields from Formalin-Fixed Paraffin-Embedded (FFPE) cells.**

| Sample Name | Concentration by Nanodrop (ng/µL) | A260/280 ratio (should be ∼2.0 for pure RNA) | A260/230 ratio | Concentration by Qubit RNA HS (ng/µL) | Total Yield (5 sections - according to Qubit analysis) |
|---|---|---|---|---|---|
| FFPE High | 10.7 | 2.01 | 1.97 | 5.76 | 201.6 ng |
| FFPE Med | 11.7 | 2.03 | 1.59 | 6.24 | 218.4 ng |
| FFPE Low | 13.2 | 1.97 | 1.73 | 6.93 | 242.5 ng |

Approximately 125 ng of total nucleic acid was used for library preparation using the Archer™ Universal RNA Reagent Kit v2 for Illumina and the Archer™ FusionPlex™ Lung Thyroid Panel. Library preparation followed the instructions from ArcherDx, and each sample was analyzed using an Illumina MiSeq instrument. The results for the "High" sample displayed off-target fusions, and the "Low" sample failed to detect most expected fusions. However, the "Med" sample detected 11 out of 12 expected fusions as shown in Table 10 below. There was more variability between the number of spanning reads for the different fusion targets when total nucleic acid was extracted from FFPE relative to the lightly fixed cells of Examples 2 and 3 (Table 11).

CD74-ROS1 was not detected in "FFPE med" sample; however, it was detected in the "FFPE high" sample, indicating that the construct was designed appropriately. The reason for the low reads for both CD74-ROS1 and SLC34A2- ROS1 is unknown; however, the ROS1 RNA may be susceptible to damage either during the electroporation step or during formalin fixation, such that, in this region of the RNA construct, fewer molecules could be amplified during library preparation.

**Table 10.**

| Number of ArcherDx reads across the junction of each fusion for the 1:100 FFPE sample. | |
|---|---|
| **Fusion** | **Spanning Reads** |
| EML4-ALK | 82 |
| NPM-ALK | 233 |
| KIF5B-RET | 300 |
| NCOA4-RET | 650 |
| CD74-ROS1 | 0 |
| SLC34A2-ROS1 | 47 |
| TPM3-NTRK1 | 83 |
| TFG-NTRK1 | 146 |
| FGFR3-BAIAP2L1 | 688 |
| FGFR3-TACC3 | 1001 |
| Pax8-PPARG | 237 |
| ETV6-NTRK3 | 252 |

**Table 11. Comparison of reads across the junction of each fusion for the samples of Example 2 (Run #1), Example 3 (Run #2), and Example 4 (FFPE)**

| **Fusion** | **Run #1 (pilot)-combined** | **Run #2 (102342 fixed Cells)** | **FFPE** |
|---|---|---|---|
| EML4-ALK | 90 | 118 | 82 |
| NPM-ALK | 136 | 108 | 233 |
| KIF5B-RET | 199 | 191 | 300 |
| NCOA4-RET | 341 | 226 | 650 |
| CD74-ROS1 | 54 | 65 | 0 |
| SLC34A2-ROS1 | 38 | 34 | 47 |
| TPM3-NTRK1 | 127 | 143 | 83 |
| TFG-NTRK1 | 124 | 115 | 146 |
| FGFR3-BAIAP2L1 | 361 | 412 | 688 |
| FGFR3-TACC3 | 396 | 328 | 1001 |
| Pax8-PPARG | 152 | 179 | 237 |
| ETV6-NTRK3 | 195 | 172 | 252 |

The extracted nucleic acid from the "FFPE med" sections was tested by a commercial laboratory, which uses the OncoMine® Cancer Research Panel. Results are shown in Table 12. NPM1-ALK, ETV6-NTRK3 and TFG-NTRK1 were not detected, but the remaining nine fusions in the reference material were positively detected. Examination of the OncoMine manifest suggests that the assay does not test for NPM1-ALK or TFG-NTRK1, and so positive results for these fusions were not expected. OncoMine was expected to assay for ETV6- NTRK3, however, and the exact reason for the failure to detect this fusion is unknown.

**Table 12.**

| Number of OncoMine reads across the junction of various fusions for the 1:100 FFPE sample. | | | |
|---|---|---|---|
| **Locus** | **Oncomine Variant Class** | **Genes** | **Read Counts** |
| chr2: 42491871 - chr2: 29446394 | Fusion | EML4(6) - ALK(20) | 92 |
| chr2: 42522656 - chr2: 29446394 | Fusion | EML4(13) - ALK(20) | 8380 |
| chr10: 32306070 - chr10: 43609927 | Fusion | KIF5B(24) - RET(11) | 12561 |
| chr10: 51582939 - chr10: 43612031 | Fusion | NCOA4(7) - RET(12) | 2403 |
| chr5: 149784242 - chr6: 117645578 | Fusion | CD74(6) - ROS1(34) | 513 |
| chr4: 25665952 - chr6: 117645578 | Fusion | SLC34A2(4) - ROS1(34) | 410 |
| chr1: 154142875 - chr1: 156844362 | Fusion | TPM3(7) - NTRK1(10) | 14706 |
| chr4: 1808661 - chr7: 97991744 | Fusion | FGFR3(17) - BAIAP2L(12) | 3282 |
| chr4: 1808661 - chr4: 1741428 | Fusion | FGFR3(17) - TACC3(11) | 22269 |
| chr2: 113992970 - chr3: 12421202 | Fusion | PAX8(9) - PPARG(2) | 9346 |

The FFPE sample was sent to a second commercial laboratory for testing (data not shown).

At first glance, there appeared to be multiple discrepancies between the results from the ArcherDx analysis and the other two labs. Closer inspection shows that there was generally no disagreement on the RNA fusions present, but on the exact breakpoints and exons that were joined together. For example, both FGFR3 fusions were called in the Archer Assay as FGFR3(18)-BAIAP2L1(2) and FGFR3(18)-TACC3(11), and they were designed so that exon 18 of FGFR3 was fused to the other gene (Table 1). However, Exon 17 and 18 are both less than 200 bp, and so both exon sequences were present in the construct. For an assay that depends on the production of a PCR product, it makes sense that a fusion to exon 17 would be detected. It seems the NCOA4-RET fusion may have been assessed similarly. This fusion RNA was designed and detected on Archer assay as fusion of NCOA4 exon 8 with RET exon 12, but on OncoMine, it is called as a NCOA4 exon 7 fusion to RET exon 12. Again, exon 7 and exon 8 of NCOA4 are both very small, and so both are present in the construct. The difference in the exact breakpoint is unlikely to affect clinical decision making. As long as the functional domains are joined in the fusion protein, the downstream effects will be the same.

### Example 5. FFPE Reference Materials with Higher Cell Concentration

Although results from the "FFPE med" block of Example 4 were generally good, feedback from ArcherDx and others suggested that the amount of extractable RNA was low and might not meet customer expectations for nucleic acid yield. Therefore, a new FFPE block was prepared using the same fixed, transfected cells and same 1:100 mix ratio as in the "FFPE med" block. For this new preparation, ∼50 million cells were embedded to give rise to a ∼10 mm high core (of 5x higher concentration than before), which could be used to prepare ∼800 x 10 µm sections in 2 identical FFPE blocks.

Results are shown in Table 13. Whereas the "FFPE med" block only yielded approximately 218 ng of total nucleic acid from five 10 µm sections, the new block (lot number 102380) yielded approximately this same amount from only one section, indicating that the yield was approximately five-fold higher.

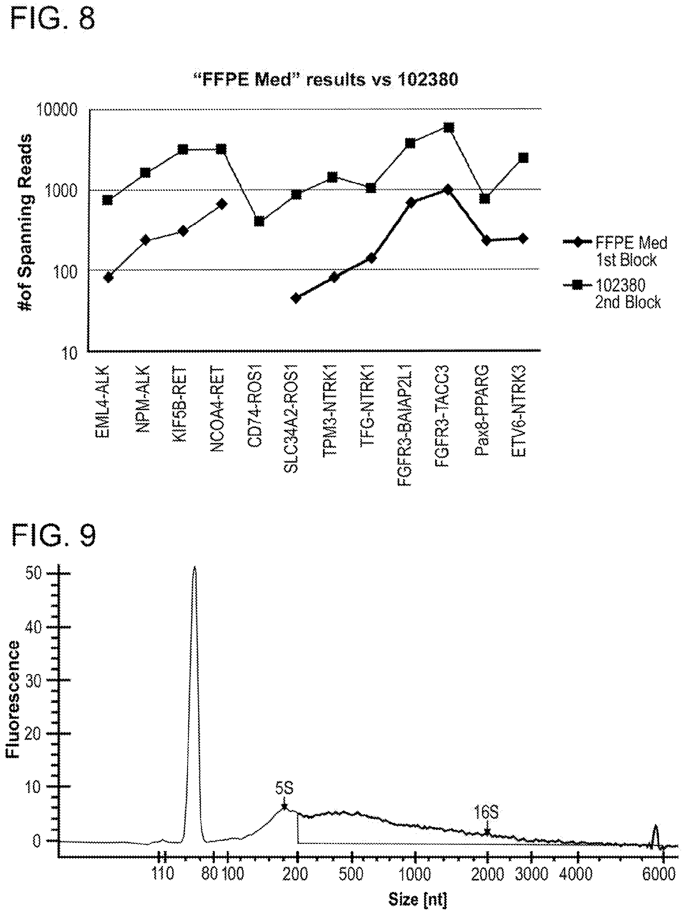

Lot 102380 was assayed using the ArcherDx FusionPlex Lung-Thyroid panel as in the previous examples except that approximately 250 ng of input nucleic acid was used for library preparation. Importantly, ArcherDx introduced a major update to its Archer Analysis software, from version 3.3 to version 4.0. The major difference between these versions is that 3.3 aligned each read to a human reference sequence. Reads that mapped to two disparate locations supported the fusion calls. However, in version 4.0, reads are used for *de novo* assembly. The software can essentially use the reads to assemble across the SeraCare multiplex fusion construct. Therefore, fusions of three or four genes were observed. Additionally, the new software version also listed fusions separately, even if they had the same breakpoint, resulting in a report with duplicate calls (for example, NCOA4-RET and FGFR3-TACC3 were both called twice, both with the bulls-eye symbol, indicating that the exact breakpoint was known). These issues are inherent to the software and not specific to the design of the reference material. Despite the confusing additional calls, all 12 expected fusions were detected as strong evidence fusions, and the numbers of spanning reads, although higher on this run, were consistent with those from Example 4 (Figure 8).

**Table 13.**

| Nucleic Acid yields from Formalin-Fixed Paraffin-Embedded (FFPE) cells (Lot 102380). | | | | | |
|---|---|---|---|---|---|
| **Lot Number** | **# of curls per vial** | **Recovered elution volume** | **Concentration per uL (by Qubit RNA HS)** | **TOTAL Yield** | **Average yield per curl** |
| 102380 | 1 curl | 35 uL | 8.8 ng/uL | 308 ng | 273 ng/curl |
| 102380 | 1 curl | 35 uL | 6.6 ng/uL | 231 ng | |
| 102380 | 1 curl | 35 uL | 8.05 ng/uL | 282 ng | |
| 102380 | 5 curls | 35 uL | 28.5 ng/uL | 998 ng | 198 ng/curl |
| 102380 | 5 curls | 35 uL | 28.0 ng/uL | 980 ng | |

Lot 102380 was extracted and tested by a commercial laboratory using the ArcherDx FusionPlex Solid Tumor Panel with similar results as those described in the preceding paragraph.

Lot 102380 was also extracted and tested by a second commercial laboratory using an unknown assay, which identified each of the twelve gene fusions. This laboratory also confirmed that the ROS1 fusions were relatively low-abundance in comparison to the other fusions in the reference material.

### Example 6. Analysis of RNA extracted from FFPE Reference Materials

Lot 102380 was shipped to a commercial laboratory to assess the yield and integrity of the RNA after extraction. The commercial laboratory extracted 135 ng RNA from a first 10 µm section and 164 ng RNA from a second 10 µm section. The RNA sizes were broadly distributed with a peak at approximately 200-500 nucleotides (Figure 9). The RNA was degraded to such a point that the 18S and 28S ribosomal RNA peaks were not evident.

## Claims

1. A nucleic acid, comprising a plurality of nucleotide sequences, wherein:
each nucleotide sequence of the plurality comprises a first subsequence and a second subsequence;
the first subsequence comprises a 3' sequence of a first exon;
the second subsequence comprises a 5' sequence of a second exon;
the first subsequence and second subsequence are adjoining sequences in the nucleic acid;
the first subsequence and second subsequence are each at least 20 nucleotides long;
the first subsequence is 5' relative to the second subsequence in the nucleic acid;
the first exon is an exon of a first gene;
the second exon is an exon of a second gene;
the first gene and second gene are different genes; and
each nucleotide sequence of the plurality of nucleotide sequences comprises either a 3' sequence of a first exon that is different from every other first exon of the nucleotide sequences of the plurality or a 5' sequence of a second exon that is different from every other second exon of the nucleotide sequences of the plurality.

2. The nucleic acid of claim 1, wherein:
(a) the nucleic acid is DNA or RNA; and/or
(b) each nucleotide sequence of the plurality is associated with a neoplasm, optionally wherein the neoplasm is a lung cancer, non-small cell lung cancer, soft tissue cancer, lymphoid cancer, acute lymphoid leukemia, acute myeloid leukemia, chronic myelogenous leukemia, non-Hodgkin's lymphoma, Burkitt lymphoma, melanoma, intraocular melanoma, central nervous system cancer, neuroblastoma, thyroid cancer, parathyroid cancer, hepatocellular cancer, stomach cancer, large intestine cancer, colon cancer, urinary tract cancer, bladder cancer, kidney cancer, prostate cancer, cervical cancer, ovarian cancer, or breast cancer.

3. The nucleic acid of claims 1 or 2, wherein:
(a) each first gene and each second gene is selected from the group consisting of anaplastic lymphoma receptor tyrosine kinase (ALK), brain-specific angiogenesis inhibitor 1-associated protein 2-like protein 1 (BAIAP2L1), CD74, echinoderm microtubule-associated protein-like 4 (EML4), ETS variant 6 (ETV6), fibroblast growth factor receptor 3 (FGFR3), kinesin-1 heavy chain (KIF5B), nuclear receptor coactivator 4 (NCOA4), nucleophosmin (NPM1), neurotrophic tyrosine receptor kinase 1 (NTRK1), neurotrophic tyrosine receptor kinase 3 (NTRK3), paired box gene 8 (Pax8), peroxisome proliferator-activated receptor gamma (PPARG), RET proto-oncogene (RET), ROS proto-oncogene 1 (ROS1), sodium-dependent phosphate transport protein SLC34A, transforming acidic coiled-coil-containing protein 3 (TACC3), TRK-fused gene (TFG), and tropomyosin 3 (TPM3);
optionally wherein each first exon and each second exon is selected from the group consisting of ALK exon 20, BAIAP2L1 exon 2, CD74 exon 6, EML4 exon 13, ETV6 exon 5, FGFR3 exon 18, KIF5B exon 24, NCOA4 exon 8, NPM1 exon 5, NTRK1 exon 10, NTRK3 exon 13, PAX8 exon 8, PPARG exon 1, RET exon 11, RET exon 12, ROS1 exon 34, SLC34A exon 4, TACC3 exon 11, TFG exon 5, and TPM3 exon 8;
optionally wherein the first exon and second exon of each nucleotide sequence is selected from the group consisting of EML4 exon 13 and ALK exon 20; NPM1 exon 5 and ALK exon 20; KIF5B exon 24 and Ret exon 11; NCOA4 exon 8 and RET exon 12; CD74 exon 6 and ROS1 exon 34; SLC34A exon 4 and ROS1 exon 34; TPM3 exon 8 and NTRK1 exon 10; TFG exon 5 and NTRK1 exon 10; FGFR3 exon 18 and BAIAP2L1 exon 2; FGFR3 exon 18 and TACC3 exon 11; PAX8 exon 8 and PPARG exon 1; and ETV6 exon 5 and NTRK3 exon 13, respectively; or
(b) each nucleotide sequence of the plurality comprises a subsequence of a gene selected from the group consisting of anaplastic lymphoma receptor tyrosine kinase (ALK), brain-specific angiogenesis inhibitor 1-associated protein 2-like protein 1 (BAIAP2L1), CD74, echinoderm microtubule-associated protein-like 4 (EML4), ETS variant 6 (ETV6), fibroblast growth factor receptor 3 (FGFR3), kinesin-1 heavy chain (KIF5B), nuclear receptor coactivator 4 (NCOA4), nucleophosmin (NPM1), neurotrophic tyrosine receptor kinase 1 (NTRK1), neurotrophic tyrosine receptor kinase 3 (NTRK3), paired box gene 8 (Pax8), peroxisome proliferator-activated receptor gamma (PPARG), RET proto-oncogene (RET), ROS proto-oncogene 1 (ROS1), sodium-dependent phosphate transport protein SLC34A, transforming acidic coiled-coil-containing protein 3 (TACC3), TRK-fused gene (TFG), and tropomyosin 3 (TPM3);
optionally wherein each subsequence of a gene is a subsequence from a single exon of the gene;
optionally wherein each single exon is selected from the group consisting of ALK exon 20, BAIAP2L1 exon 2, CD74 exon 6, EML4 exon 13, ETV6 exon 5, FGFR3 exon 18, KIF5B exon 24, NCOA4 exon 8, NPM1 exon 5, NTRK1 exon 10, NTRK3 exon 13, PAX8 exon 8, PPARG exon 1, RET exon 11, RET exon 12, ROS1 exon 34, SLC34A exon 4, TACC3 exon 11, TFG exon 5, and TPM3 exon 8;
optionally wherein the first exon and second exon of each nucleotide sequence are selected from the group consisting of EML4 exon 13 and ALK exon 20; NPM1 exon 5 and ALK exon 20; KIF5B exon 24 and Ret exon 11; NCOA4 exon 8 and RET exon 12; CD74 exon 6 and ROS1 exon 34; SLC34A exon 4 and ROS1 exon 34; TPM3 exon 8 and NTRK1 exon 10; TFG exon 5 and NTRK1 exon 10; FGFR3 exon 18 and BAIAP2L1 exon 2; FGFR3 exon 18 and TACC3 exon 11; PAX8 exon 8 and PPARG exon 1; and ETV6 exon 5 and NTRK3 exon 13, respectively; or
(c) each first gene and each second gene, respectively, is selected from the group consisting of ACBD6 and RRP15; ACSL3 and ETV1; ACTB and GLI1; AGPAT5 and MCPH1; AGTRAP and BRAF; AKAP9 and BRAF; ARFIP1 and FHDC1; ARID1A and MAST2; ASPSCR1 and TFE3; ATG4C and FBXO38; ATIC and ALK; BBS9 and PKD1L1; BCR and ABL1; BCR and JAK2; BRD3 and NUTM1; BRD4 and NUTM1; C2orf44 and ALK; CANT1 and ETV4; CARS and ALK; CCDC6 and RET; CD74 and NRG1; CD74 and ROS1; CDH11 and USP6; CDKN2D and WDFY2; CEP89 and BRAF; CHCHD7 and PLAG1; CIC and DUX4L1; CIC and FOXO4; CLCN6 and BRAF; CLIP1 and ROS1; CLTC and ALK; CLTC and TFE3; CNBP and USP6; COL1A1 and PDGFB; COL1A1 and USP6; COL1A2 and PLAG1; CRTC1 and MAML2; CRTC3 and MAML2; CTAGE5 and SIP1; CTNNB1 and PLAG1; DCTN1 and ALK; DDX5 and ETV4; DNAJB1 and PRKACA; EIF3E and RSPO2; EIF3K and CYP39A1; EML4 and ALK; EPC1 and PHF1; ERC1 and RET; ERC1 and ROS1; ERO1L and FERMT2; ESRP1 and RAF1; ETV6 and ITPR2; ETV6 and JAK2; ETV6 and NTRK3; EWSR1 and ATF1; EWSR1 and CREB1; EWSR1 and DDIT3; EWSR1 and ERG; EWSR1 and ETV1; EWSR1 and ETV4; EWSR1 and FEV; EWSR1 and FLI1; EWSR1 and NFATC1; EWSR1 and NFATC2; EWSR1 and NR4A3; EWSR1 and PATZ1; EWSR1 and PBX1; EWSR1 and POU5F1; EWSR1 and SMARCA5; EWSR1 and SP3; EWSR1 and WT1; EWSR1 and YY1; EWSR1 and ZNF384; EWSR1 and ZNF444; EZR and ROS1; FAM131B and BRAF; FBXL18 and RNF216; FCHSD1 and BRAF; FGFR1 and ZNF703; FGFR1 and PLAG1; FGFR1 and TACC1; FGFR3 and BAIAP2L1; FGFR3 and TACC3; FN1 and ALK; FUS and ATF1; FUS and CREB3L1; FUS and CREB3L2; FUS and DDIT3; FUS and ERG; FUS and FEV; GATM and BRAF; GMDS and PDE8B; GNAI1 and BRAF; GOLGA5 and RET; GOPC and ROS1; GPBP1L1 and MAST2; HACL1 and RAF1; HAS2 and PLAG1; HERPUD1 and BRAF; HEY1 and NCOA2; HIP1 and ALK; HLA-A and ROS1; HMGA2 and ALDH2; HMGA2 and CCNB1IP1; HMGA2 and COX6C; HMGA2 and EBF1; HMGA2 and FHIT; HMGA2 and LHFP; HMGA2 and LPP; HMGA2 and NFIB; HMGA2 and RAD51B; HMGA2 and WIF1; HN1 and USH1G; HNRNPA2B1 and ETV1; HOOK3 and RET; IL6R and ATP8B2; INTS4 and GAB2; IRF2BP2 and CDX1; JAZF1 and PHF1; JAZF1 and SUZ12; KIAA1549 and BRAF; KIAA1598 and ROS1; KIF5B and ALK; KIF5B and RET; KLC1 and ALK; KLK2 and ETV1; KLK2 and ETV4; KMT2A and ABI1; KMT2A and ABI2; KMT2A and ACTN4; KMT2A and AFF1; KMT2A and AFF3; KMT2A and AFF4; KMT2A and ARHGAP26; KMT2A and ARHGEF12; KMT2A and BTBD18; KMT2A and CASC5; KMT2A and CASP8AP2; KMT2A and CBL; KMT2A and CREBBP; KMT2A and CT45A2; KMT2A and DAB2IP; KMT2A and EEFSEC; KMT2A and ELL; KMT2A and EP300; KMT2A and EPS15; KMT2A and FOXO3; KMT2A and FOXO4; KMT2A and FRYL; KMT2A and GAS7; KMT2A and GMPS; KMT2A and GPHN; KMT2A and KIAA0284; KMT2A and KIAA1524; KMT2A and LASP1; KMT2A and LPP; KMT2A and MAPRE1; KMT2A and MLLT1; KMT2A and MLLT10; KMT2A and MLLT11; KMT2A and MLLT3; KMT2A and MLLT4; KMT2A and MLLT6; KMT2A and MYOIF; KMT2A and NCKIPSD; KMT2A and NRIP3; KMT2A and PDS5A; KMT2A and PICALM; KMT2A and PRRC1; KMT2A and SARNP; KMT2A and SEPT2; KMT2A and SEPT5; KMT2A and SEPT6; KMT2A and SEPT9; KMT2A and SH3GL1; KMT2A and SORBS2; KMT2A and TET1; KMT2A and TOP3A; KMT2A and ZFYVE19; KTN1 and RET; LIFR and PLAG1; LMNA and NTRK1; LRIG3 and ROS1; LSM14A and BRAF; MARK4 and ERCC2; MBOAT2 and PRKCE; MBTD1 and CXorf67; MEAF6 and PHF1; MKRN1 and BRAF; MSN and ALK; MYB and NFIB; MYO5A and ROS1; NAB2 and STAT6; NACC2 and NTRK2; NCOA4 and RET; NDRG1 and ERG; NF1 and ACCN1; NFIA and EHF; NFIX and MAST1; NONO and TFE3; NOTCH1 and GABBR2; NPM1 and ALK; NTN1 and ACLY; NUP107 and LGR5; OMD and USP6; PAX3 and FOXO1; PAX3 and NCOA1; PAX3 and NCOA2; PAX5 and JAK2; PAX7 and FOXO1; PAX8 and PPARG; PCM1 and JAK2; PCM1 and RET; PLA2R1 and RBMS1; PLXND1 and TMCC1; PPFIBP1 and ALK; PPFIBP1 and ROS1; PRCC and TFE3; PRKAR1A and RET; PTPRK and RSPO3; PWWP2A and ROS1; QKI and NTRK2; RAF1 and DAZL; RANBP2 and ALK; RBM14 and PACS1; RGS22 and SYCP1; RNF130 and BRAF; SDC4 and ROS1; SEC16A_NM_014866.1 and NOTCH1; SEC31A and ALK; SEC31A and JAK2; SEPT8 and AFF4; SFPQ and TFE3; SLC22A1 and CUTA; SLC26A6 and PRKAR2A; SLC34A2 and ROS1; SLC45A3 and BRAF; SLC45A3 and ELK4; SLC45A3 and ERG; SLC45A3 and ETV1; SLC45A3 and ETV5; SND1 and BRAF; SQSTM1 and ALK; SRGAP3 and RAF1; SS18 and SSX1; SS18 and SSX2; SS18 and SSX4; SS18L1 and SSX1; SSBP2 and JAK2; SSH2 and SUZ12; STIL and TALI; STRN and ALK; SUSD1 and ROD1; TADA2A and MAST1; TAF15 and NR4A3; TCEA1 and PLAG1; TCF12 and NR4A3; TCF3 and PBX1; TECTA and TBCEL; TFG and ALK; TFG and NR4A3; TFG and NTRK1; THRAP3 and USP6; TMPRSS2 and ERG; TMPRSS2 and ETV1; TMPRSS2 and ETV4; TMPRSS2 and ETV5; TP53 and NTRK1; TPM3 and ALK; TPM3 and NTRK1; TPM3 and ROS1; TPM3 and ROS1; TPM4 and ALK; TRIM24 and RET; TRIM27 and RET; TRIM33 and RET; UBE2L3 and KRAS; VCL and ALK; VTI1A and TCF7L2; YWHAE and FAM22A; YWHAE and NUTM2B; ZC3H7B and BCOR; ZCCHC8 and ROS1; ZNF700 and MAST1; and ZSCAN30 and BRAF

4. The nucleic acid of any one of claims 1, 2 or 3 parts (a) and (b), wherein:
each nucleotide sequence of the plurality comprises a spanning subsequence of a nucleotide sequence selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, and SEQ ID NO:10; and
the spanning subsequence comprises the first subsequence and the second subsequence.

5. The nucleic acid of any one of claims 1-4, wherein:
(a) the plurality of nucleotide sequences consists of 2, 3, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, or 200 different nucleotide sequences; and/or
(b) the plurality of nucleotide sequences consists of 2 to 12 nucleotide sequences; and/or
(c) the plurality of nucleotide sequences consists of 6 nucleotide sequences; and/or
(d) each first subsequence and each second subsequence is at least 120 to 500 nucleotides long.

6. The nucleic acid of any one of claims 1-5, wherein:
(a) a first subsequence or a second subsequence of a nucleotide sequence of the plurality comprises two or more exons;
each exon of the two or more exons is an exon of the same gene; and
the exons of the two or more exons are ordered in the nucleic acid according to the order of the exons in a naturally-occurring mRNA,
optionally
wherein:
a first subsequence comprises two or more exons, and the first exon of the first subsequence is less than 200 nucleotides long; or
a second subsequence comprises two or more exons, and the second exon of the second subsequence is less than 200 nucleotides long; and/or
(b) the nucleic acid is as set out in part (a) and the sum of the lengths of the two or more exons is at least 50 nucleotides long or 120 to 500 nucleotides long; and/or
(c) the nucleic acid further comprises a nucleotide sequence comprising an intron, wherein:
the nucleotide sequence comprising an intron comprises a first subsequence and a second subsequence;
the first subsequence comprises a 3' subsequence of an intron or exon of a first gene;
the second subsequence comprises a 5' subsequence of an intron or exon of a second gene;
the first subsequence and second subsequence are adjoining sequences in the nucleic acid;
the first subsequence is 5' relative to the second subsequence; and
the first gene and second gene are the same gene or different genes;
optionally wherein:
the first subsequence comprises a 3' subsequence of an intron of a first gene; or
the second subsequence comprises a 5' subsequence of an intron of a second gene; and/or
(d) the nucleic acid is as set out in part (c) and the first gene and second gene of the nucleotide sequence comprising an intron are different genes or are the same gene; and/or
(e) the nucleic acid further comprises a poly-A tail.

7. The nucleic acid of claim 6, wherein the nucleic acid further comprises at least one methylated nucleoside, optionally wherein the at least one methylated nucleoside comprises 7-methyl guanosine, optionally wherein the nucleic acid comprises a 5' [m7G(5')ppp(5')G] cap.

8. The nucleic acid of claim 6, wherein:
(a) the nucleic acid further comprises a promoter, optionally wherein the promoter is an SP6 promoter; and/or
(b) the nucleic acid further comprises a promoter wherein the promoter is from a different species than the nucleotide sequences of the plurality, optionally wherein the promoter is an SP6 promoter; and/or
(c) the nucleic acid is a plasmid.

9. A method for making the nucleic acid of any one of claims 1-7, comprising incubating a reaction mixture comprising a DNA template, RNA polymerase, and ribonucleotide triphosphates at a temperature at which the RNA polymerase displays polymerase activity, thereby making the nucleic acid.

10. A cell comprising the nucleic acid of any one of claims 1-8.

11. The cell of claim 10, wherein:
(a) the cell is a human cell; and/or
(b) the cell is a fibroblast or a lymphocyte, optionally an immortalized B lymphocyte; and/or
(c) the cell is GM12878, GM24149, GM24143, GM24385, GM24631, GM24694, or GM24695,optionally GM24385; and/or
(d) the cell comprises 1 to 1000 copies or 5 to 500 copies of the nucleic acid; or
(e) the cell is *E. coli.*

12. A composition, comprising a first plurality of cells and a second plurality of cells, wherein:
the first plurality of cells consists of cells according to claim 11 part (d) which cells comprise 5 to 500 copies of the nucleic acid;
the second plurality of cells consists of cells that do not comprise the nucleic acid;
the first plurality of cells and the second plurality of cells are human cells;
the first plurality of cells and the second plurality of cells are admixed in the composition; and
the ratio of the number of cells of the first plurality to the number of cells of the second plurality is about 1:1 to about 1:10,000 in the composition.

13. A method for making a biological reference material, comprising transfecting a plurality of cells with the nucleic acid of any one of claims 1-8,
optionally further comprising:
(a) fixing the cells of the plurality, optionally wherein fixing the cells comprises fixing the cells with formalin; and/or
(b) embedding the cells in paraffin; and/or
(c) diluting the plurality of cells with untransfected cells, optionally wherein diluting the plurality of cells with untransfected cells comprises diluting at a ratio of transfected cells to untransfected cells of about 1:1 to about 1:10,000.

14. A biological reference material, comprising:
(a) a plurality of cells of any one of claims 10 or 11 parts (a)-(d) and paraffin, wherein the plurality of cells are fixed and embedded in the paraffin, optionally further comprising untransfected cells wherein the untransfected cells do not comprise the nucleic acid or the plurality of nucleic acid fragments, optionally wherein the ratio of cells comprising the nucleic acid or the plurality of nucleic acid fragments to untransfected cells is about 1:1 to about 1:10,000, about 1:5 to about 1:5,000 or about 1:10 to about 1:1,000; or
(b) a plurality of cells of any one of claims 10 or 11 parts (a)-(d); and a liquid, optionally wherein the liquid is blood plasma.

15. A composition comprising the nucleic acid of any one of claims 1-8, and an aqueous buffer, optionally wherein the buffer comprises tris(hydroxymethyl)aminomethane and ethylenediaminetetraacetic acid, or a salt of any one of the foregoing.

## Patentansprüche

1. Nukleinsäure, umfassend eine Vielzahl von Nukleotidsequenzen, wobei:
jede Nukleotidsequenz der Vielzahl eine erste Untersequenz und eine zweite Untersequenz umfasst;
die erste Untersequenz eine 3'-Sequenz eines ersten Exons umfasst;
die zweite Untersequenz eine 5'-Sequenz eines zweiten Exons umfasst;
die erste Untersequenz und die zweite Untersequenz benachbarte Sequenzen in der Nukleinsäure sind;
die erste Untersequenz und die zweite Untersequenz jeweils mindestens 20 Nukleotide lang sind;
die erste Untersequenz 5' relativ zur zweiten Untersequenz in der Nukleinsäure ist;
das erste Exon ein Exon eines ersten Gens ist;
das zweite Exon ein Exon eines zweiten Gens ist;
das erste Gen und das zweite Gen verschiedene Gene sind; und
jede Nukleotidsequenz der Vielzahl von Nukleotidsequenzen entweder eine 3'-Sequenz eines ersten Exons, das sich von jedem anderen ersten Exon der Nukleotidsequenzen der Vielzahl unterscheidet, oder eine 5'-Sequenz eines zweiten Exons, das sich von jedem anderen zweiten Exon der Nukleotidsequenzen der Vielzahl unterscheidet, ist.

2. Nukleinsäure nach Anspruch 1, wobei:
(a) die Nukleinsäure DNS oder RNS ist; und/oder
(b) jede Nukleotidsequenz der Vielzahl mit einem Neoplasma assoziiert ist, wobei das Neoplasma optional Lungenkrebs, nicht-kleinzelliger Lungenkrebs, Weichteilkrebs, Lymphkrebs, akute lymphatische Leukämie, akute myeloische Leukämie, chronische myeloische Leukämie, Non-Hodgkin-Lymphom, Burkitt-Lymphom, Melanom, intraokulares Melanom, Krebs des zentralen Nervensystems, Neuroblastom, Schilddrüsenkrebs, Nebenschilddrüsenkrebs, Leberzellkrebs, Magenkrebs, Dickdarmkrebs, Kolonkrebs, Harnwegskrebs, Blasenkrebs, Nierenkrebs, Prostatakrebs, Gebärmutterhalskrebs, Eierstockkrebs oder Brustkrebs ist.

3. Nukleinsäure nach Anspruch 1 oder 2, wobei:
(a) jedes erste Gen und jedes zweite Gen aus der Gruppe ausgewählt ist, die aus anaplastischer Lymphom-Rezeptor-Tyrosinkinase (ALK), gehirnspezifischer Angiogenese-Inhibitor 1-assoziiertes Protein 2-ähnlichem Protein 1 (BAIAP2L1), CD74, echinoderme Mikrotubuli-assoziiertem Protein-4 (EML4), ETS-Variante 6 (ETV6), Fibroblasten-Wachstumsfaktor-Rezeptor 3 (FGFR3), Kinesin-1-Schwerkette (KIF5B), Kernrezeptor-Koaktivator 4 (NCOA4), Nukleophosmin (NPM1), neurotropher Tyrosinrezeptorkinase 1 (NTRK1), neurotropher Tyrosinrezeptorkinase 3 (NTRK3), Paired-Box-Gen 8 (Pax8), Peroxisom-Proliferator-aktiviertem Rezeptor gamma (PPARG), RET-Proto-Onkogen (RET), ROS-Proto-Onkogen 1 (ROS1), Natrium-abhängigem Phosphat-Transportprotein SLC34A, transformierendem saurem Coiled-Coilenthaltendem Protein 3 (TACC3), TRK-fusioniertem Gen (TFG) und Tropomyosin 3 (TPM3) besteht;
wobei optional jedes erste Exon und jedes zweite Exon aus der Gruppe ausgewählt ist, die aus ALK Exon 20, BAIAP2L1 Exon 2, CD74 Exon 6, EML4 Exon 13, ETV6 Exon 5, FGFR3 Exon 18, KIF5B Exon 24, NCOA4 Exon 8, NPM1 Exon 5, NTRK1 Exon 10, NTRK3 Exon 13, PAX8 Exon 8, PPARG Exon 1, RET Exon 11, RET Exon 12, ROS1 Exon 34, SLC34A Exon 4, TACC3 Exon 11, TFG Exon 5 und TPM3 Exon 8 besteht;
optional wobei das erste Exon und das zweite Exon jeder Nukleotidsequenz aus der Gruppe ausgewählt ist, die aus EML4 Exon 13 und ALK Exon 20; NPM1 Exon 5 und ALK Exon 20; KIF5B Exon 24 und Ret Exon 11; NCOA4 Exon 8 und RET Exon 12; CD74 Exon 6 und ROS1 Exon 34; SLC34A Exon 4 und ROS1 Exon 34; TPM3 Exon 8 und NTRK1 Exon 10; TFG Exon 5 und NTRK1 Exon 10; FGFR3 Exon 18 und BAIAP2L1 Exon 2; FGFR3 Exon 18 und TACC3 Exon 11; PAX8 Exon 8 und PPARG Exon 1; bzw. ETV6 Exon 5 und NTRK3 Exon 13 besteht; oder
(b) jede Nukleotidsequenz der Vielzahl eine Untersequenz eines Gens umfasst, das aus der Gruppe ausgewählt ist, die aus anaplastischer Lymphom-Rezeptor-Tyrosinkinase (ALK), gehirnspezifischer Angiogenese-Inhibitor 1-assoziiertes Protein 2-ähnlichem Protein 1 (BAIAP2L1), CD74, echinoderme Mikrotubuli-assoziiertem Protein-4 (EML4), ETS-Variante 6 (ETV6), Fibroblasten-Wachstumsfaktor-Rezeptor 3 (FGFR3), Kinesin-1-Schwerkette (KIF5B), Kernrezeptor-Koaktivator 4 (NCOA4), Nukleophosmin (NPM1), neurotropher Tyrosinrezeptorkinase 1 (NTRK1), neurotropher Tyrosinrezeptorkinase 3 (NTRK3), Paired-Box-Gen 8 (Pax8), Peroxisom-Proliferator-aktiviertem Rezeptor gamma (PPARG), RET-Proto-Onkogen (RET), ROS-Proto-Onkogen 1 (ROS1), Natrium-abhängigem Phosphat-Transportprotein SLC34A, transformierendem saurem Coiled-Coilenthaltendem Protein 3 (TACC3), TRK-fusioniertem Gen (TFG) und Tropomyosin 3 (TPM3) besteht;
optional wobei jede Untersequenz eines Gens eine Untersequenz von einem einzelnen Exon des Gens ist;
optional wobei jedes einzelne Exon aus der Gruppe ausgewählt ist, die aus ALK Exon 20, BAIAP2L1 Exon 2, CD74 Exon 6, EML4 Exon 13, ETV6 Exon 5, FGFR3 Exon 18, KIF5B Exon 24, NCOA4 Exon 8, NPM1 Exon 5, NTRK1 Exon 10, NTRK3 Exon 13, PAX8 Exon 8, PPARG Exon 1, RET Exon 11, RET Exon 12, ROS1 Exon 34, SLC34A Exon 4, TACC3 Exon 11, TFG Exon 5 und TPM3 Exon 8 besteht;
optional wobei das erste Exon und das zweite Exon jeder Nukleotidsequenz aus der Gruppe ausgewählt sind, die aus EML4 Exon 13 und ALK Exon 20; NPM1 Exon 5 und ALK Exon 20; KIF5B Exon 24 und Ret Exon 11; NCOA4 Exon 8 und RET Exon 12; CD74 Exon 6 und ROS1 Exon 34; SLC34A Exon 4 und ROS1 Exon 34; TPM3 Exon 8 und NTRK1 Exon 10; TFG Exon 5 und NTRK1 Exon 10; FGFR3 Exon 18 und BAIAP2L1 Exon 2; FGFR3 Exon 18 und TACC3 Exon 11; PAX8 Exon 8 und PPARG Exon 1; bzw. ETV6 Exon 5 und NTRK3 Exon 13 besteht; oder
(c) jedes erste Gen bzw. jedes zweite Gen aus der Gruppe ausgewählt ist, die aus ACBD6 und RRP15; ACSL3 und ETV1; ACTB und GLI1; AGPAT5 und MCPH1; AGTRAP und BRAF; AKAP9 und BRAF; ARFIP1 und FHDC1; ARID1A und MAST2; ASPSCR1 und TFE3; ATG4C und FBXO38; ATIC und ALK; BBS9 und PKD1L1; BCR und ABL1; BCR und JAK2; BRD3 und NUTM1; BRD4 und NUTM1; C2orf44 und ALK; CANT1 und ETV4; CARS und ALK; CCDC6 und RET; CD74 und NRG1; CD74 und ROS1; CDH11 und USP6; CDKN2D und WDFY2; CEP89 und BRAF; CHCHD7 und PLAG1; CIC und DUX4L1; CIC und FOXO4; CLCN6 und BRAF; CLIP1 und ROS1; CLTC und ALK; CLTC und TFE3; CNBP und USP6; COL1A1 und PDGFB; COL1A1 und USP6; COL1A2 und PLAG1; CRTC1 und MAML2; CRTC3 und MAML2; CTAGE5 und SIP1; CTNNB1 und PLAGI; DCTN1 und ALK; DDX5 und ETV4; DNAJB1 und PRKACA; EIF3E und RSPO2; EIF3K und CYP39A1; EML4 und ALK; EPC1 und PHF1; ERC1 und RET; ERC1 und ROSI; ERO1L und FERMT2; ESRP1 und RAFI; ETV6 und ITPR2; ETV6 und JAK2; ETV6 und NTRK3; EWSR1 und ATF1; EWSR1 und CREB1; EWSR1 und DDIT3; EWSR1 und ERG; EWSR1 und ETV1; EWSR1 und ETV4; EWSR1 und FEV; EWSR1 und FLI1; EWSR1 und NFATC1; EWSR1 und NFATC2; EWSR1 und NR4A3; EWSR1 und PATZ1; EWSR1 und PBX1; EWSR1 und POU5F1; EWSR1 und SMARCA5; EWSR1 und SP3; EWSR1 und WT1; EWSR1 und YY1; EWSR1 und ZNF384; EWSR1 und ZNF444; EZR und ROS1; FAM131B und BRAF; FBXL18 und RNF216; FCHSD1 und BRAF; FGFR1 und ZNF703; FGFR1 und PLAG1; FGFR1 und TACC1; FGFR3 und BAIAP2L1; FGFR3 und TACC3; FN1 und ALK; FUS und ATF1; FUS und CREB3L1; FUS und CREB3L2; FUS und DDIT3; FUS und ERG; FUS und FEV; GATM und BRAF; GMDS und PDE8B; GNAU und BRAF; G0LGA5 und RET; GOPC und ROSI; GPBP1L1 und MAST2; HACL1 und RAF1; HAS2 und PLAG1; HERPUD1 und BRAF; HEY1 und NC0A2; HIP1 und ALK; HLA-A und ROS1; HMGA2 und ALDH2; HMGA2 und CCNB1IP1; HMGA2 und COX6C; HMGA2 und EBF1; HMGA2 und FHIT; HMGA2 und LHFP; HMGA2 und LPP; HMGA2 und NFIB; HMGA2 und RAD51B; HMGA2 und WIF1; HN1 und USH1G; HNRNPA2B1 und ETV1; H00K3 und RET; IL6R und ATP8B2; INTS4 und GAB2; IRF2BP2 und CDX1; JAZF1 und PHF1; JAZF1 und SUZ12; KIAA1549 und BRAF; KIAA1598 und ROS1; KIF5B und ALK; KIF5B und RET; KLC1 und ALK; KLK2 und ETV1; KLK2 und ETV4; KMT2A und ABU; KMT2A und ABI2; KMT2A und ACTN4; KMT2A und AFF1; KMT2A und AFF3; KMT2A und AFF4; KMT2A und ARHGAP26; KMT2A und ARHGEF12; KMT2A und BTBD18; KMT2A und CASC5; KMT2A und CASP8AP2; KMT2A und CBL; KMT2A und CREBBP; KMT2A und CT45A2; KMT2A und DAB2IP; KMT2A und EEFSEC; KMT2A und ELL; KMT2A und EP300; KMT2A und EPS15; KMT2A und FOXO3; KMT2A und FOXO4; KMT2A und FRYL; KMT2A und GAS7; KMT2A und GMPS; KMT2A und GPHN; KMT2A und KIAA0284; KMT2A und KIAA1524; KMT2A und LASP1; KMT2A und LPP; KMT2A und MAPRE1; KMT2A und MLLT1; KMT2A und MLLT10; KMT2A und MLLT11; KMT2A und MLLT3; KMT2A und MLLT4; KMT2A und MLLT6; KMT2A und MYO1F; KMT2A und NCKIPSD; KMT2A und NRIP3; KMT2A und PDS5A; KMT2A und PICALM; KMT2A und PRRC1; KMT2A und SARNP; KMT2A und SEPT2; KMT2A und SEPT5; KMT2A und SEPT6; KMT2A und SEPT9; KMT2A und SH3GL1; KMT2A und SORBS2; KMT2A und TET1; KMT2A und TOP3A; KMT2A und ZFYVE19; KTN1 und RET; LIFR und PLAG1; LMNA und NTRK1; LRIG3 und ROS1; LSM14A und BRAF; MARK4 und ERCC2; MB0AT2 und PRKCE; MBTD1 und CXorf67; MEAF6 und PHF1; MKRN1 und BRAF; MSN und ALK; MYB und NFIB; MYO5A und ROS1; NAB2 und STAT6; NACC2 und NTRK2; NCOA4 und RET; NDRG1 und ERG; NF1 und ACCN1; NFIA und EHF; NFIX und MASTI; NONO und TFE3; NOTCH1 und GABBR2; NPM1 und ALK; NTN1 und ACLY; NUP107 und LGR5; OMD und USP6; PAX3 und FOXO1; PAX3 und NCOA1; PAX3 und NCOA2; PAX5 und JAK2; PAX7 und FOXO1; PAX8 und PPARG; PCM1 und JAK2; PCM1 und RET; PLA2R1 und RBMS1; PLXND1 und TMCC1; PPFIBP1 und ALK; PPFIBP1 und ROSI; PRCC und TFE3; PRKAR1A und RET; PTPRK und RSPO3; PWWP2A und ROSI; QKI und NTRK2; RAFI und DAZL; RANBP2 und ALK; RBM14 und PACS1; RGS22 und SYCP1; RNF130 und BRAF; SDC4 und ROS1; SEC16A_NM_014866.1 und NOTCH1; SEC31A und ALK; SEC31A und JAK2; SEPT8 und AFF4; SFPQ und TFE3; SLC22A1 und CUTA; SLC26A6 und PRKAR2A; SLC34A2 und ROS1; SLC45A3 und BRAF; SLC45A3 und ELK4; SLC45A3 und ERG; SLC45A3 und ETV1; SLC45A3 und ETV5; SND1 und BRAF; SQSTM1 und ALK; SRGAP3 und RAFI; SS18 und SSX1; SS18 und SSX2; SS18 und SSX4; SS18L1 und SSX1; SSBP2 und JAK2; SSH2 und SUZ12; STIL und TALI; STRN und ALK; SUSD1 und RODI; TADA2A und MASTI; TAF15 und NR4A3; TCEA1 und PLAGI; TCF12 und NR4A3; TCF3 und PBX1; TECTA und TBCEL; TFG und ALK; TFG und NR4A3; TFG und NTRK1; THRAP3 und USP6; TMPRSS2 und ERG; TMPRSS2 und ETV1; TMPRSS2 und ETV4; TMPRSS2 und ETV5; TP53 und NTRK1; TPM3 und ALK; TPM3 und NTRK1; TPM3 und ROS1; TPM3 und ROS1; TPM4 und ALK; TRIM24 und RET; TRIM27 und RET; TRIM33 und RET; UBE2L3 und KRAS; VCL und ALK; VTI1A und TCF7L2; YWHAE und FAM22A; YWHAE und NUTM2B; ZC3H7B und BCOR; ZCCHC8 und ROS1; ZNF700 und MAST1; und ZSCAN30 und BRAF besteht.

4. Nukleinsäure nach einem der Ansprüche 1, 2 oder 3, Teile (a) und (b), wobei:
jede Nukleotidsequenz der Vielzahl eine überspannende Untersequenz einer Nukleotidsequenz umfasst, die aus der Gruppe ausgewählt ist, die aus SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 und SEQ ID NO: 10 besteht; und
die überspannende Untersequenz die erste Untersequenz und die zweite Untersequenz umfasst.

5. Nukleinsäure nach einem der Ansprüche 1-4, wobei:
(a) die Vielzahl von Nukleotidsequenzen aus 2, 3, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199 oder 200 verschiedenen Nukleotidsequenzen besteht; und/oder
(b) die Vielzahl von Nukleotidsequenzen aus 2 bis 12 Nukleotidsequenzen besteht; und/oder
(c) die Vielzahl von Nukleotidsequenzen aus 6 Nukleotidsequenzen besteht; und/oder
(d) jede erste Untersequenz und jede zweite Untersequenz mindestens 120 bis 500 Nukleotide lang ist.

6. Nukleinsäure nach einem der Ansprüche 1-5, wobei:
(a) eine erste Untersequenz oder eine zweite Untersequenz einer Nukleotidsequenz der Vielzahl zwei oder mehr Exons umfasst;
jedes Exon der zwei oder mehr Exons ein Exon desselben Gens ist; und
die Exons der zwei oder mehr Exons in der Nukleinsäure entsprechend der Reihenfolge der Exons in einer natürlich vorkommenden mRNA geordnet sind,
optional
wobei:
eine erste Untersequenz zwei oder mehr Exons umfasst und das erste Exon der ersten Untersequenz weniger als 200 Nukleotide lang ist; oder
eine zweite Untersequenz zwei oder mehr Exons umfasst und das zweite Exon der zweiten Untersequenz weniger als 200 Nukleotide lang ist; und/oder
(b) die Nukleinsäure wie in Teil (a) dargelegt ist und die Summe der Längen der zwei oder mehr Exons mindestens 50 Nukleotide lang ist oder 120 bis 500 Nukleotide lang ist; und/oder
(c) die Nukleinsäure ferner eine Nukleotidsequenz umfasst, die ein Intron umfasst, wobei:
die Nukleotidsequenz, die ein Intron umfasst, eine erste Untersequenz und eine zweite Untersequenz umfasst;
die erste Untersequenz eine 3'-Untersequenz eines Introns oder Exons eines ersten Gens umfasst;
die zweite Untersequenz eine 5'-Untersequenz eines Introns oder Exons eines zweiten Gens umfasst;
die erste Untersequenz und die zweite Untersequenz benachbarte Sequenzen in der Nukleinsäure sind;
die erste Untersequenz 5' relativ zur zweiten Untersequenz ist; und
das erste Gen und das zweite Gen das gleiche Gen oder verschiedene Gene sind; optional wobei:
die erste Untersequenz eine 3'-Untersequenz eines Introns eines ersten Gens umfasst; oder
die zweite Untersequenz eine 5'-Untersequenz eines Introns eines zweiten Gens umfasst; und/oder
(d) die Nukleinsäure wie in Teil (c) dargelegt ist und das erste Gen und das zweite Gen der Nukleotidsequenz, die ein Intron umfasst, verschiedene Gene oder dasselbe Gen sind; und/oder
(e) die Nukleinsäure ferner einen Poly(A)-Schwanz umfasst.

7. Nukleinsäure nach Anspruch 6, wobei die Nukleinsäure ferner mindestens ein methyliertes Nukleosid umfasst, optional wobei das mindestens eine methylierte Nukleosid 7-Methylguanosin umfasst, optional wobei die Nukleinsäure ein 5'-[m7G(5')ppp(5')G]-Cap umfasst.

8. Nukleinsäure nach Anspruch 6, wobei:
(a) die Nukleinsäure ferner einen Promotor umfasst, wobei der Promotor optional ein SP6-Promotor ist; und/oder
(b) die Nukleinsäure ferner einen Promotor umfasst, wobei der Promotor von einer anderen Spezies als die Nukleotidsequenzen der Vielzahl stammt, wobei der Promotor optional ein SP6-Promotor ist; und/oder
(c) die Nukleinsäure ein Plasmid ist.

9. Verfahren zum Herstellen der Nukleinsäure nach einem der Ansprüche 1-7, umfassend das Inkubieren einer Reaktionsmischung, die eine DNS-Matrize, RNS-Polymerase und Ribonukleotidtriphosphate umfasst, bei einer Temperatur, bei der die RNS-Polymerase Polymeraseaktivität zeigt, wodurch die Nukleinsäure hergestellt wird.

10. Zelle, umfassend die Nukleinsäure nach einem der Ansprüche 1-8.

11. Zelle nach Anspruch 10, wobei:
(a) die Zelle eine menschliche Zelle ist; und/oder
(b) die Zelle ein Fibroblast oder ein Lymphozyt ist, optional ein immortalisierter B-Lymphozyt; und/oder
(c) die Zelle GM12878, GM24149, GM24143, GM24385, GM24631, GM24694 oder GM24695, optional GM24385 ist; und/oder
(d) die Zelle 1 bis 1000 Kopien oder 5 bis 500 Kopien der Nukleinsäure umfasst; oder
(e) die Zelle *E. coli* ist.

12. Zusammensetzung, umfassend eine erste Vielzahl von Zellen und eine zweite Vielzahl von Zellen, wobei:
die erste Vielzahl von Zellen aus Zellen nach Anspruch 11, Teil (d), besteht, wobei die Zellen 5 bis 500 Kopien der Nukleinsäure umfassen;
die zweite Vielzahl von Zellen aus Zellen besteht, die die Nukleinsäure nicht umfassen; wobei die erste Vielzahl von Zellen und die zweite Vielzahl von Zellen menschliche Zellen sind;
die erste Vielzahl von Zellen und die zweite Vielzahl von Zellen in die Zusammensetzung gemischt werden; und
das Verhältnis der Anzahl der Zellen der ersten Vielzahl zur Anzahl der Zellen der zweiten Vielzahl in der Zusammensetzung etwa 1:1 bis etwa 1:10.000 beträgt.

13. Verfahren zum Herstellen eines biologischen Referenzmaterials, umfassend das Transfizieren einer Vielzahl von Zellen mit der Nukleinsäure nach einem der Ansprüche 1-8,
optional ferner umfassend:
(a) das Fixieren der Zellen der Vielzahl, optional wobei das Fixieren der Zellen das Fixieren der Zellen mit Formalin umfasst; und/oder
(b) das Einbetten der Zellen in Paraffin; und/oder
(c) das Verdünnen der Vielzahl von Zellen mit nicht transfizierten Zellen, optional wobei das Verdünnen der Vielzahl von Zellen mit nicht transfizierten Zellen das Verdünnen in einem Verhältnis von transfizierten Zellen zu nicht transfizierten Zellen von etwa 1:1 bis etwa 1:10.000 umfasst.

14. Biologisches Referenzmaterial, umfassend:
(a) eine Vielzahl von Zellen nach einem der Ansprüche 10 oder 11, Teile (a)-(d), und Paraffin, wobei die Vielzahl von Zellen fixiert und in das Paraffin eingebettet ist, optional ferner umfassend nicht transfizierte Zellen, wobei die nicht transfizierten Zellen die Nukleinsäure oder die Vielzahl von Nukleinsäurefragmenten nicht umfassen, optional wobei das Verhältnis von Zellen, die die Nukleinsäure oder die Vielzahl von Nukleinsäurefragmenten umfassen, zu nicht transfizierten Zellen etwa 1:1 bis etwa 1:10.000, etwa 1:5 bis etwa 1:5.000 oder etwa 1:10 bis etwa 1:1.000 beträgt; oder
(b) eine Vielzahl von Zellen nach einem der Ansprüche 10 oder 11, Teile (a)-(d); und eine Flüssigkeit, optional wobei die Flüssigkeit Blutplasma ist.

15. Zusammensetzung, umfassend die Nukleinsäure nach einem der Ansprüche 1-8 und einen wässrigen Puffer, optional wobei der Puffer Tris(hydroxymethyl)aminomethan und Ethylendiamintetraessigsäure oder ein Salz von einem der Vorhergehenden umfasst.

## Revendications

1. Acide nucléique, comprenant une pluralité de séquences nucléotidiques, dans lequel :
chaque séquence nucléotidique de la pluralité comprend une première sous-séquence et une seconde sous-séquence ;
la première sous-séquence comprend une séquence 3' d'un premier exon ;
la seconde sous-séquence comprend une séquence 5' d'un second exon ;
la première sous-séquence et la seconde sous-séquence sont des séquences adjacentes dans l'acide nucléique ;
la première sous-séquence et la seconde sous-séquence ont chacune au moins 20 nucléotides de long ;
la première sous-séquence est 5' par rapport à la seconde sous-séquence dans l'acide nucléique ;
le premier exon est un exon d'un premier gène ;
le second exon est un exon d'un second gène ;
le premier gène et le second gène sont des gènes différents ; et
chaque séquence nucléotidique de la pluralité de séquences nucléotidiques comprend soit une séquence 3' d'un premier exon qui est différente de chaque autre premier exon des séquences nucléotidiques de la pluralité, soit une séquence 5' d'un second exon qui est différente de chaque autre second exon des séquences nucléotidiques de la pluralité.

2. Acide nucléique selon la revendication 1, dans lequel :
(a) l'acide nucléique est de l'ADN ou de l'ARN ; et/ou
(b) chaque séquence nucléotidique de la pluralité est associée à un néoplasme, le néoplasme étant éventuellement un cancer du poumon, un cancer du poumon non à petites cellules, un cancer des tissus mous, un cancer lymphoïde, une leucémie lymphoïde aiguë, une leucémie myéloïde aiguë, une leucémie myéloïde chronique, un lymphome non-hodgkinien, un lymphome de Burkitt, un mélanome, un mélanome intraoculaire, un cancer du système nerveux central, un neuroblastome, un cancer de la thyroïde, un cancer de la parathyroïde, un cancer hépatocellulaire, un cancer de l'estomac, un cancer du gros intestin, un cancer du côlon, un cancer des voies urinaires, un cancer de la vessie, un cancer du rein, un cancer de la prostate, un cancer du col de l'utérus, un cancer de l'ovaire ou un cancer du sein.

3. Acide nucléique selon les revendications 1 ou 2, dans lequel :
(a) chaque premier gène et chaque second gène est choisi dans le groupe constitué par le récepteur de la tyROS1ne kinase du lymphome anaplasique (ALK), la protéine 1 de type protéine 2 associée à l'inhibiteur 1 de l'angiogenèse spécifique au cerveau (BAIAP2L1), CD74, le type 4 de protéine associée au microtubule d'échinoderme (EML4), le variant ETS 6 (ETV6), le récepteur 3 du facteur de croissance des fibroblastes (FGFR3), la chaîne lourde de la kinésine-1 (KIF5B), le coactivateur de récepteur nucléaire 4 (NCOA4), la nucléophosmine (NPM1), le récepteur neurotrophique de la tyROS1ne kinase 1 (NTRK1), le récepteur neurotrophique de la tyROS1ne kinase 3 (NTRK3), la paired box de gène 8 (Pax8), les récepteurs activés par les proliférateurs des péroxysomes gamma (PPARG), le proto-oncogène RET (RET), le proto-oncogène 1 ROS (ROS1), la protéine de transport de phosphate dépendant du sodium SLC34A, la protéine 3 transformante acide contenant une superhélice (TACC3), le gène fusionné avec TRK (TFG) et la tropomyosine 3 (TPM3) ;
éventuellement dans lequel chaque premier exon et chaque second exon est choisi dans le groupe constitué par ALK exon 20, BAIAP2L1 exon 2, CD74 exon 6, EML4 exon 13, ETV6 exon 5, FGFR3 exon 18, KIF5B exon 24, NCOA4 exon 8, NPM1 exon 5, NTRK1 exon 10, NTRK3 exon 13, PAX8 exon 8, PPARG exon 1, RET exon 11, RET exon 12, ROS1 exon 34, SLC34A exon 4, TACC3 exon 11, TFG exon 5, et TPM3 exon 8 ;
éventuellement dans lequel le premier exon et le second exon de chaque séquence nucléotidique est choisi dans le groupe constitué par EML4 exon 13 et ALK exon 20 ; NPM1 exon 5 et ALK exon 20 ; KIF5B exon 24 et Ret exon 11 ; NCOA4 exon 8 et RET exon 12 ; CD74 exon 6 et ROS1 exon 34 ; SLC34A exon 4 et ROS1 exon 34 ; TPM3 exon 8 et NTRK1 exon 10 ; TFG exon 5 et NTRK1 exon 10 ; FGFR3 exon 18 et BAIAP2L1 exon 2 ; FGFR3 exon 18 et TACC3 exon 11 ; PAX8 exon 8 et PPARG exon 1 ; et ETV6 exon 5 et NTRK3 exon 13, respectivement ; ou
(b) chaque séquence nucléotidique de la pluralité comprend une sous-séquence d'un gène choisi dans le groupe constitué par le récepteur de la tyROS1ne kinase du lymphome anaplasique (ALK), la protéine 1 de type protéine 2 associée à l'inhibiteur 1 de l'angiogenèse spécifique au cerveau (BAIAP2L1), CD74, le type 4 de protéine associée au microtubule d'échinoderme (EML4), le variant ETS 6 (ETV6), le récepteur 3 du facteur de croissance des fibroblastes (FGFR3), la chaîne lourde de la kinésine-1 (KIF5B), le coactivateur de récepteur nucléaire 4 (NCOA4), la nucléophosmine (NPM1), le récepteur neurotrophique de la tyROS1ne kinase 1 (NTRK1), le récepteur neurotrophique de la tyROS1ne kinase 3 (NTRK3), la paired box de gène 8 (Pax8), les récepteurs activés par les proliférateurs des péroxysomes gamma (PPARG), le proto-oncogène RET (RET), le proto-oncogène 1 ROS (ROS1), la protéine de transport de phosphate dépendant du sodium SLC34A, la protéine 3 transformante acide contenant une superhélice (TACC3), le gène fusionné avec TRK (TFG) et la tropomyosine 3 (TPM3) ;
éventuellement dans lequel chaque sous-séquence d'un gène est une sous-séquence d'un seul exon du gène ;
éventuellement dans lequel chaque exon unique est choisi dans le groupe constitué par ALK exon 20, BAIAP2L1 exon 2, CD74 exon 6, EML4 exon 13, ETV6 exon 5, FGFR3 exon 18, KIF5B exon 24, NCOA4 exon 8, NPM1 exon 5, NTRK1 exon 10, NTRK3 exon 13, PAX8 exon 8, PPARG exon 1, RET exon 11, RET exon 12, ROS1 exon 34, SLC34A exon 4, TACC3 exon 11, TFG exon 5, et TPM3 exon 8 ;
éventuellement dans lequel le premier exon et le second exon de chaque séquence nucléotidique sont choisis dans le groupe constitué par EML4 exon 13 et ALK exon 20 ; NPMI exon 5 et ALK exon 20 ; KIF5B exon 24 et Ret exon 11 ; NCOA4 exon 8 et RET exon 12 ; CD74 exon 6 et ROS1 exon 34 ; SLC34A exon 4 et ROS1 exon 34 ; TPM3 exon 8 et NTRK1 exon 10 ; TFG exon 5 et NTRK1 exon 10 ; FGFR3 exon 18 et BAIAP2L1 exon 2 ; FGFR3 exon 18 et TACC3 exon 11 ; PAX8 exon 8 et PPARG exon I ; et ETV6 exon 5 et NTRK3 exon 13, respectivement ; ou
(c) chaque premier gène et chaque second gène, respectivement, est choisi dans le groupe constitué par ACBD6 et RRP15 ; ACSL3 et ETV1 ; ACTB et GLI1 ; AGPAT5 et MCPH1 ; AGTRAP et BRAF ; AKAP9 et BRAF ; ARFIP1 et FHDC1 ; ARID1A et MAST2 ; ASPSCR1 et TFE3 ; ATG4C et FBXO38 ; ATIC et ALK ; BBS9 et PKD1L1 ; BCR et ABL1 ; BCR et JAK2 ; BRD3 et NUTM1 ; BRD4 et NUTM1 ; C2orf44 et ALK ; CANT1 et ETV4 ; CARS et ALK ; CCDC6 et RET ; CD74 et NRG1 ; CD74 et ROS1 ; CDH11 et USP6 ; CDKN2D et WDFY2 ; CEP89 et BRAF ; CHCHD7 et PLAG1 ; CIC et DUX4L1 ; CIC et FOXO4 ; CLCN6 et BRAF ; CLIP1 et ROS1 ; CLTC et ALK ; CLTC et TFE3 ; CNBP et USP6 ; COL1A1 et PDGFB ; COL1A1 et USP6 ; COL1A2 et PLAG1 ; CRTC1 et MAML2 ; CRTC3 et MAML2 ; CTAGE5 et SIP1 ; CTNNB1 et PLAG1 ; DCTN1 et ALK ; DDX5 et ETV4 ; DNAJB1 et PRKACA ; EIF3E et RSPO2 ; EIF3K et CYP39A1 ; EML4 et ALK ; EPC1 et PHF1 ; ERC1 et RET ; ERC1 et ROS1 ; ERO1L et FERMT2 ; ESRP1 et RAF1 ; ETV6 et ITPR2 ; ETV6 et JAK2 ; ETV6 et NTRK3 ; EWSR1 et ATF1 ; EWSR1 et CREB1 ; EWSR1 et DDIT3 ; EWSR1 et ERG ; EWSR1 et ETV1 ; EWSR1 et ETV4 ; EWSR1 et FEV ; EWSR1 et FLI1 ; EWSR1 et NFATC1 ; EWSR1 et NFATC2 ; EWSR1 et NR4A3 ; EWSR1 et PATZ1 ; EWSR1 et PBX1 ; EWSR1 et POU5F1 ; EWSR1 et SMARCA5 ; EWSR1 et SP3 ; EWSR1 et WT1 ; EWSR1 et YY1 ; EWSR1 et ZNF384 ; EWSR1 et ZNF444 ; EZR et ROS1 ; FAM131B et BRAF ; FBXLI8 et RNF216 ; FCHSDI et BRAF ; FGFR1 et ZNF703 ; FGFR1 et PLAG1 ; FGFR1 et TACC1 ; FGFR3 et BAIAP2L1 ; FGFR3 et TACC3 ; FN1 et ALK ; FUS et ATF1 ; FUS et CREB3L1 ; FUS et CREB3L2 ; FUS et DDIT3 ; FUS et ERG ; FUS et FEV ; GATM et BRAF ; GMDS et PDE8B ; GNAI1 et BRAF ; GOLGA5 et RET ; GOPC et ROS1 ; GPBP1L1 et MAST2 ; HACL1 et RAF1 ; HAS2 et PLAG1 ; HERPUD1 et BRAF ; HEY1 et NCOA2 ; HIP1 et ALK ; HLA-A et ROS1 ; HMGA2 et ALDH2 ; HMGA2 et CCNB1IP1 ; HMGA2 et COX6C ; HMGA2 et EBF1 ; HMGA2 et FHIT ; HMGA2 et LHFP ; HMGA2 et LPP ; HMGA2 et NF1B ; HMGA2 et RAD51B ; HMGA2 et WIF1 ; HN1 et USH1G ; HNRNPA2B1 et ETV1 ; HOOK3 et RET ; IL6R et ATP8B2 ; INTS4 et GAB2 ; IRF2BP2 et CDX1 ; JAZF1 et PHF1 ; JAZF1 et SUZ12 ; KIAA1549 et BRAF ; KIAA1598 et ROS1 ; KIF5B et ALK ; KIF5B et RET ; KLC1 et ALK ; KLK2 et ETV1 ; KLK2 et ETV4 ; KMT2A et ABI1 ; KMT2A et ABI2 ; KMT2A et ACTN4 ; KMT2A et AFF1 ; KMT2A et AFF3 ; KMT2A et AFF4 ; KMT2A et ARHGAP26 ; KMT2A et ARHGEF12 ; KMT2A et BTBD18 ; KMT2A et CASC5 ; KMT2A et CASP8AP2 ; KMT2A et CBL ; KMT2A et CREBBP ; KMT2A et CT45A2 ; KMT2A et DAB21P ; KMT2A et EEFSEC ; KMT2A et ELL ; KMT2A et EP300 ; KMT2A et EPS15 ; KMT2A et FOXO3 ; KMT2A et FOXO4 ; KMT2A et FRYL ; KMT2A et GAS7 ; KMT2A et GMPS ; KMT2A et GPHN ; KMT2A et KIAA0284 ; KMT2A et KIAA1524 ; KMT2A et LASP1 ; KMT2A et LPP ; KMT2A et MAPRE1 ; KMT2A et MLLT1 ; KMT2A et MLLT10 ; KMT2A et MLLT11 ; KMT2A et MLLT3 ; KMT2A et MLLT4 ; KMT2A et MLLT6 ; KMT2A et MYO1F ; KMT2A et NCKIPSD ; KMT2A et NRIP3 ; KMT2A et PDS5A ; KMT2A et PICALM ; KMT2A et PRRC1 ; KMT2A et SARNP ; KMT2A et SEPT2 ; KMT2A et SEPT5 ; KMT2A et SEPT6 ; KMT2A et SEPT9 ; KMT2A et SH3GL1 ; KMT2A et SORBS2 ; KMT2A et TET1 ; KMT2A et TOP3A ; KMT2A et ZFYVE19 ; KTN1 et RET ; LIFR et PLAG1 ; LMNA et NTRK1 ; LRIG3 et ROS1 ; LSM14A et BRAF ; MARK4 et ERCC2 ; MBOAT2 et PRKCE ; MBTD1 et CXorf67 ; MEAF6 et PHF1 ; MKRN1 et BRAF ; MSN et ALK ; MYB et NF1B ; MYO5A et ROS1 ; NAB2 et STAT6 ; NACC2 et NTRK2 ; NCOA4 et RET ; NDRG1 et ERG ; NF1 et ACCN1 ; NF1A et EHF ; NF1X et MAST1 ; NONO et TFE3 ; NOTCH1 et GABBR2 ; NPM1 et ALK; NTN1 et ACLY ; NUP107 et LGR5 ; OMD et USP6 ; PAX3 et FOXO1 ; PAX3 et NCOA1 ; PAX3 et NCOA2 ; PAX5 et JAK2 ; PAX7 et FOXO1 ; PAX8 et PPARG ; PCM1 et JAK2 ; PCM1 et RET ; PLA2R1 et RBMS1 ; PLXND1 et TMCC1 ; PPFIBP1 et ALK ; PPFIBP1 et ROS1 ; PRCC et TFE3 ; PRKARIA et RET ; PTPRK et RSPO3 ; PWWP2A et ROS1 ; QKI et NTRK2 ; RAF1 et DAZL ; RANBP2 et ALK ; RBM14 et PACS1 ; RGS22 et SYCP1 ; RNF130 et BRAF ; SDC4 et ROS1 ; SEC16A_NM_014866. 1 et NOTCH1 ; SEC31A et ALK ; SEC31A et JAK2 ; SEPT8 et AFF4 ; SFPQ et TFE3 ; SLC22A1 et CUTA ; SLC26A6 et PRKAR2A ; SLC34A2 et ROS1 ; SLC45A3 et BRAF ; SLC45A3 et ELK4 ; SLC45A3 et ERG ; SLC45A3 et ETV1 ; SLC45A3 et ETV5 ; SND1 et BRAF ; SQSTM1 et ALK ; SRGAP3 et RAF1 ; SS18 et SSX1 ; SS18 et SSX2 ; SS18 et SSX4 ; SS18L1 et SSX1 ; SSBP2 et JAK2 ; SSH2 et SUZ12 ; STIL et TAL1 ; STRN et ALK ; SUSD1 et ROD1 ; TADA2A et MAST1 ; TAF15 et NR4A3 ; TCEA1 et PLAG1 ; TCF12 et NR4A3 ; TCF3 et PBX1 ; TECTA et TBCEL ; TFG et ALK ; TFG et NR4A3 ; TFG et NTRK1 ; THRAP3 et USP6 ; TMPRSS2 et ERG ; TMPRSS2 et ETV1 ; TMPRSS2 et ETV4 ; TMPRSS2 et ETV5 ; TP53 et NTRK1 ; TPM3 et ALK ; TPM3 et NTRK1 ; TPM3 et ROS1 ; TPM3 et ROS1 ; TPM4 et ALK ; TRIM24 et RET ; TRIM27 et RET ; TRIM33 et RET ; UBE2L3 et KRAS ; VCL et ALK ; VTI1A et TCF7L2 ; YWHAE et FAM22A ; YWHAE et NUTM2B ; ZC3H7B et BCOR ; ZCCHC8 et ROS1 ; ZNF700 et MAST1 ; et ZSCAN30 et BRAF.

4. Acide nucléique selon l'une quelconque des revendications 1, 2 ou 3 parties (a) et (b), dans lequel :
chaque séquence nucléotidique de la pluralité comprend une sous-séquence étendue d'une séquence nucléotidique choisie dans le groupe constitué de SEQ ID NO : 1, SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9 et SEQ ID NO : 10 ; et
la sous-séquence étendue comprend la première sous-séquence et la seconde sous-séquence.

5. Acide nucléique selon l'une quelconque des revendications 1 à 4, dans lequel :
(a) la pluralité de séquences nucléotidiques est constituée de 2, 3, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, ou 200 séquences nucléotidiques différentes ; et/ou
(b) la pluralité de séquences nucléotidiques est constituée de 2 à 12 séquences nucléotidiques ; et/ou
(c) la pluralité de séquences nucléotidiques est constituée de 6 séquences nucléotidiques ; et/ou
(d) chaque première sous-séquence et chaque seconde sous-séquence est d'au moins 120 à 500 nucléotides de long.

6. Composé selon l'une quelconque des revendications 1 à 5, dans lequel :
(a) une première sous-séquence ou une seconde sous-séquence d'une séquence nucléotidique de la pluralité comprend deux exons ou plus ;
chaque exon des deux exons ou plus est un exon du même gène ; et
les exons des deux exons ou plus sont ordonnés dans l'acide nucléique selon l'ordre des exons dans un ARNm naturel,
éventuellement
dans lequel :
une première sous-séquence comprend deux exons ou plus, et le premier exon de la première sous-séquence est inférieur à 200 nucléotides de long ; ou
une seconde sous-séquence comprend deux exons ou plus, et le second exon de la seconde sous-séquence est inférieur à 200 nucléotides de long ; et/ou
(b) l'acide nucléique est tel que décrit dans la partie (a) et la somme des longueurs des deux exons ou plus est d'au moins 50 nucléotides de long ou de 120 à 500 nucléotides de long ; et/ou
(c) l'acide nucléique comprend en outre une séquence nucléotidique comprenant un intron, dans lequel :
la séquence nucléotidique comprenant un intron comprend une première sous-séquence et une seconde sous-séquence ;
la première sous-séquence comprend une sous-séquence 3' d'un intron ou d'un exon d'un premier gène ;
la seconde sous-séquence comprend une sous-séquence en 5' d'un intron ou d'un exon d'un second gène ;
la première sous-séquence et la seconde sous-séquence sont des séquences adjacentes dans l'acide nucléique ;
la première sous-séquence est de 5' par rapport à la seconde sous-séquence ; et
le premier gène et le second gène sont le même gène ou des gènes différents ; éventuellement dans lequel :
la première sous-séquence comprend une sous-séquence 3' d'un intron d'un premier gène ; ou
la seconde sous-séquence comprend une sous-séquence 5' d'un intron d'un second gène ; et/ou
(d) l'acide nucléique est tel que décrit dans la partie (c) et le premier gène et le second gène de la séquence nucléotidique comprenant un intron sont des gènes différents ou sont le même gène ; et/ou
(e) l'acide nucléique comprend en outre une queue poly-A.

7. Acide nucléique selon la revendication 6, dans lequel l'acide nucléique comprend en outre au moins un nucléoside méthylé, éventuellement dans lequel l'au moins un nucléoside méthylé comprend la 7-méthyl guanosine, éventuellement dans lequel l'acide nucléique comprend un chapeau 5' [m7G(5')ppp(5')G].

8. Acide nucléique selon la revendication 6, dans lequel :
(a) l'acide nucléique comprend en outre un promoteur, éventuellement dans lequel le promoteur est un promoteur SP6 ; et/ou
(b) l'acide nucléique comprend en outre un promoteur dans lequel le promoteur provient d'une espèce différente des séquences nucléotidiques de la pluralité, éventuellement dans lequel le promoteur est un promoteur SP6 ; et/ou
(c) l'acide nucléique est un plasmide.

9. Procédé de fabrication de l'acide nucléique selon l'une quelconque des revendications 1 à 7, comprenant l'incubation d'un mélange réactionnel comprenant une matrice d'ADN, une ARN polymérase et des ribonucléotides triphosphates à une température à laquelle l'ARN polymérase présente une activité polymérase, produisant ainsi l'acide nucléique.

10. Cellule comprenant l'acide nucléique selon l'une quelconque des revendications 1 à 8.

11. Cellule selon la revendication 10, dans laquelle :
(a) la cellule est une cellule humaine ; et/ou
(b) la cellule est un fibroblaste ou un lymphocyte, éventuellement un lymphocyte B immortalisé ; et/ou
(c) la cellule est GM12878, GM24149, GM24143, GM24385, GM24631, GM24694, ou GM24695 éventuellement GM24385 ; et/ou
(d) la cellule comprend 1 à 1 000 copies ou 5 à 500 copies de l'acide nucléique ; ou
(e) la cellule est *E. coli.*

12. Composition, comprenant une première pluralité de cellules et une seconde pluralité de cellules, dans laquelle :
la première pluralité de cellules est constituée de cellules selon la revendication 11 partie (d), lesquelles cellules comprennent 5 à 500 copies de l'acide nucléique ;
la seconde pluralité de cellules est constituée de cellules qui ne comprennent pas l'acide nucléique ;
la première pluralité de cellules et la seconde pluralité de cellules sont des cellules humaines ;
la première pluralité de cellules et la seconde pluralité de cellules sont mélangées dans la composition ; et
le rapport du nombre de cellules de la première pluralité au nombre de cellules de la seconde pluralité est d'environ 1:1 à environ 1:10 000 dans la composition.

13. Procédé de fabrication d'un matériau de référence biologique, comprenant la transfection d'une pluralité de cellules avec l'acide nucléique selon l'une quelconque des revendications 1 à 8,
comprenant en outre éventuellement :
(a) la fixation des cellules de la pluralité, éventuellement dans lequel la fixation des cellules comprenant la fixation des cellules avec de la formaline ; et/ou
(b) l'incorporation des cellules dans de la paraffine ; et/ou
(c) la dilution de la pluralité de cellules avec des cellules non transfectées, éventuellement dans lequel la dilution de la pluralité de cellules avec des cellules non transfectées comprend la dilution dans un rapport de cellules transfectées aux cellules non transfectées d'environ 1:1 à environ 1:10 000.

14. Matériau de référence biologique, comprenant :
(a) une pluralité de cellules selon l'une quelconque des revendications 10 ou 11 parties (a) - (d) et de la paraffine, dans lequel la pluralité de cellules sont fixées et incorporées dans la paraffine, comprenant en outre éventuellement des cellules non transfectées, dans lequel les cellules non transfectées ne comprennent pas l'acide nucléique ou la pluralité de fragments d'acide nucléique, éventuellement dans lequel le rapport de cellules comprenant l'acide nucléique ou la pluralité de fragments d'acide nucléique aux cellules non transfectées est d'environ 1:1 à environ 1:10 000, d'environ 1:5 à environ 1:5 000 ou environ 1:10 à environ 1:1 000 ; ou
(b)une pluralité de cellules selon l'une quelconque des revendications 10 ou 11 parties (a) - (d) ; et un liquide, éventuellement dans lequel le liquide est du plasma sanguin.

15. Composition comprenant l'acide nucléique selon l'une quelconque des revendications 1 à 8, et un tampon aqueux, éventuellement dans laquelle le tampon comprend du tris(hydroxyméthyl)aminométhane et de l'acide éthylènediaminetétraacétique, ou un sel de l'un quelconque de ce qui précède.
